# EUROPEAN PATENT SPECIFICATION

(11) **EP 1 001 024 B1**
(45) Date of publication and mention of the grant of the patent: **16.08.2006**
(21) Application number: 99203310.0
(22) Date of filing: 23.04.1992
(51) Int. Cl.: C12N 15/12, C07K 14/00, G01N 33/68

(54) **Protein encoded by the trkB proto-oncogene, used in assay systems for neurothophin activity**
Durch das trkB Proto-onkogen kodiertes Protein, das als Nachweissystem für die Neurotrophin-aktivität gebraucht wird
Protéine codée par le proto-oncogène trkB, utilisée comme système d'analyse permettant de détecter l'activité de la neurotrophine

(30) Priority: 23.04.1991 US 690199; 26.07.1991 US 736559
(43) Date of publication of application: 17.05.2000
(62) Divisional of application: 92912036.8
(73) Proprietor: REGENERON PHARMACEUTICALS, INC., Tarrytown, NY 10591 (US)
(72) Inventor: Squinto, Stephen P., Irvington, NY 10533 (US); Glass, David J., Cortland Manor, NY 10566 (US); Aldrich, Thomas H., New York, NY 10021 (US); DiStephano, Peter, Southborough, MA01772 (US); Stitt, Trevor, Huntington Station, NY 11746 (US); Furth, Marc E., Morrisville, NC 27560 (US); Yancopoulos, George D., Briarcliff Manor, NY 10510 (US); Maisonpierre, Peter C., Croton-On-Hudson, New York 10520 (US); Masiakowski, Piotr, Milford, CT 06460 (US); Ip, Nancy, Stamford, CT 06902 (US)
(74) Representative: Pochart, François

(56) References cited:
- US-A- 5 843 749

## Description

### 1- INTRODUCTION

The present invention provides for a protein which encodes a tyrosine kinase receptor that may serve as a functional binding protein for BDNF and NT-3.

### 2. BACKGROUND OF THE INVENTION

The development and maintenance of the vertebrate nervous system depends on specific proteins, termed neurotrophic factors, originally defined by their ability to support the survival of neuronal populations (Snider and Johnson, 1989, Ann. Neurol. 26:489). Neurotrophic factors have also been implicated in processes involving the proliferation and differentiation of neurons (Cattaneo and McKay, 1990, Nature 347: 762-765; Lindsay and Harmar, 1989, Nature 337: 362-364), and they may play additional, thus far unexplored, roles both within as well as outside of the nervous system. Brain-derived neurotrophic factor (BDNF) and neurotrophin-3 (NT-3) have recently been molecularly cloned and shown to be Structurally related to the prototypical neuronal Survival molecule, nerve growth factor (NGF; Leibrock et al., 1989, Nature 341:149-152; Hohn et al., 1990, Nature 344:339-341; Maisonpierre et al., 1990a, Science 247:1446-1451; Rosenthal et al., 1990, Neuron 4:767-773; Ernfors et al., 1990, Proc. Natl. Acad. Sci. U.S.A. 87:5454-5458; Jones and Reichardt, 1990, Proc. Natl. Acad. Sci. U.S.A. 87:8060-8064). These three related factors (designated "neurotrophins") do not display any structural homology to a fourth neurotrophic factor, ciliary neurotrophic factor (CNTF; Lin et al., 1989, Science 246:1023-1025; Stockli et al., 1989, Nature 342:920-923).

The receptor and signal transduction pathways utilized by NGF have been extensively studied, in large part due to the availability of a pheochromocytoma cell line (PC12) which differentiates in response to NGF (Greene and Tischler, 1976, Proc. Natl. Acad. Sci. U.S.A. 73:2424). These studies have resulted in the cloning of a transmembrane protein (designated "LNGFR" for low-affinity NGF receptor) which binds NGF with relatively low affinity (Chao et al., 1986, Science 232:518-521; Radeke et al., 1987, Nature 325:593-597). In addition to the LNGFR another protein (designated "HNGFR" for high-affinity NGF receptor), which is involved in forming a higher affinity binding site for NGF, is apparently required to initiate NGF-induced signal transduction (Zimmerman et al., 1978, J. Supramol. Struc. 9:351-361; Sutter et al., 1979 in Transmembrane Signalling (N.Y. Alan Liss) pp. 659-667; Bernd and Greene, 1984, J. Bio. Chem. 259:15509-15516; Hempstead et al., 1989, Science 243:373-375). This HNGFR is phosphorylated on tyrosine in response to NGF, and apparently contains intrinsic tyrosine kinase activity (Meakin and Shooter, 1991a, Neuron 6:153-163). Furthermore, the ERK kinases (also known as the MAP2 kinases), early intermediates in tyrosine kinase activated signal cascades, are rapidly activated and phosphorylated on tyrosine in response to NGF. Thus, like many other growth factor responses, NGF signal transduction may be initiated by the activation of a receptor-linked tyrosine kinase.

Recent studies have revealed that the product of the trk proto-oncogene, which resembles a growth factor receptor (i.e., it is a transmembrane protein containing an intracytoplasmic tyrosine kinase domain) for which no ligand had been identified, is rapidly phosphorylated in response to NGF treatment in PC12 cells (Kaplan et al., 1991, Nature 350:156-160; Klein et al., 1991, Cell 65:189-197) and to directly bind NGF with relatively high affinity when expressed in heterologous cells (Klein et al. supra). This finding, together with the restricted neuronal distribution of the trk protein in vivo, suggests that trk may be the component of the HNGFR responsible for initiating NGF signal transduction.

In contrast to the extensive study of NGF receptors and signal transduction pathways, the receptors and signal transduction pathways utilized by the other neurotrophic factors have only recently begun to be explored. However, BDNF appears to bind to the LNGFR with an affinity similar to that of NGF (Rodriguez-Tebar et al., 1990, Neuron 4:487-492). Although both low and high affinity receptors for BDNF exist on neurons responsive to BDNF, the findings that BDNF and NGF act on different neurons and that NGF-responsive neurons do not express high-affinity BDNF receptors suggest that BDNF utilizes a different high affinity receptor than NGF (Rodriguez-Tebar and Barde, 1988, J. Neurosc. 8:3337-3342).

A variety of findings seem to link BDNF and NT-3, while distinguishing both of these neurotrophins from NGF. NT-3 and BDNF (but not NGF) expression displays striking reciprocal relationships during development, with NT-3 being expressed more prominently early and BDNF more prominently late during the development of some of the same brain regions (Maisonpierre et al., 1990, Neuron 5: 501-509). Interestingly, the distribution profiles that BDNF and NT-3 (but not NGF) ultimately achieve in various adult brain regions are quite similar. Id. In peripheral ganglia both BDNF and NT-3 (but not NGF) have their major effects on dorsal root ganglia and nodose ganglia, although NT-3 does seem to have minor effects on sympathetic ganglia (Maisonpierre et al. 1990a, Science 247: 1446-1451). NGF, in contrast, predominantly affects dorsal root ganglia and sympathetic ganglia.

These findings led to the suggestion that BDNF and NT-3 might in some cases act on the same neuronal populations, and that an early effect of NT-3 on these neurons might be replaced by a later effect of BDNF (Maisonpierre et al., 1990b, Neurons 5:501-509). Furthermore, the finding that BDNF and NT-3 (but not NGF) are the most highly conserved growth factors yet described led to the suggestion that both these factors might be interacting with multiple receptors and that their strict conservation was required to maintain the specificity of their interactions with these multiple receptors.

Klein et al. (1989, EMBO J. 8:3701-3709) reported the isolation of trkB, which encodes a new member of the tyrosine protein kinase family of receptors found to be highly related to the human trk protooncogene. At the amino acid level, the products of trk and trkB were found to share 57 percent homology in their extracellular regions, including 9 of the 11 cysteines present in trk. This homology was found to increase to 88 percent within their respective tyrosine kinase catalytic domains. In adult mice, trkB was found to be preferentially expressed in brain tissue, although significant levels of trkB RNAs were also observed in lung, muscle, and ovaries. Further, trkB transcripts were detected in mid and late gestation embryos. In situ hybridization analysis of 14 and 18 day old mouse embryos indicated that trkB transcripts were localized in the central and peripheral nervous systems, including brain, spinal cord, spinal and cranial ganglia, paravertebral trunk of the sympathetic nervous system and various innervation pathways, suggesting that the trkB gene product may be a receptor involved in neurogenesis and early neural development as well as playing a role in the adult nervous system.

In 1990, Klein et al. (Cell 61:647-656) reported that the mouse trkB locus codes for at least two classes of receptor-like molecules, which they designated gp145^{trkB} and gp95^{trkB}. These molecules appear to have identical extracellular and transmembrane domains, but only gp145^{trkB} was found to contain a long cytoplasmic region that included a catalytic protein kinase domain. TrkB transcripts coding for this protein were observed in the cerebral cortex and the pyramidal cell layer of the hippocampus, whereas transcripts encoding gp95^{trkB} were found in the ependymal linings of the cerebral ventricles and in the choroid plexus. Further, Middlemas et al. (1991, Mol. Cell. Biol. 11:143-153) reported the existence of two distinct C-terminally truncated receptors which share the complete extracellular region and transmembrane domain with gp145^{trkB} but which differ from gp145^{trkB} (hitherto referred to simply as trkB) in their short cytoplasmic tails.

### 3. SUMMARY OF THE INVENTION

The present invention provides for a novel tyrosine kinase receptor rtk3 that may serve as a functional binding protein for BDNF and NT-3. Such assay systems may be of particular value in identifying new neurotrophins or agents with aeurotrophin-like activity.

The expression of receptor tyrosine kinases in fibroblasts allows for the use of these cells in survival/proliferation assays that may be used in both the assay of pre-defined agents, (such as the neurotrophins) as well as the discovery of novel agents, that act on these receptor tyrosine kinases; or other receptor tyrosine kinases for which no known ligand exists; these systems can be used even with receptor/ligand systems (such as the trk receptors and the neurotrophins) which may not normally act to mediate cellular proliferation. Once a particular receptor/ligand system is defined (as is done here with trkB and BDNF/NT-3), a variety of additional specific assay systems can be utilized.

he present invention further provides for a orphan tyrosine kinase receptor-like molecules.

The present invention also has diagnostic and therapeutic utilities.

Methods of detecting aberrancies in trkB function or expression may be used in the diagnosis of neurological disorders. Manipulation of the trkB/neurotrophin interaction may be used in the treatment of neurological disorders, including Alzheimer's disease, Parkinson's disease, and amyotrophic lateral sclerosis (Lou Gehrig's disease).

### 3.1. ABBREVIATIONS

- BDNF: brain derived neurotrophic factor
- BSA: bovine serum albumin
- CNTF: ciliary neurotrophic factor
- DSS: Disuccinimidyl suberate
- HNGFR: high affinity nerve growth factor receptor
- LNGFR: low affinity nerve growth factor receptor
- NGF: nerve growth factor
- NT-3: neurotrophin-3
- pCMX-LNGFR: pCMX expression vector for expression of low affinity nerve growth factor receptor
- pCMX-trkB: pCMX expression vector modified for expression of full length rat trkB cDNA
- pCMX-trkB(del): modified form of pCMX-trkB designed to express truncated form of trkB lacking most of the intracytoplasmic tyrosine kinase domain
- pT24-ras: plasmid containing mutated (activated) version of ras oncogen

### 4. DESCRIPTION OF THE FIGURES

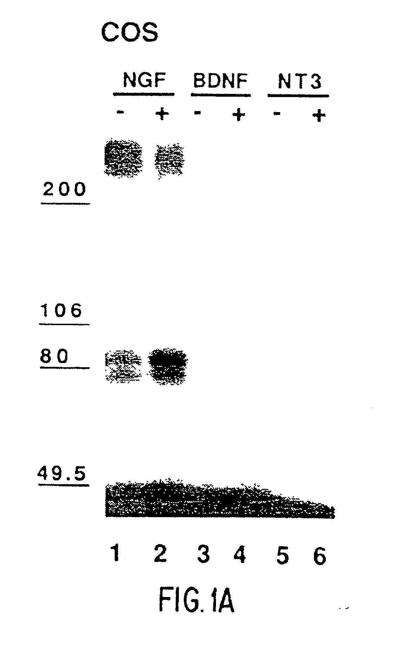

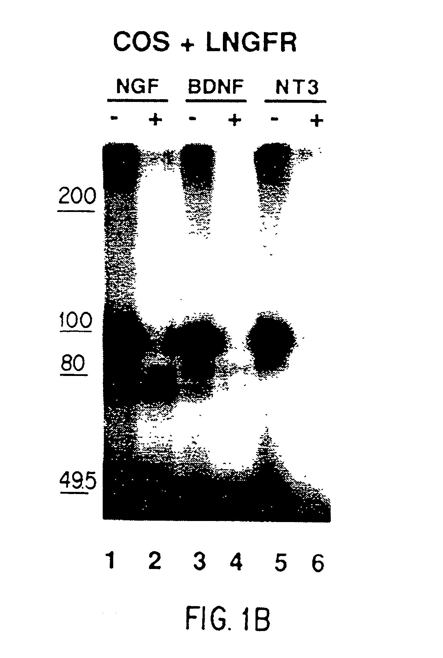

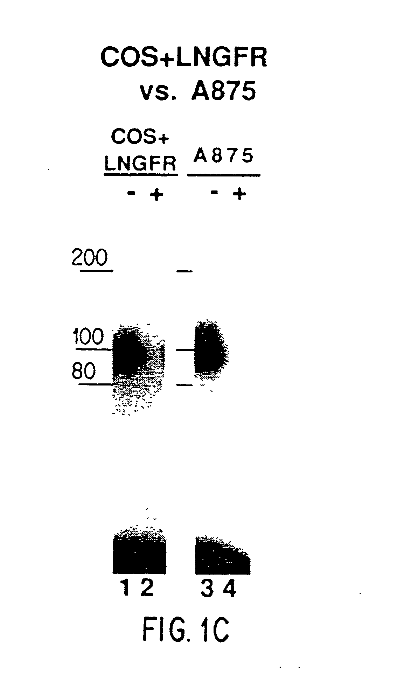

FIGURE 1. All three neurotrophins specifically cross-link to the LNGFR expressed in COS cells.
   A. None of the neurotrophins display competable crosslinking to COS cells transfected with control vector, pCMX. The radiolabeled ligand utilized for each pair of lanes is indicated at the top of the lanes (each radiolabeled ligand is estimated to be at a concentration between 0.1 and 0.25 nM); "-" indicates absence and "+" indicates presence of unlabeled homologous ligand at a concentration of 500 nM. Notable bands seen with radiolabeled NGF varied from experiment to experiment and were not competable with unlabeled NGF, as indicated.
   B. All three radiolabeled neurotrophins display competable cross-linking (resulting in a complex of approximately 100 kD, as expected for LNGFR) in COS cells transfected with pCMX-LNGFR; lanes marked as in panel A. C. Cross-linked species in COS cells transfected with pCMX-LNGFR co-migrates with cross-linked species in human A875 melanoma cells. Radiolabeled ligand used in this panel was BDNF; cells used for cross-linking indicated at top of each pair of lanes, and "-" and "+" as in panel A.

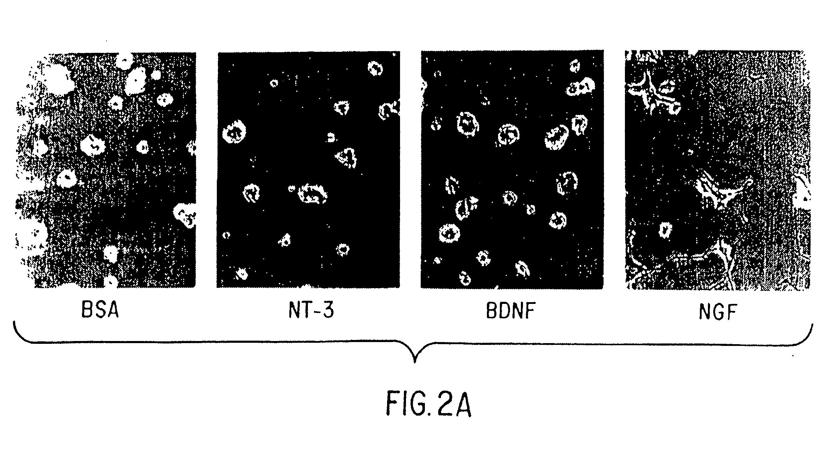

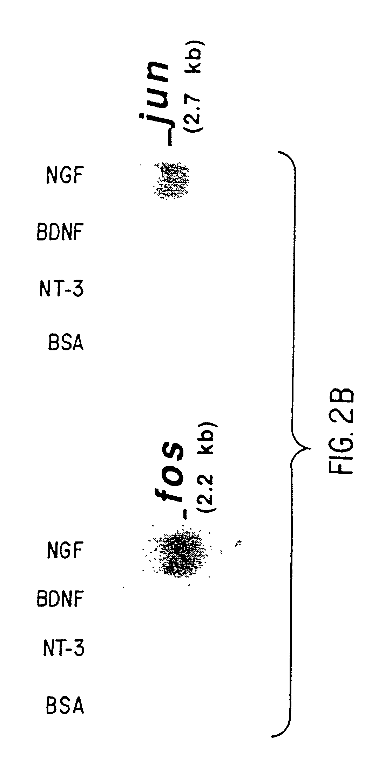

FIGURE 2. Induction of neurite outgrowth and immediate-early gene expression in PC12 cells in response to NGF but not BDNF or NT-3.
   A. PC12 cells cultured as recommended by Greene et al., 1987, Methods Enzymol. 147:, 207-216 in the presence of 100 ng/ml of BSA, NT-3, BDNF or NGF, as indicated. Varying concentrations of neurotrophins were tried; concentrations deemed saturating for NGF are depicted.
   B. fos and jun transcripts (2.2 and 2.7 kb, respectively) identified by Northern analysis of total cellular RNA prepared from PC12 cells cultured as above in the absence of exogenous neurotrophic factors, and then treated for 30 minutes with 100 ng/ml BSA, NT-3, BDNF or NGF as indicated.

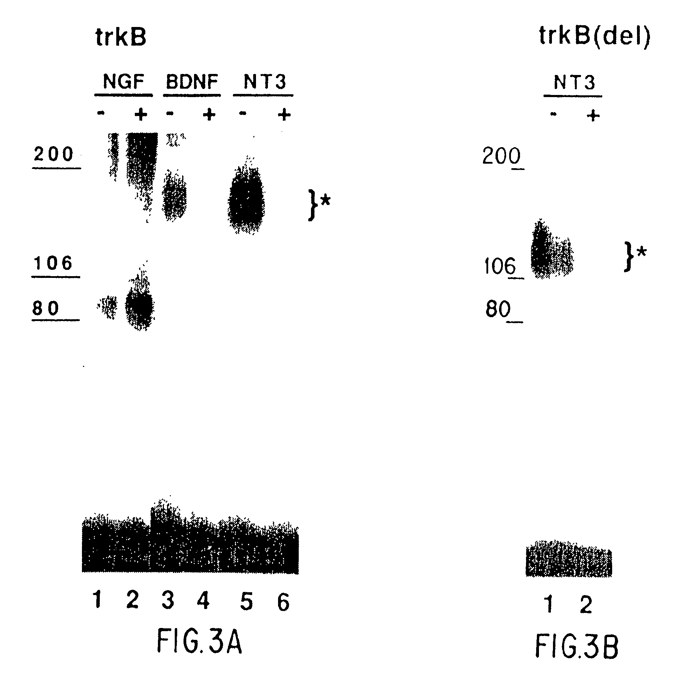

FIGURE 3. BDNF and NT-3, but not NGF, bind specifically to trkB expressed in COS cells.
   A. COS cells transfected with pCMX-trkB and chemically cross-linked to each of the three radiolabeled neurotrophins. Radiolabeled ligand utilized for each pair of lanes is indicated at top of lanes (each radiolabeled ligand is estimated to be at a concentration between 0.1 and 0.25 nM); "-" indicates absence and "+" indicates presence of unlabeled homologous ligand at a concentration of 500 nM. Bracket and asterisk indicate cross-linked species corresponding to trkB protein (approximately 160 to 180 kD).
   B. Cross-linking of radiolabeled NT-3, in the absence ("-") or presence ("+") of unlabeled NT-3 at 500 nM, to COS cells transfected with an expression vector for a truncated form (lacking the tyrosine kinase domain) of trkB (pCMX-trkB(del)). Bracket and asterisk indicate cross-linked species corresponding to truncated version of trkB (120 to 150 kD, as expected for this deletion mutant).

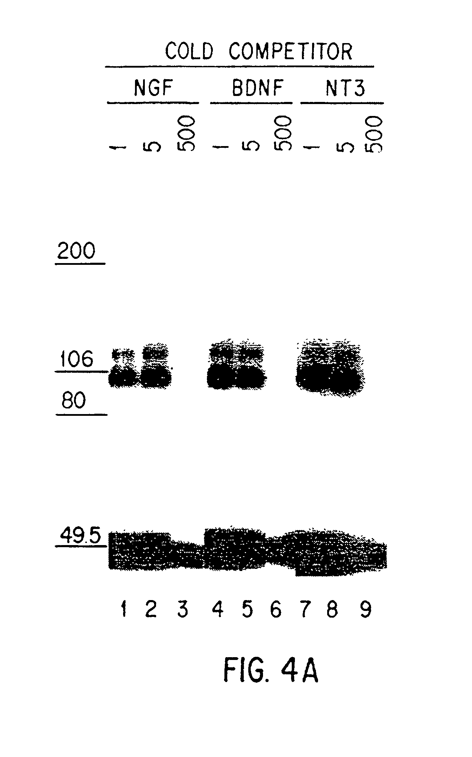

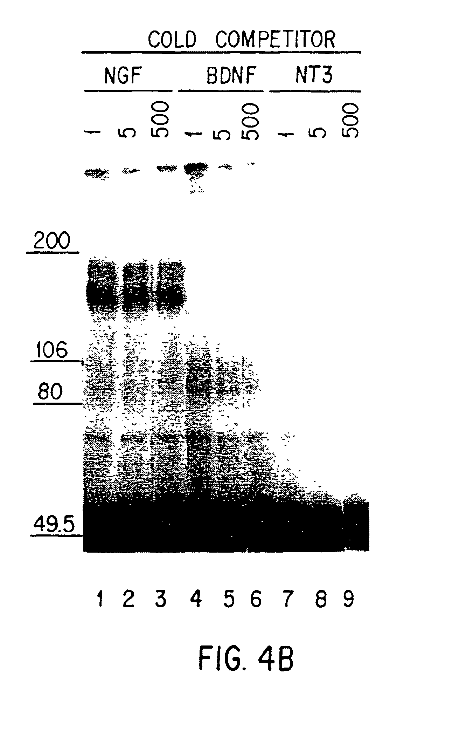

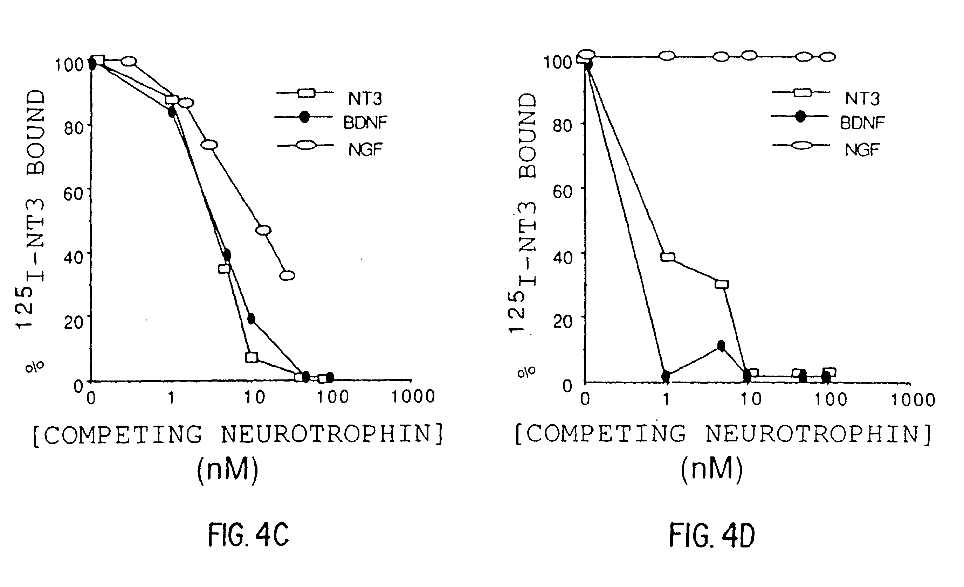

FIGURE 4. Cross-linking and binding of ¹²⁵I-NT-3 to LNGFR is specifically blocked by all three neurotrophins, while cross-linking and binding of NT-3 to trkB is specifically blocked only by BDNF and NT-3.
   A. COS cells transfected with pCMX-LNGFR and cross-linked to radiolabeled NT-3 (estimated concentration between 0.1 to 0.25 nM); the unlabeled neurotrophin used as cold competitor is indicated at the top of each triplet of lanes, with the concentration used per lane (in nM) indicated.
   B. COS cells transfected with pCMX-trkB and cross-linked to radiolabeled NT-3; ligand concentrations and lane markings as in panel A.
   C. COS cells transfected with pCMX-LNGFR used in solution binding experiments with radiolabeled NT-3 (at a concentration estimated to be between 0.1 and 0.25 nM) competed with varying concentrations of unlabeled NT-3, BDNF and NGF, as indicated.
   D. COS cells transfected with pCMX-trkB used in solution binding experiments with radiolabeled NT-3 (at a concentration estimated to be between 0.1 and 0.25 nM) competed with varying concentrations of unlabeled NT-3, BDNF and NGF, as indicated. Date represent the percentage of total cpm bound and are the average of duplicate assays.

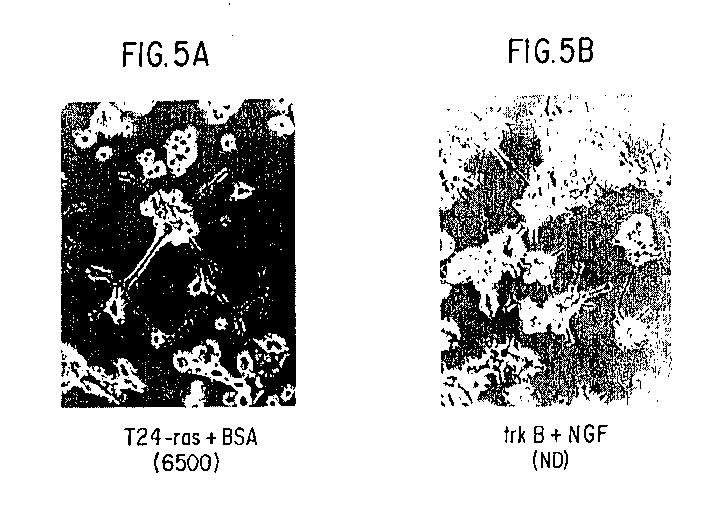

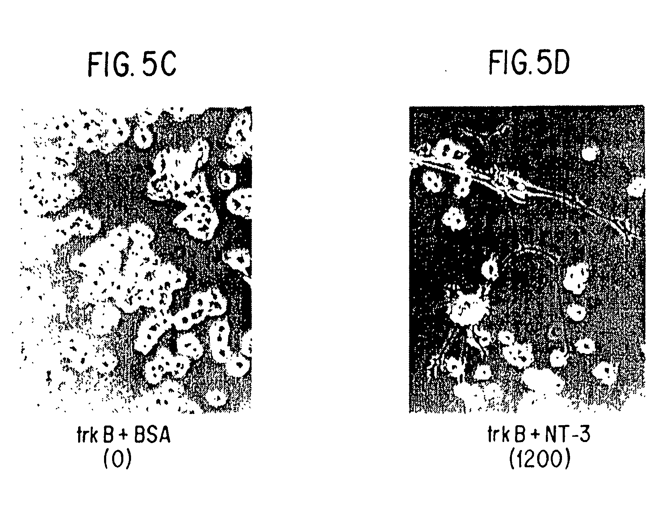

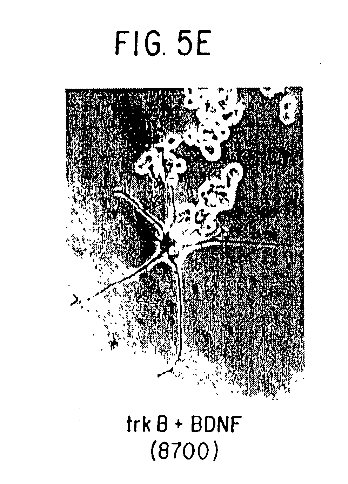

FIGURE 5. PC12 cells transfected with pCMX-trkB differentiate in the presence of BDNF and NT-3.
   A. PC12 cells transiently transfected with control plasmid pT24-ras and treated with 100 ng/ml BSA, used to determine number of transiently transfected cells in each experiment (see text for details).
   B, C, D and E. PC12 cells transiently transfected with pCMX-trkB and treated with 100 ng/ml NGF (panel B), BSA (panel C), NT-3 (panel D) or BDNF (panel E). Numbers (in parentheses) at the bottom of each panel indicate the number of differentiated PC12 cells (i.e. cells having neurites more than twice the length of the cell body) observed per 35 mm well following each treatment; although absolute numbers varied in the three transfections performed, the ratios of differentiated cells observed following the different treatments remained constant for three independent experiments. No differentiated cells were observed in pCMX-trkB transfected PC12 cells treated with BSA (three separate experiments); after electroporation the cells were plated directly on plastic without pre-coating to eliminate any background neurite outgrowth, as described in text and in the Experimental Procedures.

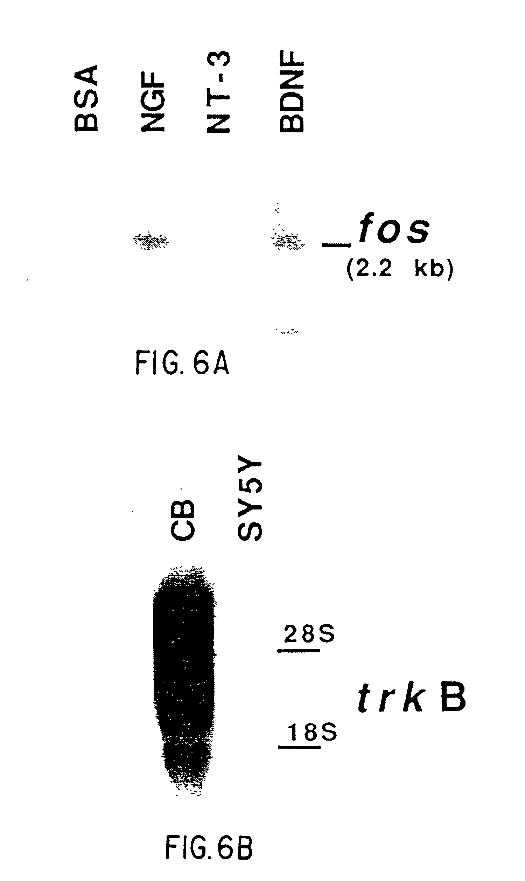

FIGURE 6. The human neuroblastoma SH-SY5Y responds to NGF and BDNF but not NT-3, and does not express trkB.
   A. fos transcripts identified by Northern analysis of total cellular RNA prepared from SH-SY5Y cells cultured in the absence of added factor, and then treated for 30 minutes with 100 ng/ml BSA, NGF, NT-3, or BDNF as indicated.
   B. trkB transcripts are detected by Northern analysis using ten micrograms of total cellular RNA from adult rat cerebellum (designated CB), but are not detectable in ten micrograms of total cellular RNA from SH-SY5Y. The complete coding region of trkB was used as a probe which identifies multiple trkB transcripts (Klein et al., 1989, EMBO J. 8: 3701-3709); the cerebellum lane appears as a smear because of gross over-exposure in the attempt to detect transcripts in SH-SY5Y.

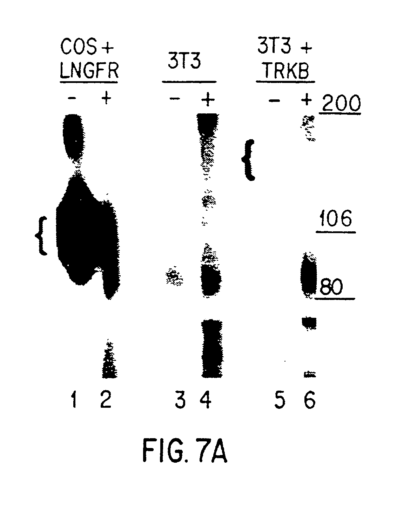

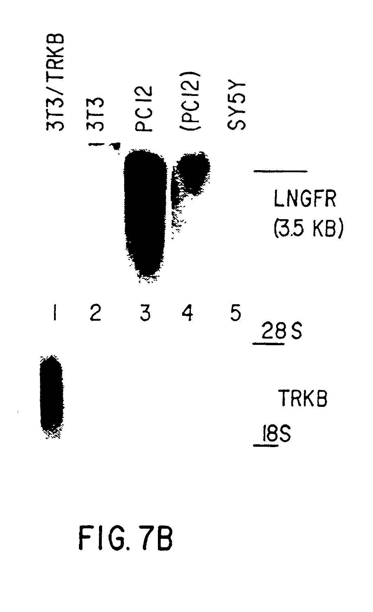

FIGURE 7. Expression of LNGFR and trkB in parental and pLTR-trkB transfected NIH3T3 cells.
   A. Chemical cross-linking of radiolabelled NT-3 to COS cells transfected with an LNGFR expression vector (Squinto et al., 1991, Cell 65:1-20) in lanes 1 and 2, to parental NIH3T3 cells in lanes 3 and 4, and to pLTR-trkB transfected NIH3T3 cells in lanes 5 and 6; "-" indicates absence and "+' indicates presence of unlabeled NT-3 at 500 nM. Cross-linked LNGFR and trkB species are indicated by brackets, and appear similar to detected cross-linked species (Squinto et al., 1991, Cell 65:1-20).
   B. Northern blot analysis of LNGFR expression in trkB-expressing NIH3T3 cells, in the parental NIH3T3 cells, in PC12 cells, and in SH-SY5Y cells. Ten micrograms of total RNA prepared from each of the indicated cell lines (except for the lane signified by PC12 in parenthesis, in which 0.5 micrograms of PC12 cell RNA was diluted in ten micrograms of NIH3T3 cell RNA) was fractionated by formaldehyde gel electrophoresis, transferred to a nylon membrane and hybridized with radiolabelled probes for the LNGFR (top) or trkB (bottom).

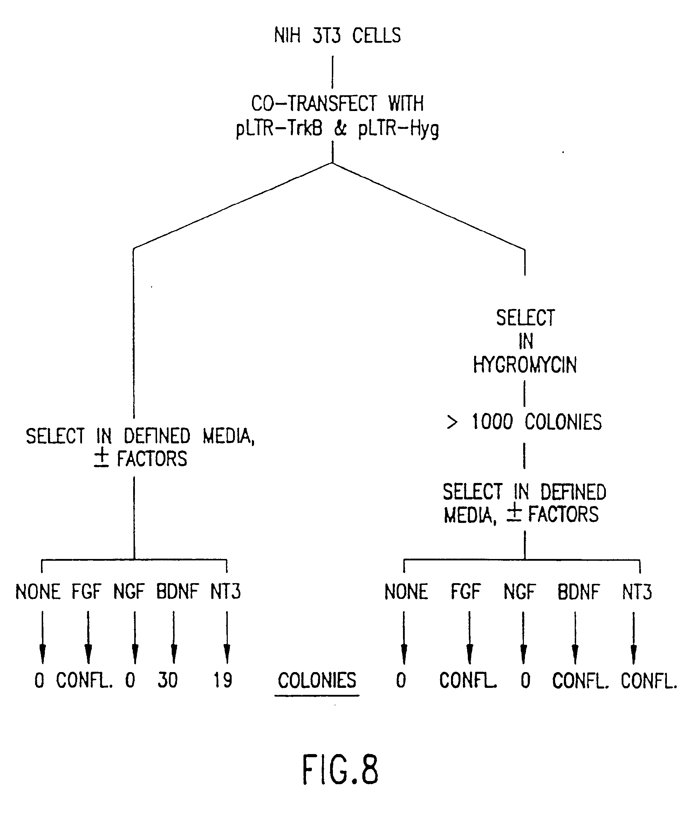

FIGURE 8. Flow chart depicting transfection and selection strategies used to establish and assay trkB-expressing NIH3T3 cells. NIH3T3 cells transfected with 5 micrograms of pLTR-trkB, 1 microgram of pLTR-Hyg and 20 micrograms of human carrier DNA as described in the experimental procedures. Equal aliquots of transfected cells were plated in defined media containing the factors indicated (at 5 nM) or selected in medium containing 10% calf serum and hygromycin. Pooled hygromycin-resistant cells were subsequently placed in defined media as above. While only a small number of colonies were detected by direct selection in defined media supplemented with BDNF or NT-3, confluent survival with BDNF or NT-3 was seen using hygromycin-selected cells ("Conf." signifies confluent survival).

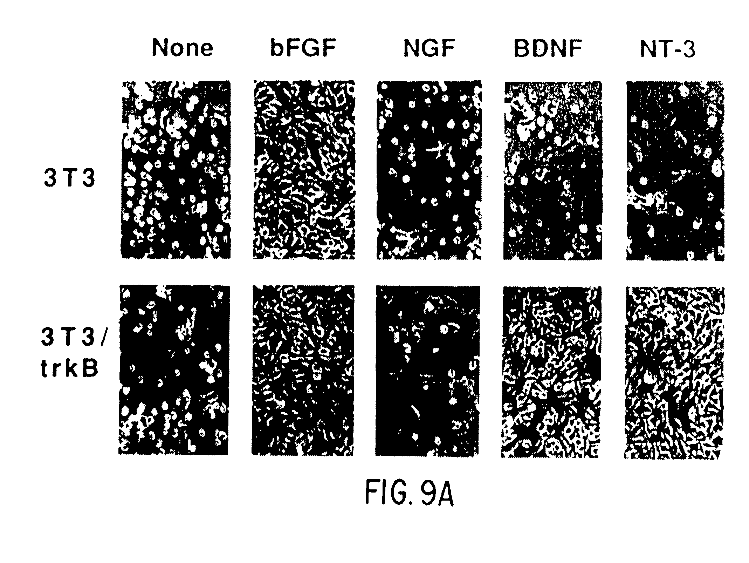

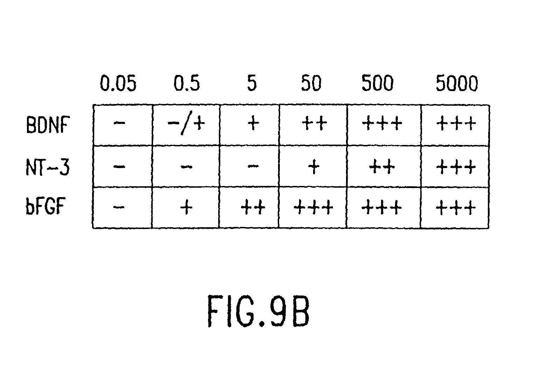

FIGURE 9. Survival of parental and trkB-expressing NIH3T3 cells in defined media supplemented with bFGF or neurotrophins.
   A. Parental (top row) or trkB-expressing NIH3T3 cells (bottom row) plated at 20% confluency, and maintained in defined media (with or without indicated factors at 5 nM) for 5 days.
   B. TrkB-expressing NIH3T3 cells maintained in various concentrations of BDNF, NT-3 or bFGF. "-" indicates nearly complete cell death similar to parental NIH3T3 cells maintained in the absence of added factors (see panel A), "+++" indicates confluent survival similar to parental NIH3T3 cell maintained with 5 nM bFGF (see panel A), and "+" or "++" represent intermediate degrees of survival.

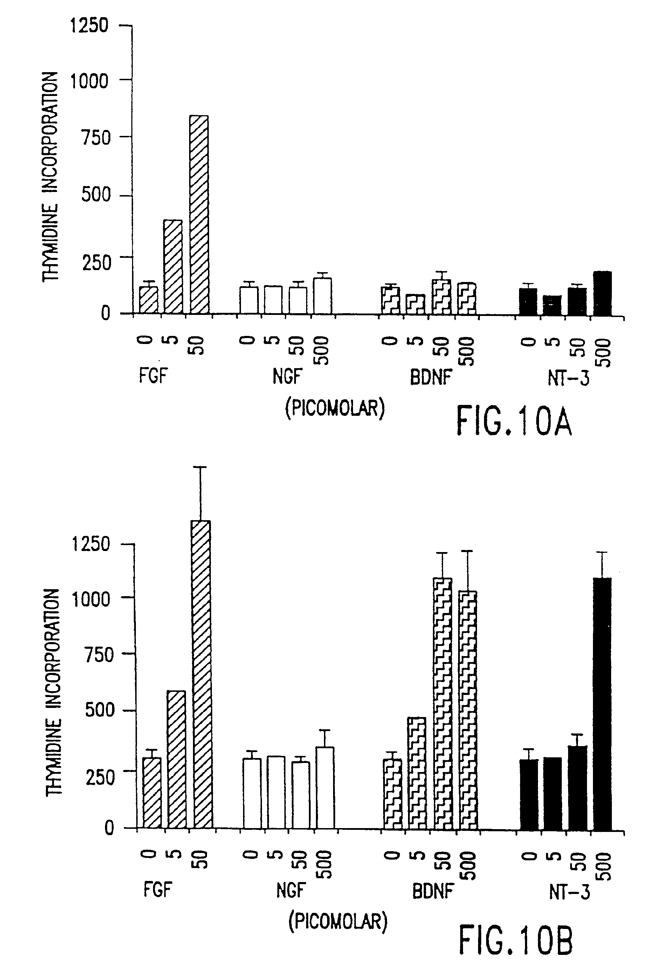

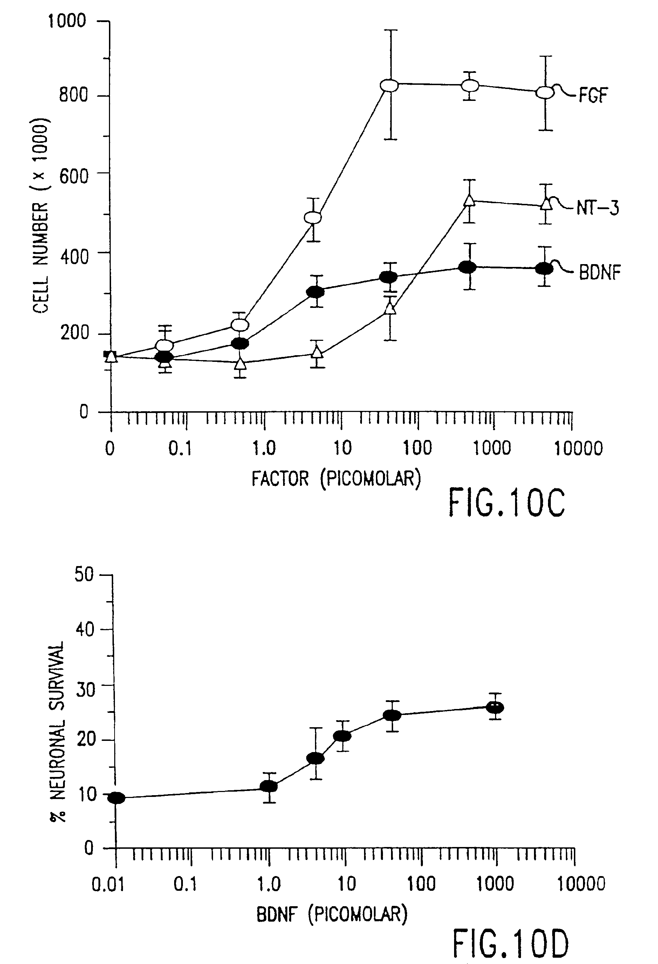

FIGURE 10. Thymidine Incorporation and Proliferation Assays in TrkB-Expressing Fibroblasts Display Similar Neurotrophin Dose Dependency as Do Survival Assays in Primary Neurons.
   A. Thymidine incorporation assay on NIH 3T3 fibroblasts, in response to indicated concentrations of bFGF, NGF, BDNF, and NT-3. Thymidine incorporation indicates counts per minute harvested from a single well of a 24-well plate; error bars are not provided for data points using a 5 pM concentration of each ligand because these points were not performed in duplicate.
   B. Thymidine incorporation assay on trkB-expressing NIH 3T3 fibroblasts, in response to indicated concentrations of bFGF, NGF, BDNF, and NT-3; ordinate units and error bars are as in A.
   C. Proliferation assay of trkB-expressing NIH 3T3 cells in response to differing concentrations of bFGF, BDNF, and NT-3; 5000 cells initially plated for each point, final cell number per well indicated.
   D. Survival assay of primary neurons in dissociated cultures in response to differing concentrations of BDNF (neurons were isolated from dorsal root ganglions of embryonic day 9 chicks), performed as described (Lindsay and Rohrer, 1985 Dev. Biol, 112:30-48).

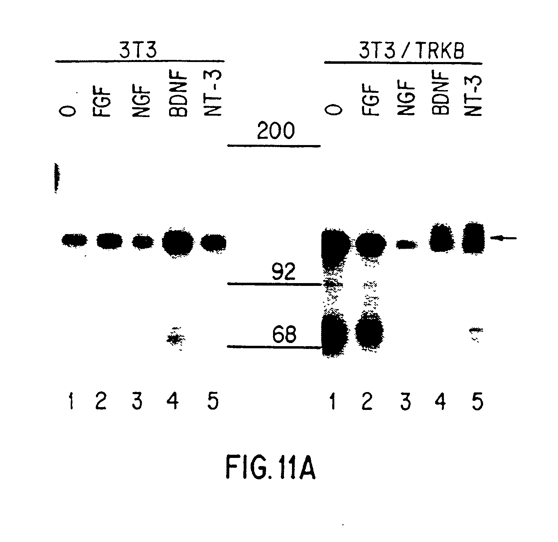

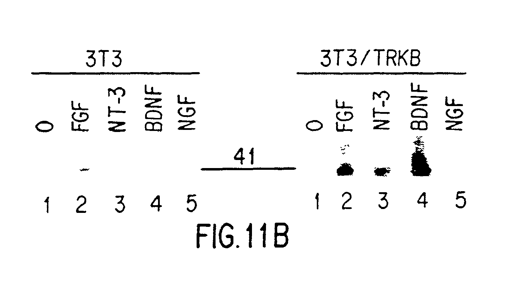

FIGURE 11. Rapid induction of tyrosine phosphorylation in trkB-expressing fibroblasts stimulated with BDNF and NT-3.
   A. Tyrosine phosphorylation of a 145 kD protein, presumed to be trkB (indicated by arrow), is rapidly induced in trkB-expressing NIH3T3 cells stimulated with BDNF or NT-3. Total cell lysates from parental or trkB-expressing NIH3T3 cells, exposed to the indicated factors for five minutes, were immunoprecipitated using an anti-phosphotyrosine monoclonal antibody conjugated to agarose beads, fractionated by acrylamide gel electrophoresis and then immunoblotted with a monoclonal antibody specific for phosphotyrosine.
   B. Tyrosine phosphorylation of a 41 kD protein (presumed to be ERK2) is rapidly induced by bFGF in parental NIH3T3 cells, and by bFGF, NT-3 and BDNF in trkB-expressing NIH3T3 cells. Total cell lysates from parental or trkB-expressing NIH3T3 cells, exposed to the indicated factors for five minutes, were fractionated by acrylamide gel electrophoresis and then immunoblotted with a monoclonal antibody specific for phosphotyrosine.

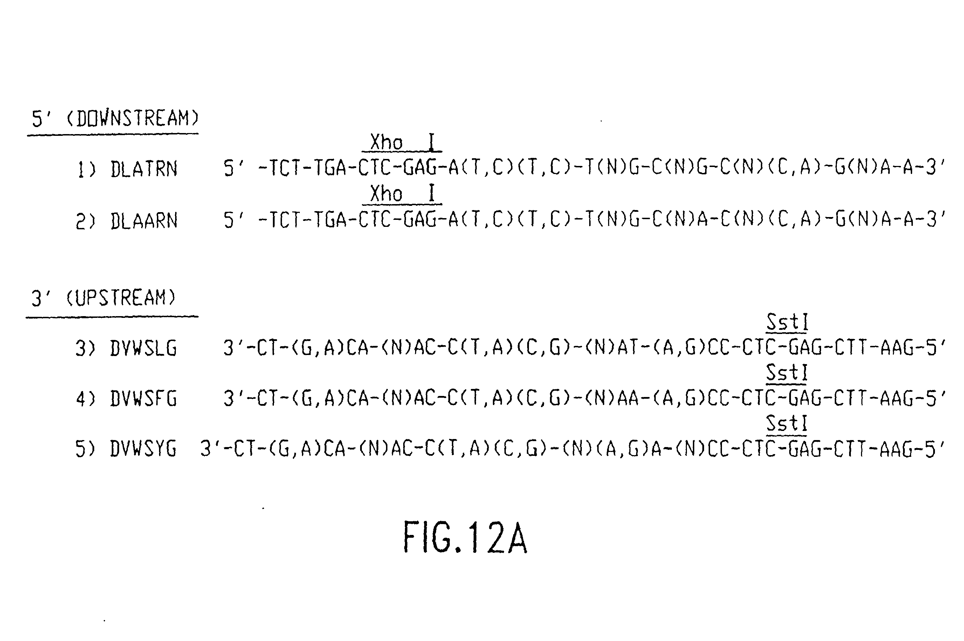

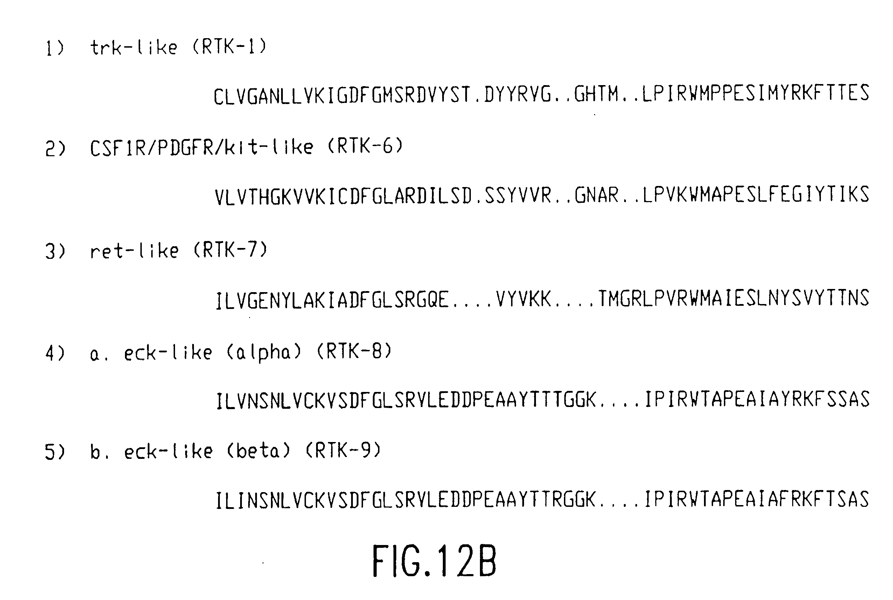

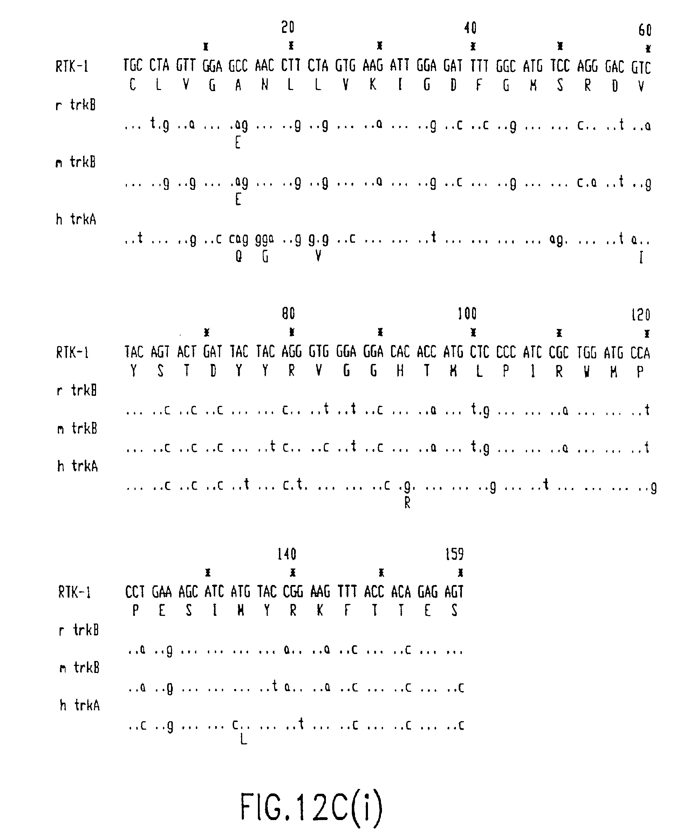

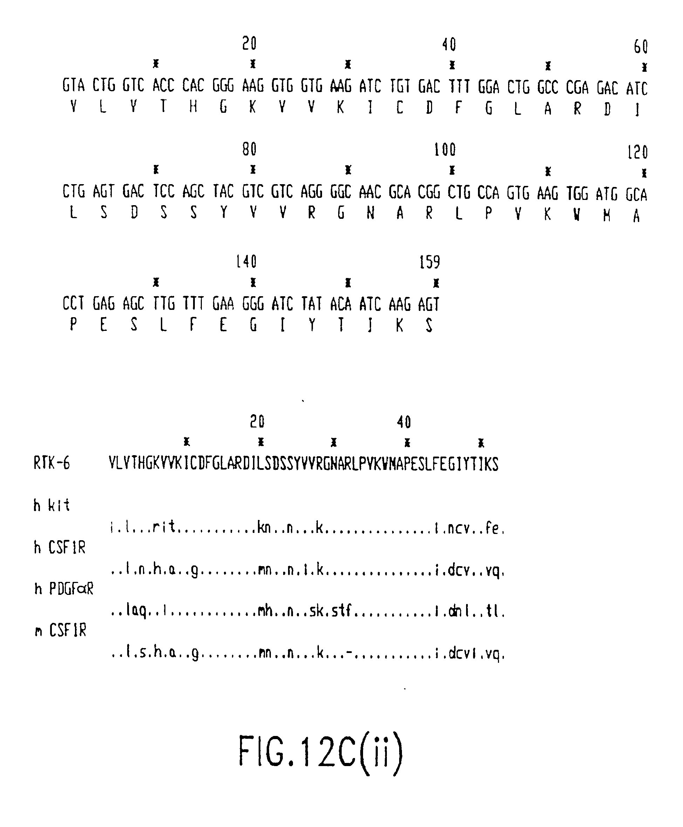

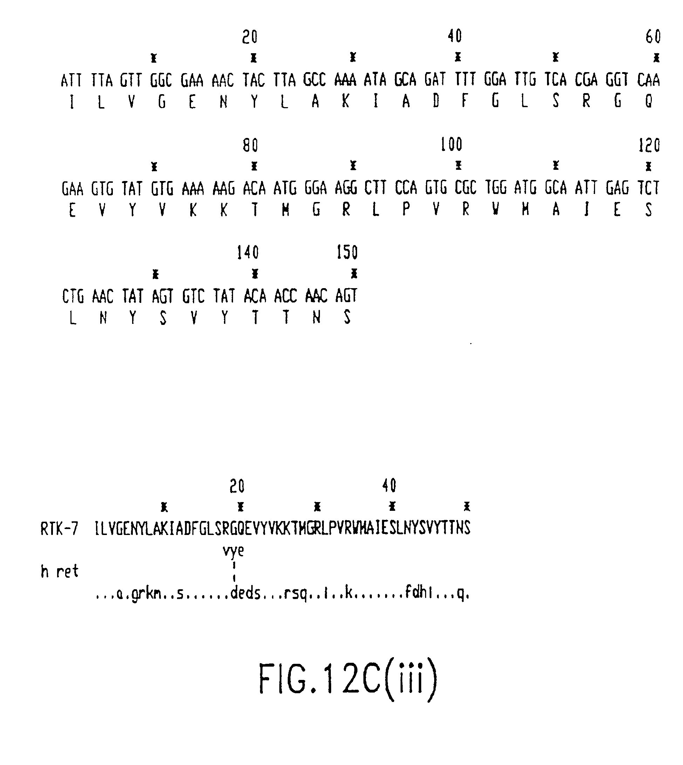

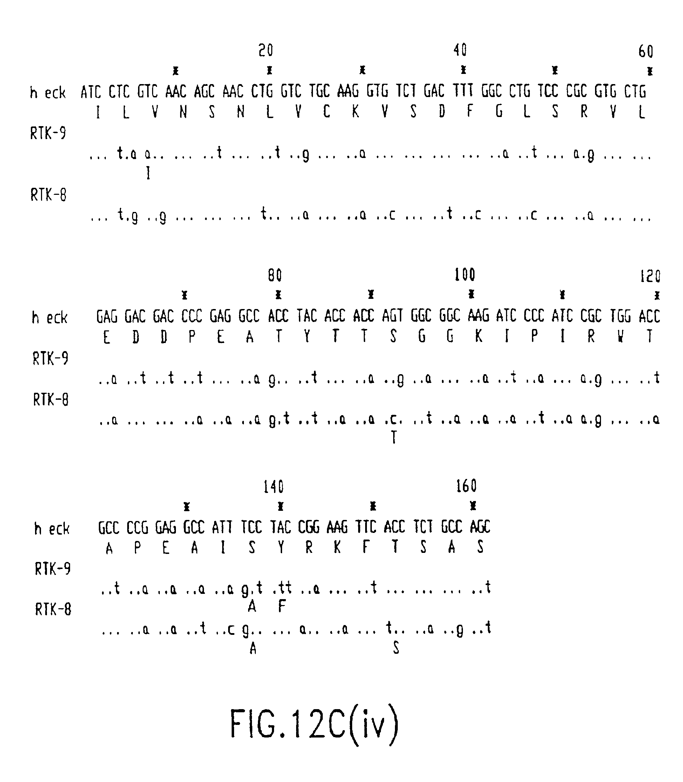

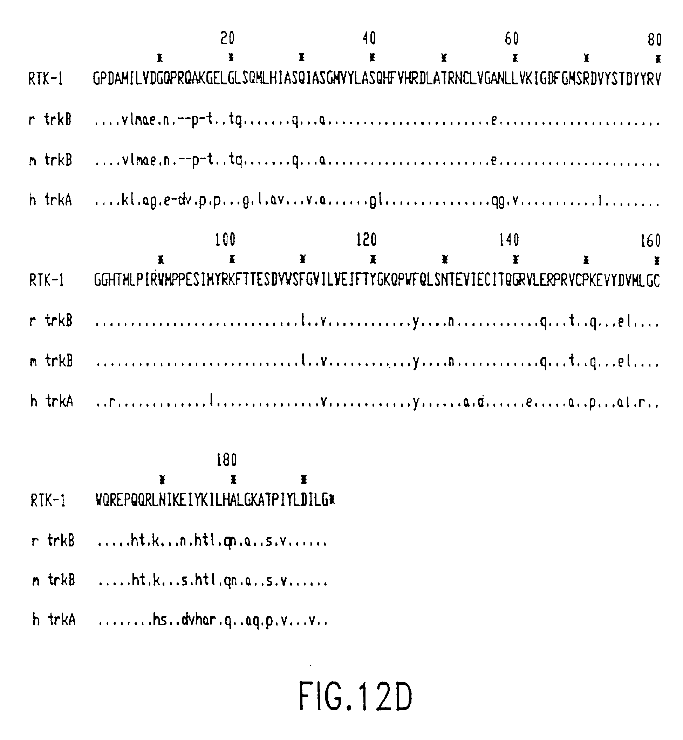

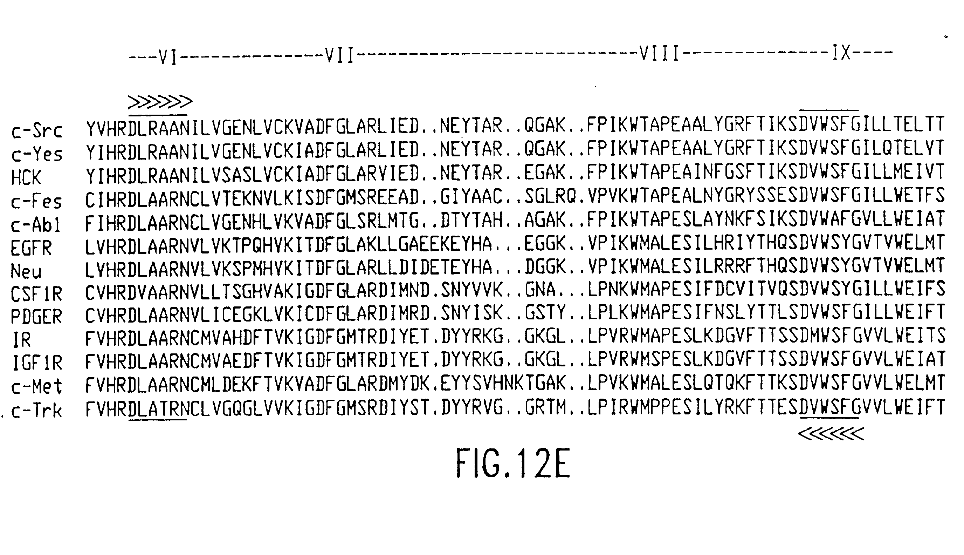

FIGURE 12. A. PCR primers used to amplify sequences homologous to known tyrosine kinase molecules.
   B. Amino acid sequences of novel cloned tyrosine kinases.
   C. Nucleic acid and amino acid sequences of tyrosine kinase clones, namely Rtk-1, Rtk-6, Rtk-7, Rtk-8 and Rtk-9, cDNA source and primers used in PCR reaction, and comparison with homologous molecules.
   D. Allignment of Rtk-1 rat cDNA protein sequence with trk A and trkB.
   E. Tyrosine Kinase homology domains as identified by Hanks et al., 1988, Science 241: 42-52.

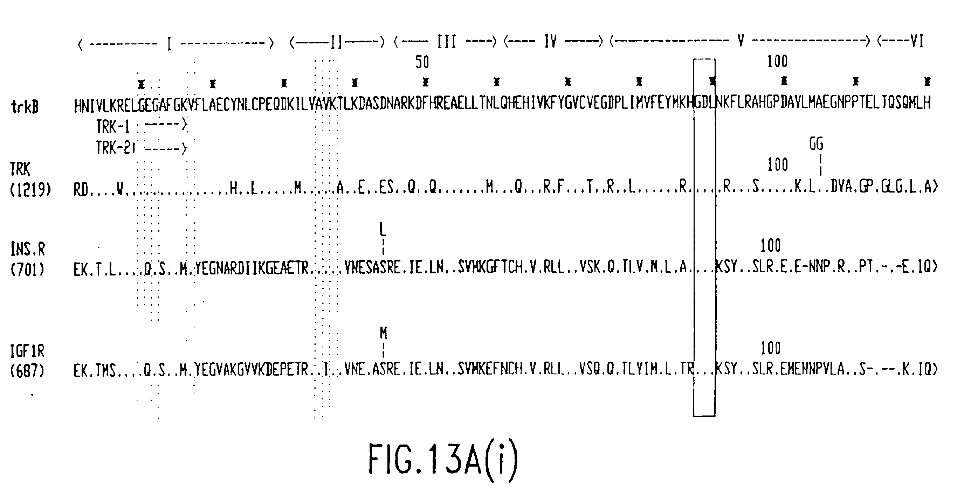

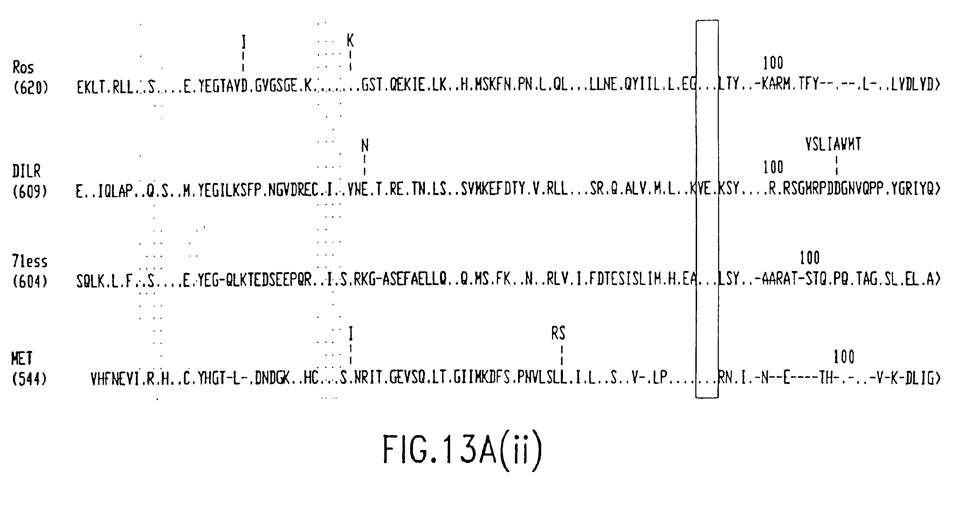

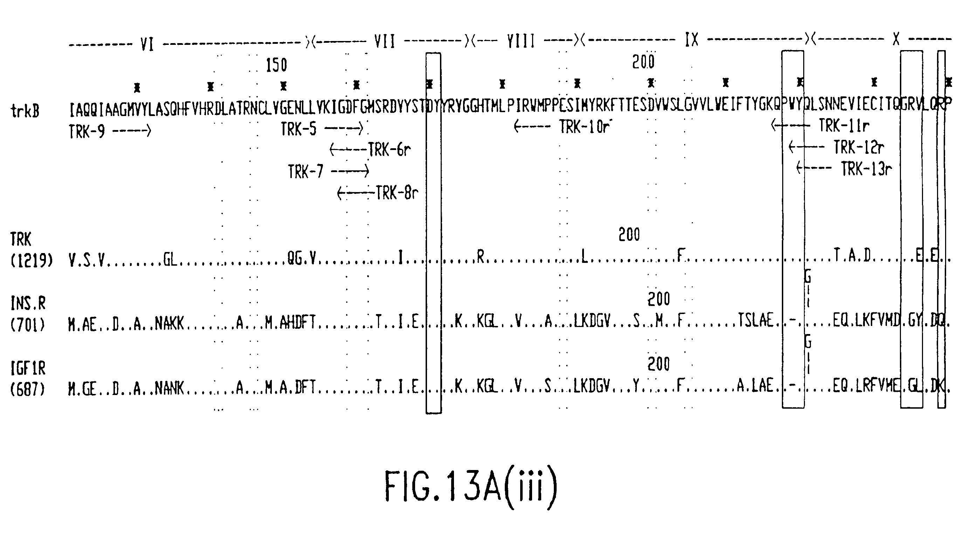

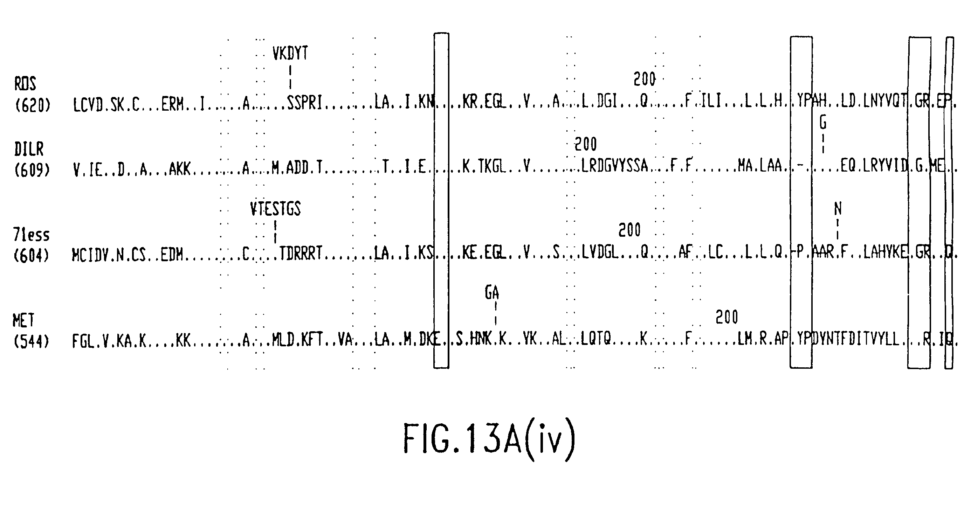

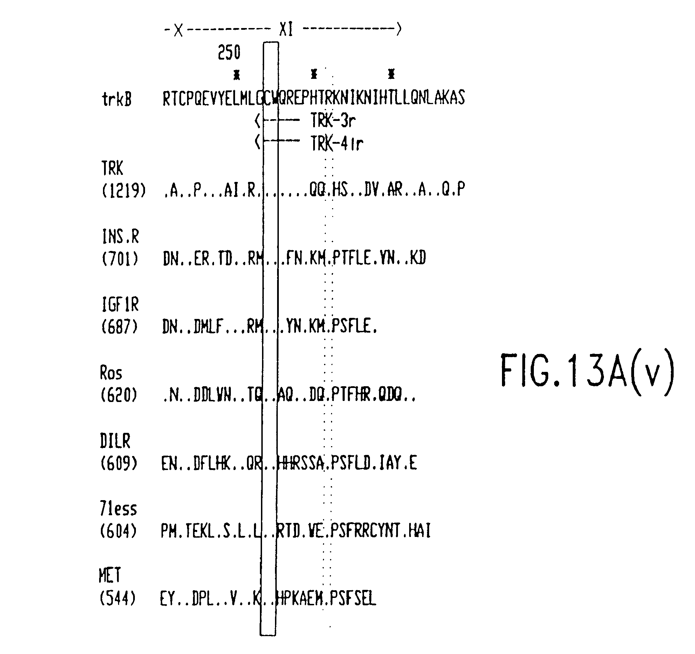

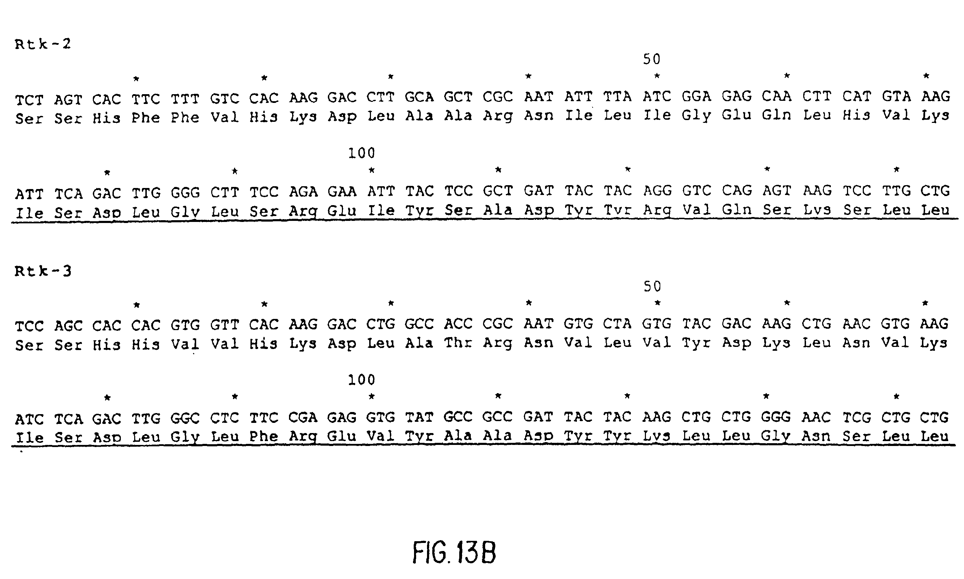

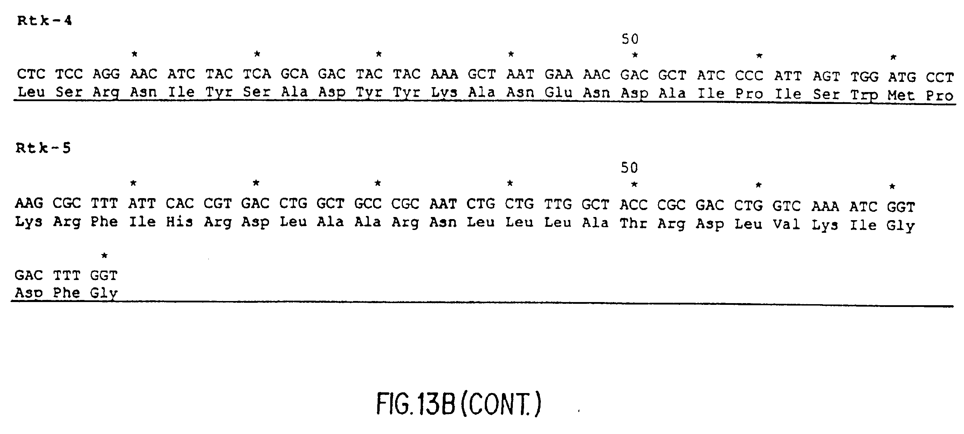

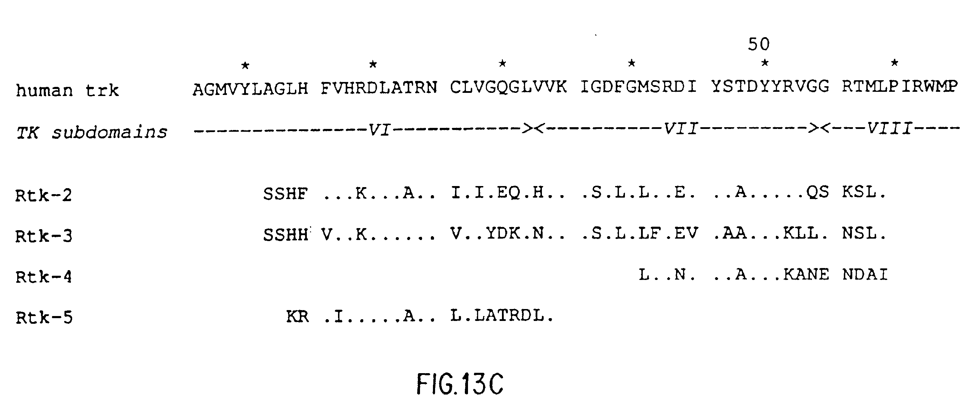

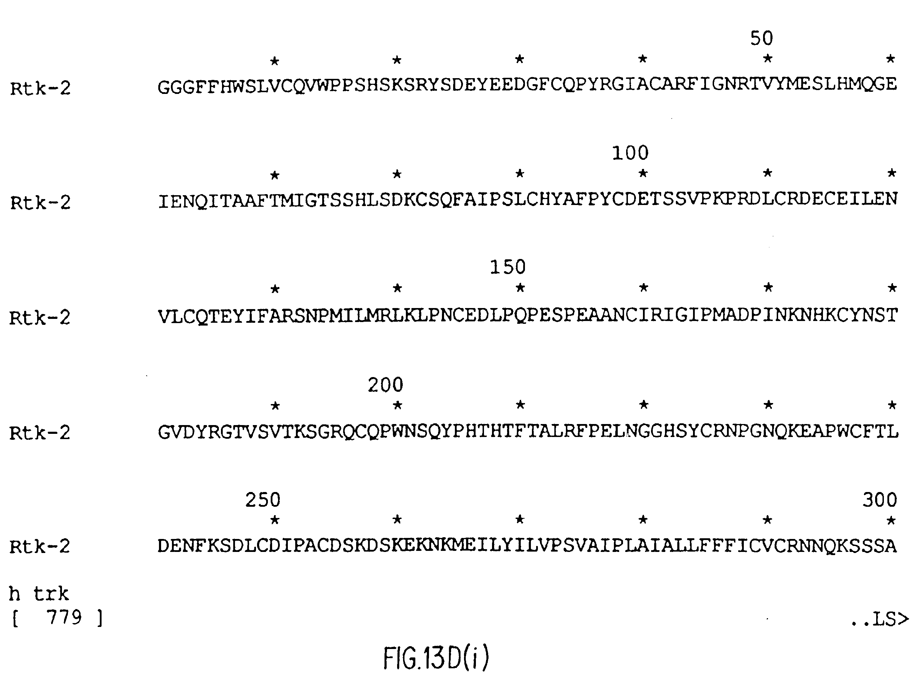

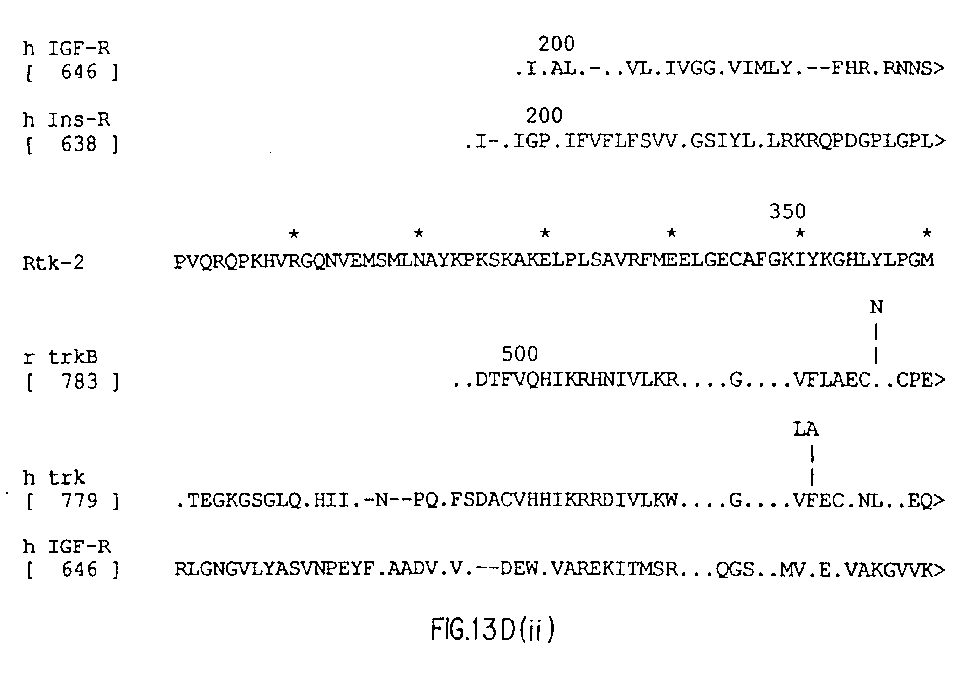

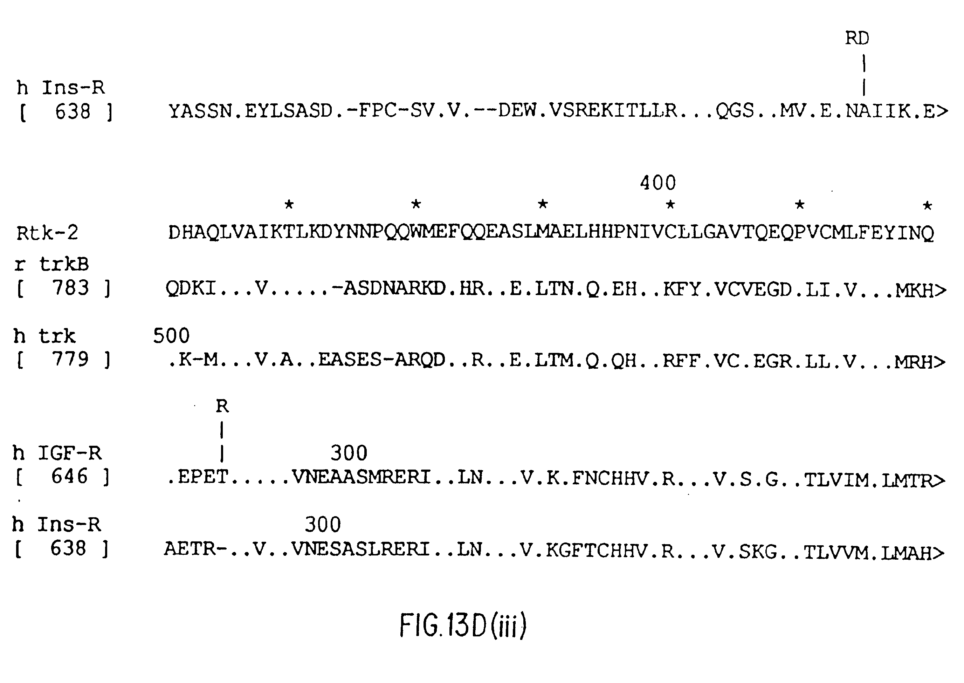

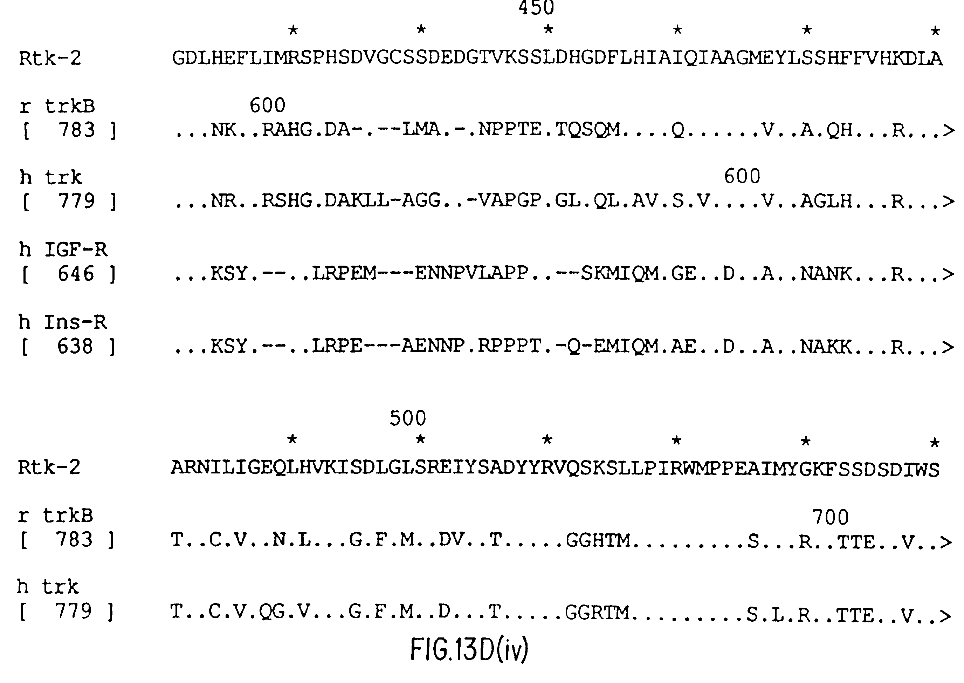

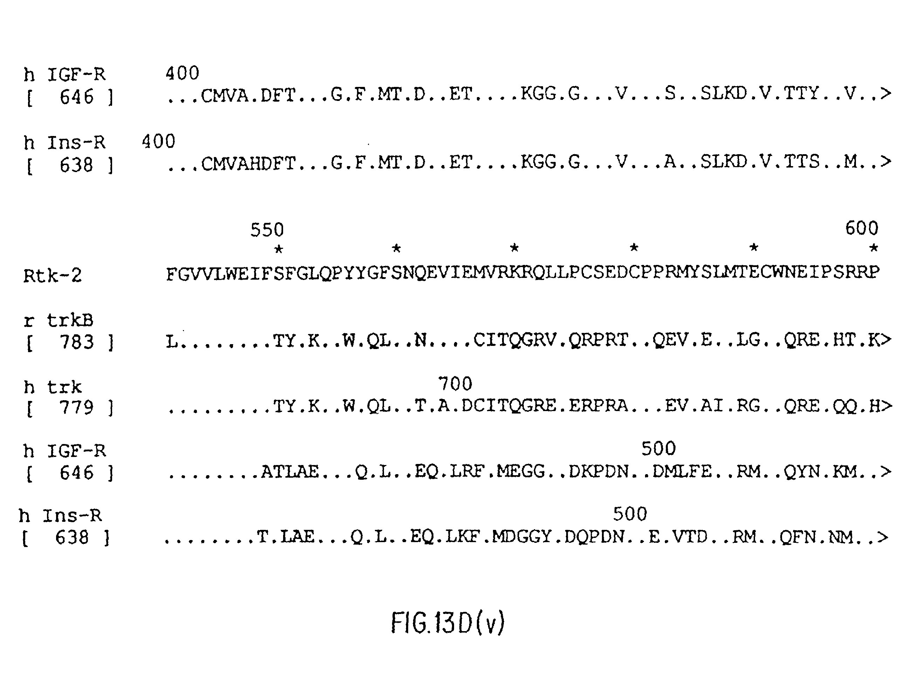

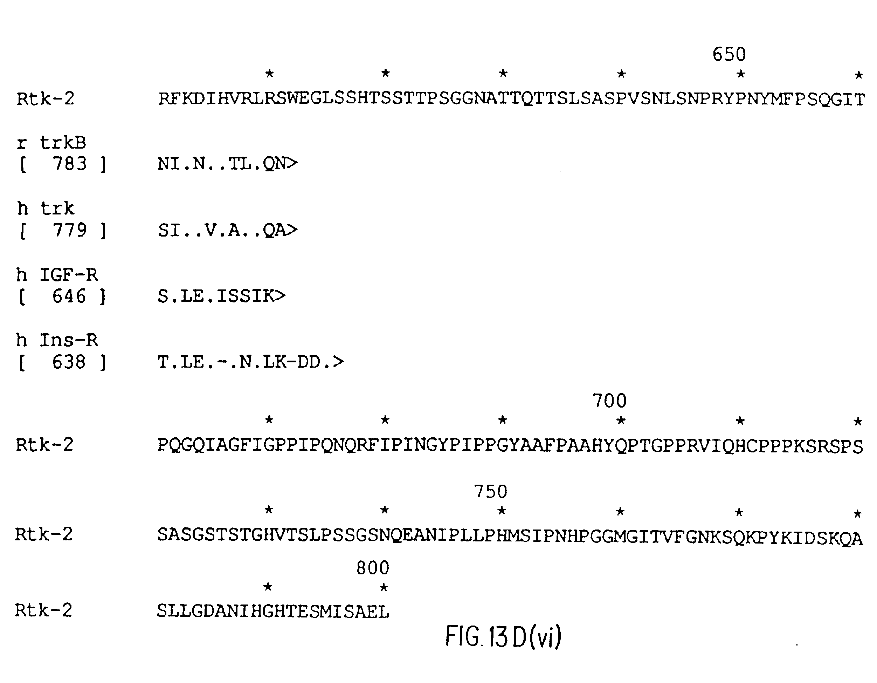

FIGURE 13. A. Comparison of the tyrosine kinase domain of the trk receptors and the insulin receptor subfamily.
   B. Nucleic acid and amino acid sequences of Rtk-2, Rtk-3, Rtk-4, and Rtk-5.
   C. Comparison of deduced amino acid sequences of Rtk-2, Rtk-3, Rtk-4, and Rtk-5 with human trk.
   D. Comparison of the deduced amino acid sequence of Rtk-2 with human trk, rat trkB, insulin related growth factor receptor, and insulin receptor.
FIGURE 14. Nucleotide and deduced amino acid sequence of Rtk-2.
FIGURE 15. Nucleotide and deduced amino acid seuqence of Rtk-3.
FIGURE 16. Alignment of sequences of Rtk-3 with sequences from Rtk-2, trk, trkB, insulin-like growth factor receptor (IGF-R and the insulin receptor (INS-R).
FIGURE 17. Effects of neurotrophins (NGF, BDNF and NT-3) on neurite outgrowth in dorsal root ganglia of embryonic rat (E14). Concentrations of neurotrophins examined are in the range of 0.05pg/ml to 50ng/ml, and culture period was for 24 hr.
FIGURE 18. Embryonic DRG were cultured for 24 hr. in the presence of NGF (50 ng/ml), following which mRNA level for trkA and B were examined. As a control, DRG was taken from rat and not cultured.
FIGURE 19. Embryonic DRG were cultured in the presence of BDNF (50 ng/ml) or NT3 (50ng/ml) for 24 hr, following which mRNA level for trkA, B and C were examined. Trks message were also examined in mediodorsal and ventral spinal cord from embryonic rat (E14).

### 5. DETAILED DESCRIPTION OF THE INVENTION

For purposes of clarity of disclosure, and not by way of limitation, the detailed description of the invention is divided into the following subsections:
(i) assay systems and methods;
(ii) experimental model systems;
(iii) diagnostic methods;
(iv) therapeutic methods; and
(v) systems for the assay and discovery of agents that act on receptor tyrosine kinases and of novel tyrosine kinase receptors

### 5.1. ASSAY METHODS AND SYSTEMS

### 5.1.1. METHODS

The term "neurotrophin activity," as used herein, should be construed to refer to the activity of BDNF or NT-3, or of other, hitherto unidentified neurotrophic factors, or of non-neurotrophic factors (including peptide and nonpeptide molecules) which are capable of binding to trkB. Agents that exhibit neurotrophin activity include but are not limited to neurotrophic and non-neurotrophic factors, including peptide and non-peptide molecules, that have biological activity similar to BDNF and/or NT-3 with respect to immediate early gene induction, cell types affected, phenomena induced, etc. Biological activities of BDNF and NT-3 are described, respectively, in PCT application numbers PCT/US90/04915 and PCT/US90/04916, which are incorporated by reference in their entirety herein. Henceforth, both neurotrophins and agents with neurotrophin activity will be collectively referred to as test agents.

A cell that expresses trkB may either naturally express trkB or be genetically engineered to do so. For example, trkB-encoding nucleic acid sequences obtained as described in section 6.1.2., infra, may be introduced into a cell by transfection, transduction, microinjection, electroporation, via a transgenic animal, etc., using any method known in the art. See for example, the transfection of COS and PC12 cells as described in section 6, infra, and the description of assay systems provided in Section 5.1.2, , infra.

The specific binding of test agent to trkB may be measured in a number of ways. For example, the actual binding of test agent to cells expressing trkB may be detected or measured, by detecting or measuring (i) test agent bound to the surface of intact cells; (ii) test agent cross-linked to trkB protein in cell lysates; or (iii) test agent bound to trkB in vitro. The specific interaction between test agent and trkB may be evaluated by using reagents that demonstrate the unique properties of that interaction. For example, it has been demonstrated, according to the present invention (see section 6) that BDNF and NT-3, but not NGF, bind to trkB. Therefore, the specific binding of test agent to trkB may be competitively inhibited by BDNF or NT-3, but not NGF. Consider a case in which the neurotrophin level (for instance, BDNF) in a sample is to be measured. Varying dilutions of the sample (the test agent), in parallel with a negative control (NC) containing no BDNF activity, and a positive control (PC) containing a known amount of BDNF, may be exposed to cells that express trkB in the presence of detectably labeled BDNF (in this example, radioiodinated BDNF). The amount of BDNF in the test sample may be evaluated by determining the amount of ¹²⁵I-labeled BDNF that binds to the controls and in each of the dilutions, and comparing the sample values to a standard curve. The more BDNF in the sample,the less ¹²⁵I-BDNF that will bind to trkB. The amount of ¹²⁵I-BDNF bound may be determined by measuring the amount of radioactivity per cell, or by cross-linking the BDNF to cell surface proteins using DSS, as described in Meakin and Shooter, 1991, Neuron 6:153-163, and detecting the amount of labeled protein in cell extracts, using, for example, SDS polyacrylamide gel electrophoresis, which may reveal a labeled protein having a size corresponding to BDNF-bound trkB. The specific test agent/trkB interaction may further be tested by adding various dilutions of unlabeled NGF to the assays; such unlabeled NGF should have no substantial affect on the competition between labeled BDNF and test agent for trkB binding. Alternatively, an agent known to be able to disrupt neurotrophin/trkB binding, such as, but not limited to, unlabeled NT-3 or anti-trkB antibody, may be expected to interfere with the competition between ¹²⁵I-BDNF and test agent for trkB binding.

Detectably labeled neurotrophin includes, but is not limited to, neurotrophin linked covalently or noncovalently to a radioactive substance, a fluorescent substance, a substance that has enzymatic activity, a substance that may serve as a substrate for an enzyme (enzymes and substrates associated with colorimetrically detectable reactions are preferred) or to a substance that can be recognized by an antibody molecule that is preferably a detectably labeled antibody molecule.

Alternatively, the specific binding of test agent to trkB may be measured by evaluating the secondary biological effects of neurotrophin/trkB binding, including, but not limited to, the induction of neurite sprouting, immediate early gene expression or phosphorylation of trkB (see Figure 11). For example, the ability of the test agent to induce neurite sprouting can be tested in cells that lack trkB and in comparable cells that express trkB; neurite sprouting in trkB-expressing cells but not in comparable cells that lack trkB would be indicative of a specific test agent/trkB interaction. A similar analysis could be performed by detecting immediate early gene (e.g. fos and jun) induction in trkB-minus and trkB-plus cells, or by detecting phosphorylation of trkB using standard phosphorylation assays known in the art. Such analysis might be useful in identifying neurotrophin agonists or antagonists that do not competitively bind to trkB.

For example, it may be desirable to determine whether a particular sample contains BDNF. PC12 cells, a well characterized neuroblastoma cell line, do not sprout neurites in response to BDNF treatment (see section 6, infra, and Figure 2A). However, PC12 cells transfected with trkB do sprout neurites in response to BDNF (see section 6, infra, and Figure 5). Therefore, normal PC12 cells (trkB-minus cells) and PC12 cells transfected with trkB (trkB-plus cells) may be exposed to the sample (the test agent) and the presence or absence of neurite sprouting may be evaluated microscopically. The amount of BDNF in the sample may be measured by determining the amount of neurite sprouting (or immediate early gene induction) and then comparing this value with a dose response curve for the particular neurotrophin being tested, here, BDNF.

It is possible to identify an agent that has neurotrophin activity by (i) exposing a cell that expresses trkB to a test agent and (ii) detecting the specific binding of the test agent to trkB, in which specific binding to trkB positively correlates with neurotrophin-like activity. Specific binding may be detected by either assaying for direct binding or the secondary biological effects of binding, as discussed supra. Such a method may be particularly useful in identifying new members of the neurotrophin family or, in the pharmaceutical industry, in screening a large array of peptide and non-peptide agents (e.g., peptidomimetics) for neurotrophin-like activity.

A large grid of culture wells may be prepared that contain, in alternate rows, PC12 (or fibroblasts, see infra) cells that are either trkB-minus or engineered to be trkB-plus. A variety of test agents may then be added such that each column of the grid, or a portion thereof, contains a different test agent. Each well could then be scored for the presence or absence of neurite sprouting. An extremely large number of test agents could be screened for neurotrophin activity in this manner.

It is possible to measure neurotrophin activity or to identify an agent as having neurotrophin activity by (i) exposing a test agent to a trkB protein in vitro under conditions that permit binding to occur and (ii) detecting binding of the test agent to the trkB protein, in which binding of test agent to trkB correlates with neurotrophin or neurotrophin-like activity. According to such methods, the trkB may or may not be substantially purified, may be affixed to a solid support (e.g. as an affinity column or as an ELISA assay), or may be incorporated into an artificial membrane. Binding of test agent to trkB may be evaluated by any method known in the art. In preferred embodiments, the binding of test agent may be detected or measured by evaluating its ability to compete with detectably labeled known trkB ligands for trkB binding.

It possible to measure the ability of a test agent compound to function as an antagonist of neurotrophin activity by detecting the ability of the compound to inhibit an effect of neurotrophin binding to trkB on a cell that expresses trkB. Such an antagonist may or may not interfere with trkB/neurotrophin binding. Effects of neurotrophin binding to trkB are preferably biological or biochemical effects, including, but not limited to, neurite sprouting, cell survival or proliferation, cell transformation, immediate early gene induction, or trkB phosphorylation. For example, and not by way of limitation, PC12 cells (or fibroblasts, etc.) transfected with trkB may be exposed to effective amounts of either BDNF or BDNF plus a test agent suspected of being a BDNF antagonist. Neurite sprouting in these two groups of cells may be compared to sprouting in non-transfected cells exposed to BDNF, or BDNF plus the test agent, or NGF, or NGF plus the test agent. If the antagonist specifically inhibits BDNF, neurite sprouting should be inhibited only in trkB-plus cells treated with BDNF plus test agent compared to trkB-plus cells exposed to BDNF, and there should be little or no inhibition of sprouting of trkB-minus cells treated with NGF plus test agent relative to trkB-minus PC12 cells treated with NGF alone.

### 5.1.2. SYSTEMS

Cells that express trkB protein may do so naturally or may be genetically engineered to produce trkB, as described supra, by transfection, transduction, electroporation, microinjection, via a transgenic animal, etc. of nucleic acid encoding trkB in a suitable expression vector.

Any of the methods known to one skilled in the art for the insertion of DNA fragments into a vector may be used to construct expression vectors encoding trkB containing a chimeric gene consisting of appropriate transcriptional/translational control signals and the protein coding sequences. These methods may include in vitro recombinant DNA and synthetic techniques and in vivo recombinations (genetic recombination). Expression of nucleic acid sequence encoding trkB protein or peptide fragment may be regulated by a second nucleic acid sequence so that trkB protein or peptide is expressed in a host transformed with the recombinant DNA molecule. For example, expression of trkB may be controlled by any promoter/enhancer element known in the art. Promoters which may be used to control trkB expression include, but are not limited to the long terminal repeat as described in Squinto et al., (1991, Cell 65:1-20); the SV40 early promoter region (Bernoist and Chambon, 1981, Nature 290:304-310), the CMV promoter, the M-MuLV 5' terminal repeat the promoter contained in the 3' long terminal repeat of Rous sarcoma virus (Yamamoto, et al., 1980, Cell 22:787-797), the herpes thymidine kinase promoter (Wagner et al., 1981, Proc. Natl. Acad. Sci. U.S.A. 78:144-1445), the regulatory sequences of the metallothioein gene (Brinster et al., 1982, Nature 296:39-42); prokaryotic expression vectors such as the *I704*12*b*I704*10*-lactamase promoter (Villa-Kamaroff, et al., 1978, Proc. Natl. Acad. Sci. U.S.A. 75:3727-3731), or the tac promoter (DeBoer, et al., 1983, Proc. Natl. Acad. Sci. U.S.A. 80:21-25), see also "Useful proteins from recombinant bacteria" in Scientific American, 1980, 242:74-94; promoter elements from yeast or other fungi such as the Gal 4 promoter, the ADH (alcohol dehydrogenase) promoter, PGK (phosphoglycerol kinase) promoter, alkaline phophatase promoter, and the following animal transcriptional control regions, which exhibit tissue specificity and have been utilized in transgenic animals: elastase I gene control region which is active in pancreatic acinar cells (Swift et al., 1984, Cell 38:639-646; Ornitz et al., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl et al., 1984, Cell 38:647-658; Adames et al., 1985, Nature 318:533-538; Alexander et al., 1987, Mol. Cell. Biol. 7:1436-1444), mouse mammary tumor virus control region which is active in testicular, breast, lymphoid and mast cells (Leder et al., 1986, Cell 45:485-495), albumin gene control region which is active in liver (Pinkert et al., 1987, Genes and Devel. 1:268-276), alpha-fetoprotein gene control region which is active in liver (Krumlauf et al., 1985, Mol. Cell. Biol. 5:1639-1648; Hammer et al., 1987, Science 235:53-58); alpha 1-antitrypsin gene control region which is active in the liver (Kelsey et al, 1987, Genes and Devel. 1:161-171), beta-globin gene control region which is active in myeloid cells (Mogram et al., 1985, Nature 315:338-340; Kollias et al., 1986, Cell 46:89-94);myelin basic protein gene control region which is active in oligodendrocyte cells in the brain (Readhead et al., 1987, Cell 48:703-712); myosin light chain-2 gene control region which is active in skeletal muscle (Sani, 1985, Nature 314:283-286), and gonadotropic releasing hormone gene control region which is active in the hypothalamus (Mason et al., 1986, Science 234:1372-1378).

Expression vectors containing trkB gene inserts can be identified by three general approaches: (a) DNA-DNA hybridization, (b) presence or absence of "marker" gene functions, and (c) expression of inserted sequences. In the first approach, the presence of a foreign gene inserted in an expression vector can be detected by DNA-DNA hybridization using probes comprising sequences that are homologous to an inserted trkB gene. In the second approach, the recombinant vector/host system can be identified and selected based upon the presence or absence of certain "marker" gene functions (e.g., thymidine kinase activity, resistance to antibiotics, transformation phenotype, occlusion body formation in baculovirus, etc.) caused by the insertion of foreign genes in the vector. For example, if the trkB gene is inserted within the marker gene sequence of the vector, recombinants containing the trkB insert can be identified by the absence of the marker gene function. In the third approach, recombinant expression vectors can be identified by assaying the foreign gene product expressed by the recombinant. Such assays can be based, for example, on the physical or functional properties of the trkB gene product, for example, by binding of the receptor to neurotrophic factor or to an antibody which directly recognizes the trkB. Cells of the present invention may transiently or, preferably, constitutively and permanently express trkB.

One can provide cells that express trkB and that also contain recombinant nucleic acid comprising an immediate early gene promoter (e.g. the fos or jun promoters (Gilman et al., 1986, Mol. Cell. Biol. 6:4305-4316). When such a cell is exposed to a neurotrophin, the neurotrophin may be expected to bind to trkB and secondarily induce transcription off the immediate early promoter. Such a cell may be used to detect neurotrophin/trkB binding by measuring the transcriptional activity of the immediate early gene promoter, for example, by nuclear run-off analysis, Northern blot analysis, or by measuring levels of a gene controlled by the promoter. The immediate early promoter may be used to control the expression of fos or jun or any detectable gene product, including, but not limited to, any of the known reporter genes, such as a gene that confers hygromycin resistance (Murphy and Efstratiadis, 1987, Proc. Natl. Acad. Sci. U.S.A. 84:8277-8281) chloramphenicol acetyltransferase (CAT), neomycin phosphotransferase (neo), beta-galactosidase beta-glucuronidase, beta-galactosidase, etc.

Neurotrophin/trkB binding in a cell that expresses trkB and contains the human growth hormone gene under the control of the fos gene promoter may be expected to produce recombinant human growth hormone, as measured by Seldon et al., 1986, Mol. Cell. Biol. 6-3173-3179. TrkB expression may also be used as a reporter gene and be placed under the control of an immediate early promoter in addition to constitutively expressed trkB to produce an amplified response to neurotrophin. Such trkB-expression reporter gene containing cell lines may provide an exceptionally sensitive and efficient method of detecting or measuring neurotrophin activity.

Furthermore, the cells used may or may not be cells of the nervous system. For example, growth-factor dependent fibroblasts may be used as the basis for a neurotrophin assay system. See Section 7, infra. A fibroblast cell line that is growth factor dependent in serum-free media (e.g. as described in Zham and Goldfarb, 1986, Mol. Cell. Biol. 6:3541-3544) may be transfected with the trkB gene, for instance by using a CaPO₄ transfection protocol with 5 micrograms of DNA of CMV-promoter-based expression vector comprising the rat trkB gene and one microgram of hygromycin-resistance gene-containing expression vector. After about 48 hours, the cells may then be selected for hygromycin resistance to identify positive transfectants. The cells may then be cultured for about three weeks in the presence of hygromycin,and then resistant colonies may be pooled. These cells may then be plated on tissue culture plates coated with poly-D-lysine and human fibronectin, and allowed to grow in DMEM plus 10% bovine calf serum for about four hours to allow the cells to bind to the plates. The serum-containing media may then be aspirated and the cells may be washed about three times with PBS to remove any residual serum. The cells may then be taken up with either serum free defined media (A 3:1 mixture of DMEM and Hams F12, supplemented with 8 mM sodium bicarbonate, 15 mM HEPES, 4 x 10⁻⁶M MnCl₂, 3 mM histidine, 10⁻⁵M ethanolamine, 10⁻⁷M sodium selenite, 5 mg transferrin per liter, 200 mg bovine serum albumin-linoleic acid complex per liter gentamicin, penicillin, and streptomycin, 20 mM L-glutamine). Cells produced in this manner, then incubated with neurotrophin (e.g. 100 ng/ml NT-3 or BDNF), may, after about 5 days in culture (replacing media and growth factors every 48 hours), be expected to be growing and proliferating; cells treated with NGF at 100 ng/ml or in serum free-medium should not, however, proliferate (see also Figure 7). As discussed in Section 6, infra, data suggests that there is another, non-trkB receptor for BDNF expressed on SH-SY5Y cells (see also Figure 6).

### 5.2. EXPERIMENTAL MODEL SYSTEMS

One can provide experimental model systems for studying the physiological role of the neurotrophin gene family. In these model systems, trkB protein, peptide fragment, or a derivative thereof, may be either supplied to the system or produced within the system. Such model systems could be used to study the effects of neurotrophin excess or neurotrophin depletion. The experimental model systems may be used to study the effects of increased or decreased response to neurotrophin in cell or tissue cultures, in whole animals, in particular cells or tissues within whole animals or tissue culture systems, or over specified time intervals (including during embryogenesis) in embodiments in which trkB expression is controlled by an inducible or developmentally regulated promoter. The CMV promoter may be used to control expression of trkB in transgenic animals. The term "transgenic animals," as used herein, refers to non-human transgenic animals, including transgenic mosaics, which carry a transgene in some or all of their cells, which include any non-human species, and which are produced by any method known in the art, including, but not limited to microinjection, cell fusion, transfection, electroporation, etc. For example, the animals may be produced by a microinjection of zygotes by a method such as that set forth in "Brinster et al., 1989, Proc. Natl. Acad. Sci. U.S.A. 82:4438-4442.

One can provide model systems for autoimmune disease in which an autoimmune response is directed toward trkB. Such models comprise animals which have been immunized with immunogenic amounts of trkB and preferably found to produce anti-trkB antibodies and/or cell-mediated immunity. To produce such a model system, it may be desirable to administer the trkB in conjunction with an immune adjuvant, such as Bacille Calmette Guerin (BCG).

### 5.2.1. MODELS FOR INCREASED NEUROTROPHIN ACTIVITY

An experimental model system may be created which may be used to study the effects of excess neurotrophin activity. In such a system, the response to neurotrophin may be increased by engineering an increased number of trkB molecules on cells of the model system relative to cells which have not been so engineered. It may be preferable to provide an increased number of neurotrophins selectively on cells which normally express neurotrophins.

Cells may be engineered to produce increased amounts of trkB protein by infection with a virus which carries a trkB gene of the invention. Alternatively, the trkB gene may be provided to the cells by transfection.

If the model system is an animal, a recombinant trkB gene may be introduced into the cells of the animal by infection with a virus which carries the trkB gene. Alternatively, a transgenic animal may be created which carries the trkB gene as a transgene.

In order to ensure expression of trkB, the trkB gene should be placed under the control of a suitable promoter sequence. It may be desirable to put the trkB gene under the control of a constitutive and/or tissue specific promoter, including but not limited to the CNS neuron specific enolase, neurofilament, and tyrosine hydroxylase promoter, an inducible promoter, such as the metallothionein promoter, the UV activated promoter in the human immunodeficiency virus long terminal repeat (Valeri et al., 1988, Nature 333:78-81), or the CMV promoter (as contained in pCMX, infra) or a developmentally regulated promoter.

By increasing the number of cellular trkB molecules, the response to endogenous neurotrophin may be increased. If the model system contains little or no neurotrophin, neurotrophin may be added to the system. It may also be desirable to add additional neurotrophin to the model system in order to evaluate the effects of excess neurotrophin activity. Over expressing neurotrophin (or secreted neurotrophin) may be the preferable method for studying the effects of elevated levels of neurotrophin on cells already expressing trkB. More preferably would be to express trkB in all cells (general expression) and determine which cells are then endowed with functional responsiveness to neurotrophin, thus allowing the potential identification of a second receptor component, if one exists.

### 5.2.2. MODELS FOR DECREASED NEUROTROPHIN ACTIVITY

Alternatively, an experimental model system may be created which may be used to study the effects of diminished neurotrophin activity. This system may permit identification of processes or neurons which require neurotrophin, and which may represent potential therapeutic targets. In such a system, the response to neurotrophin may be decreased by providing recombinant trkB proteins which are not associated with a cell surface or which are engineered so as to be ineffective in transducing a response to neurotrophin.

For example, trkB protein, peptide, or derivative may be supplied to the system such that the supplied receptor may compete with endogenous trkB for neurotrophin binding, thereby diminishing the response to neurotrophin. The trkB may be a cell free receptor which is either added to the system or produced by the system. For example, a trkB protein which lacks the transmembrane domain may be produced by cells within the system, such as an anchorless trkB that may be secreted from the producing cell. Alternatively, trkB protein, peptide or derivative may be added to an extracellular space within the system.

A recombinant trkB gene may be used to inactivate or "knock out" the endogenous gene by homologous recombination, and thereby create a trkB deficient cell, tissue, or animal. For example, and not by way of limitation, a recombinant trkB gene may be engineered to contain an insertional mutation, for example the neo gene, which inactivates trkB. Such a construct, under the control of a suitable promoter, may be introduced into a cell, such as an embryonic stem cell, by a technique such as transfection, transduction, injection, etc. Cells containing the construct may then be selected by G418 resistance. Cells which lack an intact trkB gene may then be identified, e.g. by Southern blotting or Northern blotting or assay of expression. Cells lacking an intact trkB gene may then be fused to early embryo cells to generate transgenic animals deficient in trkB. A comparison of such an animal with an animal not expressing endogenous neurotrophin would reveal that either the two phenotypes match completely or that they do not, implying the presence of additional neurotrophin-like factors or receptors.

Such an animal may be used to define specific neuronal populations, or any other in vivo processes, normally dependent upon neurotrophin. Thus, these populations or processes may be expected to be affected if the animal did not express trkB and therefore could not respond to neurotrophin.

Alternatively, a recombinant trkB protein, peptide, or derivative which competes with endogenous receptor for neurotrophin may be expressed on the surface of cells within the system, but may be engineered so as to fail to transduce a response to neurotrophin binding.

The recombinant trkB proteins, peptides or derivatives described above may bind to neurotrophin with an affinity that is similar to or different from the affinity of endogenous trkB to neurotrophin. To more effectively diminish the response to neurotrophin, the trkB protein, peptide, or derivative may desirably bind to neurotrophin with a greater affinity than that exhibited by the native receptor.

If the trkB protein, peptide, or derivative is produced within the model system, nucleic acid encoding the trkB protein, peptide, or derivative may be supplied to the system by infection, transduction, transfection, etc. or as a transgene. As discussed supra, the trkB gene may be placed under the control of a suitable promoter, which may be, for example, a tissue-specific promoter or an inducible promoter or developmentally regulated promoter.

The endogenous trkB gene of a cell may be replaced by a mutant trkB gene by homologous recombination.

A test animal may be immunized against trkB.

TrkB expression may be reduced by providing trkB expressing cells with an amount of trkB anti-sense RNA or DNA effective to reduce expression of trkB protein.

### 5.3. DIAGNOSTIC APPLICATIONS

TrkB probes may be used to identify cells and tissues which are responsive to neurotrophin in normal or diseased states. One can provide a method of diagnosing a neurological disorder in a patient comprising comparing the levels of expression of trkB in a patient sample with the levels of expression of trkB in a comparable sample from a healthy person, in which a difference in the levels of expression of trkB in the patient compared to the healthy person indicates that a disorder in the patient may be primarily or secondarily related to trkB metabolism. A patient sample may be any cell, tissue, or body fluid but is preferably nervous system tissue or cerebral spinal fluid. The present invention provides for methods for identifying cells which are responsive to neurotrophin comprising detecting trkB expression in such cells. TrkB expression may be evidenced by transcription of trkB mRNA or production of trkB protein. TrkB expression may be detected using probes which identify trkB nucleic acid or protein.

Yet another variety of probe which may be used is anti-trkB antibody or fragments thereof containing the binding domain. TrkB protein, or

TrkB protein, or fragments or derivatives thereof, may be used as an immunogen to generate anti-trkB antibodies. By providing for the production of relatively abundant amounts of trkB protein using recombinant techniques for protein synthesis (based upon the trkB nucleic acid sequences of the invention), the problem of limited quantities of trkB has been obviated.

To further improve the likelihood of producing an anti-trkB immune response, the amino acid sequence of trkB may be analyzed in order to identify portions of the molecule which may be associated with increased immunogenicity. For example, the amino acid sequence may be subjected to computer analysis to identify surface epitopes which present computer-generated plots of hydrophilicity, surface probability, flexibility, antigenic index, amphiphilic helix, amphiphilic sheet, and secondary structure of trkB. Alternatively, the deduced amino acid sequences of trkB from different species could be compared, and relatively non-homologous regions identified; these non-homologous regions would be more likely to be immunogenic across various species.

For preparation of monoclonal antibodies directed toward trkB, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor et al., 1983, Immunology Today 4:72), and the EBV-hybridoma technique to produce human monoclonal antibodies (Cole et al., 1985, in "Monoclonal Antibodies and Cancer Therapy," Alan R. Liss, Inc. pp. 77-96) and the like are within the scope of the present invention.

The monoclonal antibodies for therapeutic use may be human monoclonal antibodies or chimeric human-mouse (or other species) monoclonal antibodies. Human monoclonal antibodies may be made by any of numerous techniques known in the art (e.g., Teng et al., 1983, Proc. Natl. Acad. Sci. U.S.A. 80:7308-7312; Kozbor et al., 1983, Immunology Today 4:72-79; Olsson et al., 1982, Meth. Enzymol. 92:3-16). Chimeric antibody molecules may be prepared containing a mouse antigen-binding domain with human constant regions (Morrison et al., 1984, Proc. Natl. Acad. Sci. U.S.A. 81:6851, Takeda et al., 1985, Nature 314:452).

Various procedures known in the art may be used for the production of polyclonal antibodies to epitopes of trkB. For the production of antibody, various host animals can be immunized by injection with trkB protein, or a fragment or derivative thereof, including but not limited to rabbits, mice, rats, etc. Various adjuvants may be used to increase the immunological response, depending on the host species, and including but not limited to Freund's (complete and incomplete), mineral gels such as aluminum hydroxide, surface active substances such as lysolecithin, pluronic polyols, polyanions, peptides, oil emulsions, keyhole limpet hemocyanins, dinitrophenol, and potentially useful human adjuvants such as BCG (Bacille Calmette-Guerin) and Corynebacterium parvum.

A molecular clone of an antibody to a trkB epitope can be prepared by known techniques. Recombinant DNA methodology (see e.g., Maniatis et al., 1982, Molecular Cloning, A Laboratory Manual, Cold Spring Harbor Laboratory, Cold Spring Harbor, New York) may be used to construct nucleic acid sequences which encode a monoclonal antibody molecule, or antigen binding region thereof.

Antibody molecules may be purified by known techniques, e.g., immunoabsorption or immunoaffinity chromatography, chromatographic methods such as HPLC (high performance liquid chromatography), or a combination thereof, etc.

One can provide antibody molecules as well as fragments of such antibody molecules. Antibody fragments which contain the idiotype of the molecule can be generated by known techniques. For example, such fragments include but are not limited to: the F(ab')₂ fragment which can be produced by pepsin digestion of the antibody molecule; the Fab' fragments which can be generated by reducing the disulfide bridges of the F(ab'), fragment, and the Fab fragments which can be generated by treating the antibody molecule with papain and a reducing agent.

The abovementioned probes may be used experimentally to identify cells or tissues which hitherto had not been shown to express trkB. Furthermore, these methods may be used to identify the expression of trkB by aberrant tissues, such as malignancies. In additional embodiments, these methods may be used diagnostically to compare the expression of trkB in cells, fluids, or tissue from a patient suffering from a disorder with comparable cells, fluid, or tissue from a healthy person. Fluid is construed to refer to any body fluid, but particularly blood or cerebrospinal fluid. A difference in the levels of expression of trkB in the patient compared to a healthy person may indicate that the patient's disorder may be primarily or secondarily related to trkB metabolism. An increase in levels of trkB, for example, could either indicate that the patient's disorder is associated with an increased sensitivity to normal levels of neurotrophin or, alternatively, may suggest that the patient's neurotrophin levels are low such that the number of receptors is increased by way of compensation. These etiologies may be distinguished from one another by administering neurotrophin to the patient. If his condition worsens, he may suffer from neurotrophin hypersensitivity; if it improves, he may be suffering from a neurotrophin deficiency. Neurotrophin or neurotrophin antagonist-based therapeutic regimens may be chosen accordingly. Differences in expression can be detected at the protein and/or RNA level; i.e. by measuring amounts of trkB protein or trkB RNA in a patient relative to those amounts in healthy persons.

The abovementioned probes may also be used to select neurotrophin-responsive cells for use in assay systems, as described above, or in U. S. Application Serial No. 07/532,285 filed June 1, 1990 (incorporated by reference herein, or according to standard methods of cell selection or cell sorting.

### 5.4. THERAPEUTIC APPLICATIONS

One can provide methods of treating a patient suffering from a neurological disorder comprising treating the patient with an effective amount of trkB protein, peptide fragment, or derivative thereof capable of binding to a neurotrophin. Therapeutic methods comprising administering trkB, trkB agonists, trkB antagonists (which compete with endogenous neurotrophin), or anti-trkB antibodies are within the scope of the present invention.

One can provide pharmaceutical compositions comprising trkB protein, peptide fragment, or derivative in a suitable pharmacologic carrier.

The trkB protein, peptide fragment, or derivative may be administered systemically or locally. Any appropriate mode of administration known in the art may be used, including, but not limited to, intravenous, intrathecal, intraarterial, intranasal, oral, subcutaneous, intraperitoneal, or by local injection or surgical implant. Sustained release formulations are also provided for.

As our understanding of neurodegenerative disease/neurotrauma becomes clearer, it may become apparent that it would be beneficial to decrease the trophic effect of endogenous neurotrophin. Therefore, in areas of nervous system trauma, it may be desirable to provide neurotrophin antagonists, including, but not limited to, soluble forms of trkB which may compete with endogenous cellular receptor for neurotrophin binding. Under such circumstances, it may be desirable to provide neurotrophin antagonist locally at the injury site rather than systemically. Use of a trkB providing implant may be desirable.

Alternatively, certain conditions may benefit from an increase in neurotrophin responsiveness. It may therefore be beneficial to increase the number or binding affinity of trkBs in patients suffering from such conditions. This could be achieved through gene therapy. Selective expression of recombinant trkB in appropriate cells could be achieved using trkB genes controlled by tissue specific or inducible promoters or by producing localized infection with replication defective viruses carrying a recombinant trkB gene. Conditions which may benefit from increased sensitivity to neurotrophin include particularly but are not limited to motorneuron disorders including amyotrophic lateral sclerosis, Werdnig-Hoffmann disease, chronic proximal spinal muscular atrophy, and Guillain-Barre syndrome. Such treatment may also be used for treatment of neurological disorders associated with diabetes, Parkinson's disease, Alzheimer's disease, and Huntington's chorea.

### 5.5. SYSTEMS FOR THE ASSAY AND DISCOVERY OF AGENTS THAT ACT ON RECEPTOR TYROSINE KINASES AND OF NOVEL TYROSINE KINASE RECEPTORS

Systems that may be generally used in both the assay of pre-defined agents, as well as the discovery of novel agents, that act on receptor tyrosine kinases. In a related aspect of this invention, the same system can be used to discover unknown receptors that mediate responses to known factors. Once a particular receptor/ligand system is defined (as is done here with trkB and BDNF/NT-3), a variety of additional specific assay systems can be utilized, as detailed in other sections supra.

The present data reveals that a receptor tyrosine kinase, when introduced into cells that do not normally express this receptor, allows these cells to exhibit profound and easily distinguishable responses to a ligand which binds this receptor. The present data reveals that the type of response elicited depends on the cell utilized, and not the specific receptor introduced into the cell. Thus, the trkB receptor in PC12 pheochromocytoma cells results in BDNF/NT-3 dependent differentiation, whereas the same receptor in fibroblasts mediates both survival and proliferation in response to either BDNF or NT-3. Appropriate cell lines can be chosen to yield a response of the greatest utility for the assay, as well as discovery of, agents that can act on tyrosine kinase receptors. "Agents" refers to any molecule(s), including but not limited to peptide and non-peptide molecules, that will act in systems to be described in a receptor specific manner. One of the more useful systems to be exploited involves the introduction of the desired receptor (e.g. trkB) into a fibroblast cell line (e.g., the particular clone of NIH3T3 cells to be described below, section 7); thus such a receptor which does not normally mediate proliferative responses can, following introduction into fibroblasts, nonetheless be assayed by a variety of well established methods to quantitate effects of fibroblast growth factors (e.g. thymidine incorporation or other types of proliferation assays; see van Zoelen, 1990, "The Use of Biological Assays For Detection Of Polypeptide Growth Factors" in Progress Factor Research, Vol. 2, pp. 131-152; Zhan and M. Goldfarb, 1986, Mol. Cell. Biol., Vol. 6, pp. 3541-3544). These assays have the added advantage that any preparation can be assayed both on the cell line having the introduced receptor as well as the parental cell line lacking the receptor; only specific effects on the cell line with the receptor would be judged as being mediated through the introduced receptor.

Such systems are not limited to the assay of known ligands for known receptors, but can also be utilized to identify novel agents that might act on these or (or any other) receptors. For example, both the cell line bearing the introduced receptor as well as the parental cell line without the receptor can be exposed to any potential source of an agent that might work through the receptor; any specific effects (e.g. on cell survival or proliferation) on the cell line bearing the receptor can be used to identify sources of agents acting on that receptor, and to eventually purify such an agent.

Receptors also need not be limited to those for which a known ligand exists. In fact, this system may allow for the identification of ligands for "orphan" receptors so named because they have no known ligand. Thus, fibroblasts expressing trkB could have been used in such systems in order to identify and eventually purify the ligands (e.g. BDNF and NT-3) that normally activate trkB; they can now be used to identify additional peptide ligands or other agents (e.g. non-peptide molecules) that could act on these receptors. Sources for "agents" could include extracts from a variety of tissues and organisms, or supernatants from cells transfected with genomic DNA or cDNA expression libraries. In a particular embodiment of this invention, fibroblasts expressing an introduced receptor for which a ligand is desired could be transfected with cDNA expression libraries derived from a potential source of such a ligand; cells which survive and form colonies in defined media lacking fibroblast growth factors (Zhan and Goldfarb, 1986, Mol. Cell. Biol., Vol. 6, pp. 3541-3544) would presumably now be making a growth factor that overcomes their normal requirements via an autocrine loop. To prove that this growth factor is working on the receptor of interest, supernatants harvested from these cells could now be assayed on the parental cell lines to prove that it only has actions on the parental cell line expressing this receptor; the transfected piece of DNA encoding the novel activity desired could then be isolated using traditional means.

One can provide methods for cloning at least a portion of tyrosine kinase receptor gene which may then be used in the identification of ligand/receptor pairs, as set forth supra. Such methods comprise (i) amplifying tyrosine kinase encoding nucleic acid sequences by polymerase chain reaction using a collection of cDNA molecules such as a cDNA library) as template and using oligonucleotide primers that correspond to regions of known tyrosine kinase molecules, said regions being associated with tyrosine kinase activity; and (ii) cloning the amplified nucleic acid into an appropriate vector molecule, such as a plasmid, bacteriophage, etc. One can provide for tyrosine kinase genes, and portions thereof, cloned by this method. In particular, non-limiting specific embodiments, set forth in Sections 9 and 10, infra, the oligonucleotide primers utilized may be as set forth in Figure 12A or, alternatively, Table II, infra.

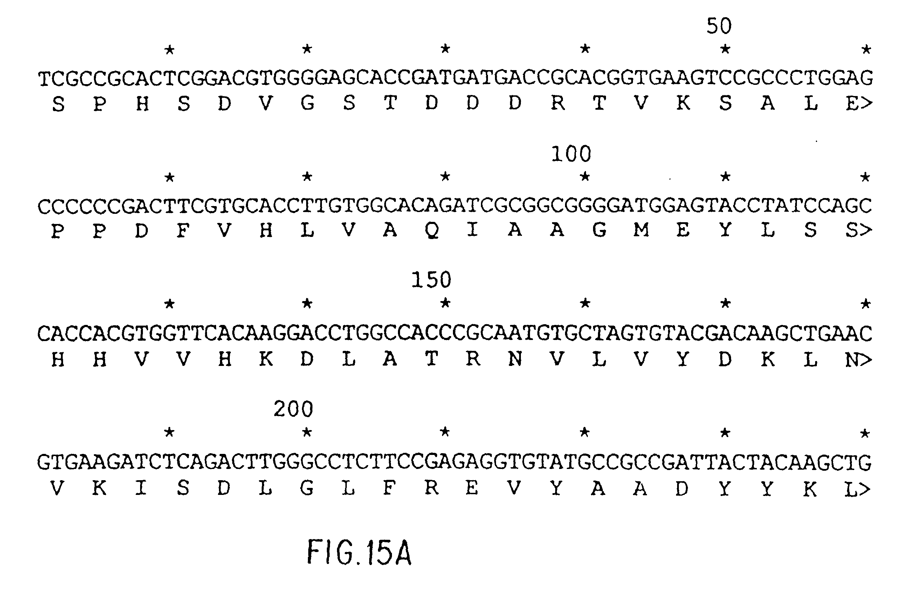

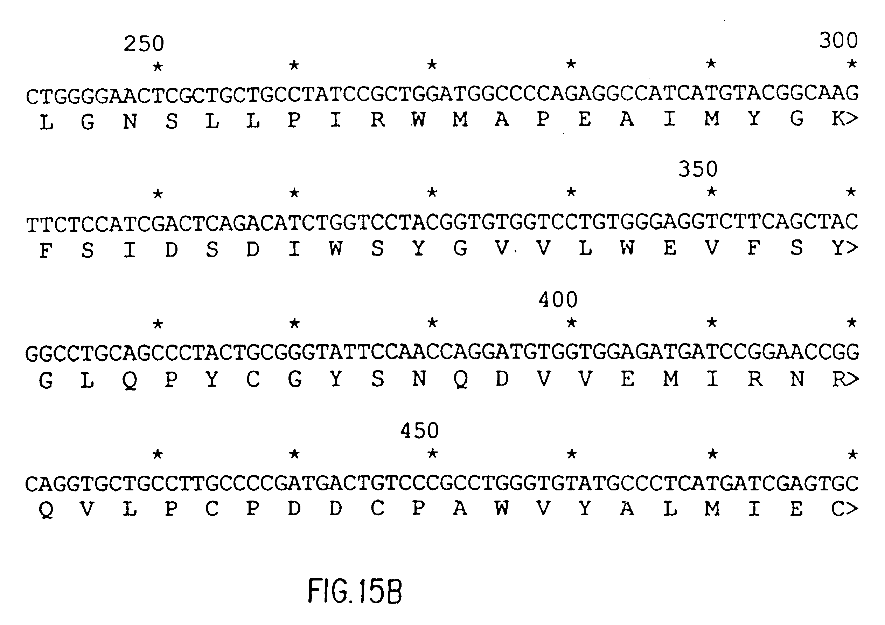

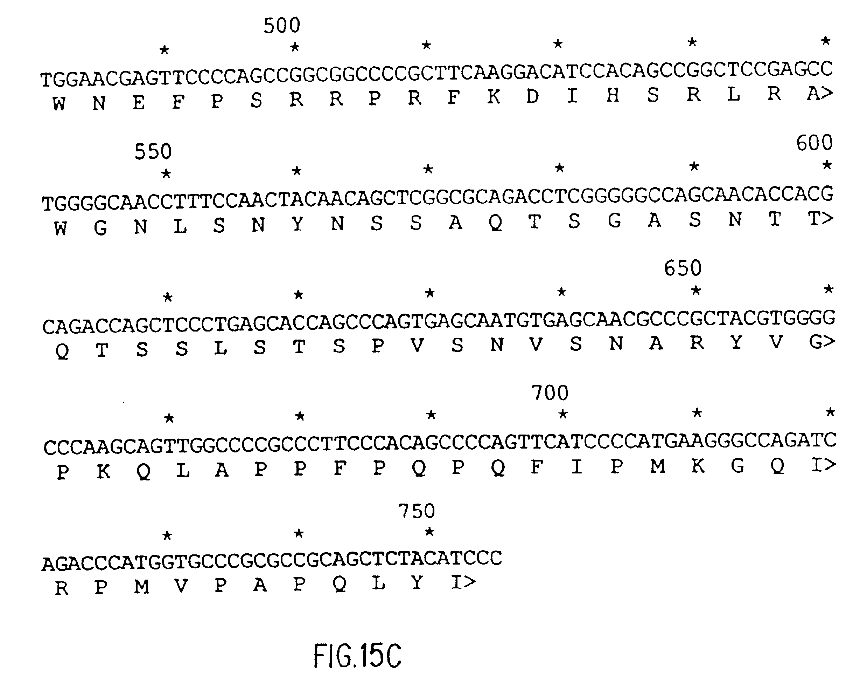

DNA amplified and cloned by this method may then be sequenced using standard techniques. The resulting sequences may then be compared to the sequences of known tyrosine kinase molecules in order to identify clones of particular interest. One can provide the cloned nucleic acid sequences identified using this method, as set forth in Sections 9 and 10, infra, and for peptides and proteins encoded by cDNAs comprising these sequences. Accordingly, the present invention provides for substantially purified recombinant nucleic acid molecules comprising the nucleic acid sequences substantially as set forth in Figure 15 for Rtk-3; or portions thereof comprising at least about ten nucleic acid residues. The present invention also provides for nucleic acids as contained in pBluescript SK-containing Rtk-3 deposited with the American Type Culture Collection and granted accession numbers 75053. The present invention further provides for substantially purified protein molecules comprising the amino acid sequences substantially as set forth in Figure 15 for Rtk-3; or portions thereof comprising at least about six amino acid molecules, or functionally equivalent molecules. Functionally equivalent molecules include those in which amino acid residues are substituted for residues within the sequence resulting in a silent change. For example, one or more amino acid residues within the sequence can be substituted by another amino acid of a similar polarity which acts as a functional equivalent, resulting in a silent alteration. Substitutes for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid. Also included within the scope of the invention are proteins or fragments or derivatives thereof which are differentially modified during or after translation, e.g., by glycosylation, proteolytic cleavage, linkage to an antibody molecule or other cellular ligand, etc.

The present invention further provides for cells and microorganisms that carry the recombinant nucleic acid molecules including, Rtk-3. In particular embodiments, the cell carrying the recombinant nucleic acid is a fibroblast. In other embodiments, the cell is a bacterium.

Amplified nucleic acid fragments may then be used to identify full-length cDNA clones. In preferred embodiments of the invention, an amplified DNA fragment of interest is used to identify a tissue or cell line that expresses relatively abundant levels of a corresponding mRNA, for example, using Northern blot or dot-blot analysis. Such a tissue or cell line may then be used to generate a cDNA library which may serve as a superior source of a full-length cDNA which comprises the sequence and the amplified fragment.

A reciprocal approach could be used to molecularly clone a receptor for an "orphan" factor (for example, a neurotrophic protein for which no receptor has been isolated). Fibroblasts exposed to this factor normally would not respond, but if transfected with a cDNA expression library prepared from cells thought to be expressing this receptor, occasional transfectants would arise which now express this receptor and should now respond to this factor in an autocrine fashion. Powerful selection mechanisms, such as the ability to form colonies in defined media in the presence of the "orphan" factor, should identify transfectants that express the receptor of interest; the gene encoding this receptor could then be isolated by traditional means.

### 6. EXAMPLE: TRKB ENCODES A FUNCTIONAL RECEPTOR FOR BDNF AND NT-3 BUT NOT NGF

### 6.1. MATERIALS AND METHODS

### 6.1.1. CELL CULTURE, NEUROTROPHINS, AND IODINATION OF NEUROTROPHINS

COS-M5 cells were cultured in Dulbecco's modified Eagle's medium (DMEM) containing 10% fetal bovine serum (FBS), 1% each of penicillin and streptomycin (P/S) and 2 mM glutamine in an atmosphere of 5% CO₂. PC12 cells were cultured in DMEM with 6% FBS and 6% horse serum, (P/S) and 2mM glutamine on Costar tissue culture plates in an atmosphere of 7.5% CO₂.PC12 cells obtained from Dr. L.A. Greene's laboratory were utilized in experiments depicted in Figure 2, and PC12 cells obtained from Dr. E.M. Shooter's laboratory were utilized in experiments depicted in Figure 5. The human neuroblastoma cell line, SH-SY5Y (obtained from June Biedler, Sloan-Kettering) was cultured in Eagle's minimal essential medium (EMEM) with 10% FBS, (P/S) and 2 mM glutamine.

Murine 2.5S NGF was obtained from Bioproducts for Science (Indianapolis, IN). Both human BDNF and NT-3 were produced in CHO cells and purified from CHO cell conditioned media to homogeneity as assessed by silver-stained polyacrylamide gels and amino acid sequence analysis. Purified neurotrophins (NGF, BDNF, and NT-3) were all iodinated using the lactoperoxidase method as described in Hempstead et al., 1989, Science, 243: 373-375. Iodinated neurotrophins were separated from unincorporated ¹²⁵I by using a Centriflo CF50A filters (Amicon, Beverly, MA). Aggregates were removed using gel filtration (S200) column chromatography.

### 6.1.2. CLONING RAT trkB: MAMMALIAN EXPRESSION, CONSTRUCTS, AND TRANSIENT TRANSFECTIONS

A full-length rat trkB cDNA clone was obtained by screening a rat brain cDNA library in the lambda ZAP2 vector (Stratagene) with rat trkB-specific oligonucleotides corresponding to the most 5' and 3' coding regions of trkB. Both the human LNGFR (Johnson et al., 1986, Cell 47: 545-554) and rat trkB cDNAs were subcloned into the mammalian expression vector, pCMX, to generate pCMX-LNGFR or pCMX-trkB respectively. pCMX-trkB(del) was generated by digesting the pCMX-trkB plasmid with Apal (which cuts just after the trkB transmembrane domain) and Not1 (which cuts just after the trkB coding region in vector sequences), blunting these ends, and religating the plasmid; the trkB coding region generated includes all of the extracellular and transmembrane domains of trkB, but is lacking the C-terminal 320 amino acids.

COS-M5 cells were transiently transfected with either the pCMX-LNGFR, pCMX-trkB, or control vector (pCMX) by the DEAE-dextran transfection protocol. Briefly, COS-M5 cells were plated at a density of 1.5 x 10⁶ cells per 100 mm plate 24 hours prior to transfection. For transfection, the cells were cultured in serum-free DMEM containing 400 *µ*g/ml of DEAE-dextran, 1 *µ*M chloroquin, 2 mM glutamine, 20 *µ*g/ml insulin, 5 *µ*g/ml transferrin, 33 nM sodium selenite, and 5 *µ*g of the appropriate DNA for 3 hours and 15 minutes at 37°C in an atmosphere of 5% CO2. The transfection media was aspirated and replaced with phosphate-buffered saline with 10% DMSO for 2 min. Following this DMSO "shock", the COS-M5 cells were placed into DMEM with 10% FBS, 1% each of penicillin and streptomycin, and 2 mM glutamine for 48 hours.

PC12 cells were transiently transfected by electroporation. Briefly, the cells were rinsed prior to transfection in ice-cold Dulbecco's phosphate-buffered saline (calcium and magnesium-free) containing 2 mg/ml glucose and then resuspended in the same buffer at a density of 1.5 x 10⁷ cells per ml containing 40 *µ*g of the appropriate DNA. PC12 cells were transfected with either pCMX, pCMX-trkB or the pT24-ras plasmid (Yancopoulos et al., 1985, Proc. Natl. Acad. Sci. USA., 82: 5455-5459). The cell mixture was incubated on ice for 10 min. and then quickly brought to room temperature and electroporated in a total volume of 1 ml at 1150 V/cm and 500 uF. Electroporated cells were incubated on ice for 30 minutes prior to plating in DMEM with 6% FBS and 6% horse serum with 1% each of penicillin and streptomycin and 2mM glutamine; cells were plated on Costar plastic in the absence of any pre-coating. 48 hours after transfection, the cells were treated with 100 ng/ml of neurotrophin or BSA (see Legend of Figure 5) and neurite outgrowth was scored 48 hours later.

### 6.1.3. CHEMICAL CROSS-LINKING

Cells were harvested in phosphate-buffered saline containing 1 mM EDTA, 1 mg/ml glucose and 25 mM HEPES (PBS-versene) and resuspended at an appropriate density (generally 1 x 10⁶ cells per ml) in ice-cold binding buffer A (PBS containing 1 mg/ml each of BSA and glucose). pCMX-trkB or pCMX vector transfected COS-M5 cells (4 x 10⁵ cells) were incubated on ice with ¹²⁵I-labeled neurotrophins (final concentration estimated to be between 0.1 and 0.25 nM) for 90 minutes in the absence or presence of unlabeled NGF, BDNF or NT-3 (see Figures 1 and 3). The chemical cross-linker DSS (Pierce, Rockford, IL) was used following conditions described in Meakin and Shooter, 1991, Neuron 6: 153-163. The cross-linking reaction was terminated after 90 minutes and quenched with 12 ml of 50 mM Tris buffer containing 160 mM NaCl. Cells were centrifuged at 300 x g for 5 minutes and then washed twice with 12 ml of buffer A. Pelleted cells were solubilized in SDS containing 2% of 2-mercaptoethanol, boiled for 5 minutes; radiolabeled cross-linked proteins were resolved on 7% polyacrylamide gels and visualized by autoradiography after exposure of the dried gel to Kodak X-Omat film at -70°C.

### 6.1.4. ¹²⁵I-NT-3 COMPETITION BINDING ASSAYS

Binding of ¹²⁵I-NT-3 to COS-M5 cells transfected with either pCMX-LNGFR, pCMX-trkB or control vector (pCMX) was assessed on cells in suspension. Cells were harvested in PBS-versene and then resuspended in binding buffer A as described above for chemical cross-linking. Cells were incubated with ¹²⁵I-NT-3 (estimated between 0.1 and 0.25 nM) in the absence or presence of increasing concentrations of unlabeled NT-3, BDNF, or NGF ranging from 0.3 to 30 nM for NGF and between 1 and 100 nM for BDNF and NT-3 (see Figure 4C and D). The binding reactions were carried out for 90 minutes on ice. Free ¹²⁵I was separated from bound ¹²⁵I by quickly centrifuging (30 second spin) the reaction mixture through a sucrose gradient formed in a long-tip microcentrifuge tube. The tubes were immediately frozen in a dry-ice/ethanol bath. The bottom of the reaction tube was cut and then counted in a gamma counter.

### 6.1.5. RNA ISOLATION AND NORTHERN BLOTTING ANALYSIS

Total cellular RNA isolated from SH-SY5Y and treated or untreated PC12 cells was fractionated on 1% formaldehyde agarose gels, transferred to nylon membranes and hybridized to a ³²P-labeled v-fos probe or a ³²P-labeled c-jun probe as previously described (Squinto et al., 1990, Neuron 5, 757-766); probings for trkB expression were performed using a ³²P-labeled rat trkB probe spanning a region encoding the intracytoplasmic tyrosine kinase domain.

### 6.2. RESULTS

### 6.2.1. ALL THREE NEUROTROPHINS BIND TO THE LNGFR BUT BDNF AND NT-3 DO NOT ACT VIA THE HNGFR

To determine whether NT-3, like NGF and BDNF (Rodriguez-Tebar, 1990, Neuron 4: 487-492), could also bind to the LNGFR, we examined all three neurotrophins for their ability to be chemically cross-linked to the LNGFR protein expressed transiently in COS cells. Each of the three radiolabeled neurotrophins could be specifically cross-linked to a species of the molecular weight expected for the LNGFR (Figure 1B cross-linked complex reported to be approximately 100 kb by Hosang and Shooter, J.Biol. Chem. 260: 655-662). The cross-linked product was not observed on COS cells that were not expressing the LNGFR protein (Figure 1A). Furthermore, as expected for specific binding the appearance of the cross-linked products could be competed effectively by an excess of the corresponding unlabeled neurotrophin (Figure 1B). Competable cross-linking to a polypeptide of the correct size was also observed in a cell line, A875 melanoma, known to stably express large amounts of the LNGFR (Figure 1C).

Having established that all three neurotrophins bind to the LNGFR protein, we assessed the ability of BDNF and NT-3 to function via low or high affinity NGF receptors. The PC12 cell line, which expresses both classes of NGF receptor, displays prominent responses including both neurite outgrowth and the transcriptional induction of a set of so-called "immediate early genes" in response to NGF (Greene and Tischler, 1976, Proc. Natl. Acad. Sci. U.S.A., 73: 2424-2428; Greenberg et al., 1985, J.Biol. Chem. 260: 14101-14110, see Figure 2A, B). However, when PC12 cells were incubated with BDNF or NT-3 at concentrations either below or exceeding that known to be saturating for responses to NGF, we observed neither morphological changes (under conditions optimal for neurite outgrowth, see below) nor the induction of immediate early gene expression Figure 2A, 2B. This implies that neither low nor high affinity NGF receptors suffice for functional responses to BDNF and NT-3 and that such responses require at least one receptor component not normally found on PC12 cells.

### 6.2.2. BDNF AND NT-3, BUT NOT NGF, BIND TO FULL-LENGTH AND TRUNCATED FORMS OF TRKB

TrkB is not expressed in PC12 cells (Kaplan et al., 1991, Nature 350: 158-160); cell lines expressing trkB have not been described. A full-length trkB cDNA was isolated and transiently expressed in COS cells, on which cross-linking experiments were carried out with radioiodinated neurotrophins. As shown in Figure 3A both BDNF and NT-3, but not NGF, could be cross-linked to a polypeptide of approximately the expected size for the trkB gene product; some heterogeneity in the size of this cross-linked species was observed, as has been previously reported for the HNGFR cross-linked to NGF (Meakin and Shooter, 1991, Neuron 6: 153-163). Cross-linking of labeled BDNF or NT-3 to the presumptive trkB gene product was not observed in the presence of excess unlabeled homologous ligand (Figure 3A, lanes labeled "+"). To verify that the protein cross-linked to BDNF or NT-3 actually corresponds to the trkB gene product, and to determine whether certain truncated forms of the protein are capable of binding ligands, a trkB deletion mutant lacking the intracytoplasmic protein-tyrosine kinase domain was constructed and expressed in COS cells. As illustrated in Figure 3B, radiolabeled ligand (in this case NT-3) was cross-linked efficiently to a surface component of cells expressing the truncated trkB product; furthermore, the marked shift in mobility (corresponding to about 35 kD) observed between the cross-linked species obtained with full-length or truncated trkB proteins agreed well with the known size of the deletion.

### 6.2.3. BDNF AND NT-3, BUT NOT NGF, COMPETE FOR BINDING TO TRKB AND DISPLAY HIGHER AFFINITY BINDING TO TRKB THAN THEY DO TO LNGFR

The specificity and relative affinity of binding of the neurotrophins to the LNGFR and to trkB was compared in competition assays. Each of the three unlabeled neurotrophins were effectively able to specifically block the cross-linking of radiolabeled NT-3 to the LNGFR expressed in COS cells when present at 500 nM levels but not at 1-5 nM levels (Figure 4A). Consistent with these results, the binding of radiolabeled NT-3 to the LNGFR expressed on COS cells was completed similarly by each of the three unlabeled neurotrophins (Figure 4C); the competition curves suggest dissociation constants in the nanomolar range for all three neurotrophins, extending previous observations for NGF and BDNF (Rodriguez-Tebar et al., 1990, Neuron 4: 487-492).

In contrast to the rather high levels of unlabeled ligands required to specifically block binding to the LNGFR, much lower levels of unlabeled BDNF and NT-3 effectively prevented binding of radiolabeled NT-3 to trkB as assayed either by cross-linking (Figure 4B) or direct binding analysis (Figure 4D). NGF, even at 500-1000 fold molar excess, did not compete for binding of radiolabeled NT-3 to trkB in either assay (Figure 4B, 4D). Because the amounts of unlabeled BDNF and NT-3 required to completely inhibit the binding of radiolabeled NT-3 to trkB were 10 to 100-fold lower than those necessary to block binding of NT-3 to the LNGFR, our data suggest that trkB displays considerably higher affinity for both BDNF and NT-3 than does the LNGFR.

### 6.2.4. TRKB MEDIATES NEURITE OUTGROWTH IN RESPONSE TO BOTH BDNF AND NT-3 IN PC12 CELLS

PC12 cells display a characteristic morphological response, neurite extension, indicative of differentiation to a more mature neuronal phenotype when exposed to NGF. As demonstrated above, these cells do not respond to either BDNF or NT-3. To test whether trkB could mediate a biologically relevant response to BDNF or NT-3, PC12 cells were transiently transfected with a trkB expression vector (pCMX-trkB) and incubated with each of the neurotrophins. In order to minimize background, the transfected cells were cultured on standard tissue culture plastic rather than either collagen-coated or Primaria surfaces; under these conditions which are suboptimal for neurite extension (Greene et al., 1987, Meth. Enzymol., 147: 207-216; Chen et al., 1990, Cell Growth Diff. 1:79-85), NGF induced rather short neurites from control PC12 cells as well as from the trk-B-transfected cells (Figure 5B). No cells with neurites were seen in the trkB-transfected cultures in the absence of added neurotrophic factor (Figure 5C). However, many cells in the trkB-transfected PC12 cultures displayed robust neuritic outgrowth in response to either NT-3 or BDNF (Figure 5D, E); no cells with neurites were seen in PC12 cells transiently transfected with control vectors and treated with BDNF or NT-3. As a positive control to assess transfection efficiency, the PC12 cells were transfected with an activated H-ras gene, which has been shown to induce ligand-independent differentiation of PC12 cells. The number of differentiated cells seen in the ras-transfected cultures indicates the number of transiently transfected PC12 cells in the cultures. Since the number of differentiated cells observed following BDNF treatment of pCMX-trkB transfected PC12 cells is comparable with the number of differentiated cells found in the ras-transfected populations (Figure 5A, E), our data suggests that every PC12 cell expressing trkB can respond to BDNF while, however, a smaller subset of trkB expressing PC12 cells responded to NT-3 as measured by neurite outgrowth (Figure 5D); more careful dose-response studies will be required to evaluate the apparent difference between BDNF and NT-3 in this assay. As depicted in Figure 5, it was striking that the extensive neuritic outgrowth seen in ras-transfected PC12 cells or in pCMX-trkB-transfected PC12 cells subjected to BDNF or NT-3 was qualitatively different than the blunted neuritic outgrowth normally seen in response to NGF under these culture conditions.

### 6.2.5. SH-SH5Y HUMAN NEUROBLASTOMA CELLS RESPOND TO BDNF, BUT NOT NT-3, AND DO NOT EXPRESS TRKB: EVIDENCE FOR ANOTHER NEUROTROPHIN RECEPTOR

We have used the induction of immediate early gene expression as an assay (Squinto et al., 1990, Neuron 5:757-766) to search for neuronal tumor cell lines responsive to BDNF, NT-3 and NGF. One such line (SH-SY5Y) was found to express c-fos mRNA in response to both NGF (as previously described) and BDNF, but not NT-3 (Figure 6A). Further studies have verified that BDNF, but not NT-3, has additional functional effects on SH-HY5Y; for example, BDNF protects these cells from oxidative insults. Although this cell line has been shown to express low levels of both high and low affinity NGF receptors (Chen et al., 1990, Cell Growth Diff., 1:79-85) and detectable levels of trkAmRNA, it did not express detectable levels of trkBmRNA (Figure 6B). The apparent lack of trkB expression in a BDNF-responsive cell line, together with its failure to respond to NT-3, leads us to predict that either there are additional modulators of the LNGFR or trkA which confer BDNF responsiveness, or that yet another functional neurotrophin receptor exists which has discrete specificity for BDNF.

### 6.3. DISCUSSION

We conclude that trkB encodes an essential component of a functional receptor for BDNF and NT-3, but not for the third neurotrophin family member, NGF. Recent reports indicate that the trkA proto-oncogene, the closest known relative of trkB, similarly encodes an essential component for a high affinity receptor which binds NGF (Kaplan et al., 1991, Nature, 350: 158-160; Klein et al., 1991, EMBO J. 8: 3701-3709). Our observations that normal PC12 cells do not respond to BDNF or NT-3 imply that trkA, which is expressed in PC12 cells, is uniquely activated by only one known member of the neurotrophin family, NGF.

We find that BDNF and NT-3 bind to trkB in the absence of the LNGFR, and that this binding is of higher affinity than their binding to the LNGFR. Similarly, Klein et al. (1991, Cell 65:189-197) report that NGF can bind to trkA with high affinity in cells that do not express the LNGFR. The function of the LNGFR remains unclear. We extend previous findings (Rodriguez-Tebar et al., 1990, Neuron 4: 487-492) by demonstrating that all three neurotrophins bind to the LNGFR with approximately equal, albeit relatively low, affinity; the conservation of this property suggests a significant biological role. It remains possible that the LNGFR modulates the binding of each of the neurotrophins to its appropriate trk receptor. Alternatively, the LNGFR may mediate signal transduction via an independent pathway, or it may not be directly involved in initiating signal transduction. For example it may act to localize, concentrate or trap the neurotrophins on the surface of LNGFR-expressing cells. In this regard it is of interest that neuronal supporting cells (such as Schwann cells) that do not respond to NGF express the LNGFR and up-regulate it in response to injury (Johnson et al., 1988, TINS 11: 299-304), perhaps providing a fixed matrix or path for the concentration and presentation of neurotrophins to regenerating neurons. Alternatively, the LNGFR may act as a "clearance" receptor that reduces free or circulating levels of the neurotrophins; the LNGFR is widely distributed both in the CNS and in the periphery (Maisonpierre et al., 1990, Neuron 5: 501-509), and secreted forms of the LNGFR (DiStefano and Johnson, 1988, Proc. Natl. Acad., Sci. USA, 85: 270-274) may aid in such clearance mechanisms In regard to non-signalling roles for the LNGFR, related mechanisms specific for BDNF and NT-3 can be proposed based on the presence of truncated forms of trkB. The co-localization of BDNF and truncated trkB transcripts outside of the nervous system (most prominently in lung and skeletal muscle) raises intriguing questions concerning the role of BDNF, trkB and other potential BDNF receptors in non-neural tissues.

The fact that BDNF and NT-3 share a functional receptor, trkB, is consistent with our previous suggestions that the distributions and overlapping neuronal specificities of BDNF and NT-3 particularly link the roles of these two neurotrophins, at least in the central nervous system. During the maturation of different brain regions marked by decreasing NT-3 levels and increasing BDNF levels, the expression of trkB remains relatively constant. However, the identification of a cell line which responds to BDNF but not NT-3 and does not detectably express trkB strongly suggests that these two neurotrophins are not entirely interchangeable, and that there may exist additional receptors or modulatory components, which allow for distinct responses to either BDNF or NT-3. Such modulation may explain, for example, the differing effects of NT-3 and BDNF on PC12 cells transfected with trkB (see above) or on sympathetic neurons (Maisonpeirre et al., 1990, Science, 247: 1446-1451). Sympathetic neurons are known to express trkB, although the available in situ hybridization data do not distinguish between the presence of functional or non-functional trkB transcripts in these neurons (Klein et al., 1989, EMBO J. 8: 3701-3709). An evolutionary comparison of BDNF and NT-3 lead us to predict that these two neurotrophins were strictly conserved to maintain their specific interactions with multiple receptors. Although NGF is well conserved evolutionarily compared to most secreted factors, it does not display the striking conservation that BDNF and NT-3 do, perhaps suggesting that it does not interact with as many receptors as the other two known neurotrophins. Our findings place the neurotrophins in the growing class of receptor/ligand systems in which multiple receptors each bind to several different related ligands (reviewed in Cross and Dexter, 1991, Cell, 64: 271-280).

The binding and activation of receptor tyrosine kinases by the neurotrophins reveals that these factors utilize signalling pathways very similar to those activated by mitogenic growth factors. This finding is consistent with recent data that neurotrophic factors can act as mitogens in certain contexts (e.g. Cattaneo and McKay, 1990, Nature, 347: 762-765), but also indicates that signals which initiate the activation of receptor tyrosine kinases normally integrate into non-mitogenic transduction pathways in neurons. Despite differences in ultimate sequelae (i.e. mitogenesis vs. survival or differentiation), at least some of the early intermediates in tyrosine kinase signalling cascades, such as the ERK family of protein kinases, are similarly activated in both neuronal and non-neuronal cells. Other examples in which activation of receptor-like tryosine kinases in neuronal cells leads to non-mitogenic sequelae include the Drosophila sevenless protein, where activation via a non-diffusable ligand is required for the differentiation of photoreceptor cells (Basler et al., 1991, Cell 64: 1069-1081).

### 7. EXAMPLE: TrK MEDIATES BDNF/NT-3-DEPENDENT SURVIVAL AND PROLIFERATION IN FIBROBLASTS LACKING THE LOW-AFFINITY NGF RECEPTOR

### 7.1. MATERIALS AND METHODS

### 7.1.1. CELLS, CELL CULTURE AND DNA TRANSFECTIONS

NIH3T3 cells were cultured in Dulbecco's modified Eagle's medium (DMEM) containing 10% bovine calf serum, 1% each of penicillin and streptomycin (P/S) and 2 mM glutamine in an atmosphere of 5% CO₂. For defined media assays, defined media was prepared as described in Zhan et al., (1987, Oncogene 1:369-376). While we have demonstrated that our NIH3T3 cells lose viability in growth factor-deficient media (Zhan et al., Oncogen 1:369-376; Zhan and Goldfarb, 1986, Mol. Cell. Biol. 6:3541-3544), it should be noted that some other variants of NIH3T3 cells do not lose viability, but rather quiesce, in growth factor deficient media. DNA transfections (using pLTR-trkB and pLTR-hyg constructs described by Squinto et al., 1991, Cell 65:1-20) into these cells were performed as reported in Wigler et al. (1979, Cell 16:777-785). COS cell culture and transfection with pCMX-LNGFR were performed as described in Squinto et al. (1991, Cell 65:1-20). Neuronal survival assays using chick dorsal root ganglion neurons were performed described in Rodrigeuz-Teber and Barde (1988, The Journal of Neuroscience 8:3337-3342).

### 7.1.2. FACTORS

Preparation, purification and radiolabelling of recombinant human BDNF and NT-3, produced in CHO cells, was as described in Squinto et al. (1991, Cell 65:1-20). Murine 2.5S NGF was obtained from Bioproducts, Indianapolis, IN. bFGF was purchased from R&D Systems, Inc., Minneapolis, MN.

### 7.1.3. SURVIVAL, PROLIFERATION AND THYMIDINE INCORPORATION ASSAYS

Survival and proliferation assays were essentially performed as described in Zhan et al. (1987, Oncogene 1:369-376; Zhan and Goldfarb, 1986, Mol. Cell. Biol. 6:3541-3544). In brief, survival assays were performed by plating cells at 20% confluency on poly-D-lysine/fibronectin coated dishes and culturing in defined media (+/- factors) for 5 days; the degree of survival was scored microscopically and ranked from "-", for almost no survival, to "+++", for confluent survival. For proliferation assays, cells were plated at approximately 0.5% confluency on poly-D-lysine/fibronectin coated dishes, cultured for 8 days with or without added factors, and counted in a Coulter counter after trypsinization. For DNA synthesis assays cells were plated in medium containing 3% calf serum, and after achieving quiescence were challenged with indicated factors for 14 hours; the media was then supplemented with ³H-thymidine, at 1 microCurie per milliliter, for two hours. Radiolabel incorporation into TCA-insolulble material was subsequently determined as described in Cavalieri and Goldfarb (1987, Mol. Cell. Biol. 7:3544-3560).

### 7.1.4. RNA ISOLATION AND NORTHERN BLOTTING ANALYSIS

RNA isolation and Northern blotting analysis was conducted as described in Maisonpierre et al. (1990, Neuron 5:501-509).

### 7.1.5. PROTEIN ISOLATION AND WESTERN BLOTTING ANALYSIS

For detection of changes in tyrosine phosphorylation in response to factor addition, cells were starved for one hour in serum-free, defined media and then treated for 5 minutes with the factors as indicated. Cells were then washed in phosphate-buffered saline (PBS) with 1 mM orthovanadate, and lysed in RIPA lysis buffer (PBS containing 1% NP40, 0.1% SDS, 0.5% deoxycholate, 1 mM PMSF, 1 mM orthovanadate and 0.14 U/ml of aprotinin). For the experiments depicted in Figure 11A, the lysates were precipitated with agarose-conjugated anti-phosphotyrosine monoclonal antibody, designation 4G10 (FOXNY Sp2 derivative myeloma x BALB/c spleen cells), obtained from Upstate Biotechnologies, Inc.; the immunoprecipitates were washed in lysis buffer, suspended by boiling in SDS-containing loading buffer, and run on 6% SDS-acrylamide gels and immunoblotted using the 4G10 anti-phosphotyrosine antibody. For the experiments depicted in Figure 11B, the total cell lysates were directly electrophoresed on 8% SDS-acrylamide gels prior to immunoblotting using the 4G10 anti-phosphotyrosine antibody. Immunoblots were performed using Immobilon-P membranes; following application of the primary antiphosphotyrosine antibody 4G10, an ¹²⁵I-labelled goat anti-mouse polyclonal antibody was used for detection.

### 7.2. RESULTS

### 7.2.1. BDNF AND NT-3 ACT AS SURVIVAL FACTORS FOR NIH3T3 CELLS EXPRESSING TRKB IN THE ABSENCE OF THE LNGFR

In order to examine the functional capabilities of a trk receptor when expressed in non-neuronal cells, we co-transfected a trkB expression construct (pLTR-trkB, Squinto et al., 1991, Cell 65:1-20) and a hygromycin selectable marker gene (pLTR-hyg) (Murphy and Efstratiadis, 1987, Proc. Natl. Acad. Sci. U.S.A. 84:8277-8281) into the variant NIH3T3 cells described above. These NIH3T3 cells did not express detectable LNGFR as assayed by direct cross-linking to radiolabelled NT-3 (Figure 7A; compare LNGFR-expressing cells in lanes 1, 2 with NIH3T3 cells in lanes 3, 4) or by ¹²⁵I NGF binding studies performed on whole cells. Northern blot analysis also failed to detect LNGFR mRNA in NIH3T3 cells, although this mRNA was readily detectable in PC12 cells (estimated to have 100,000 LNGFRs/cell) or SH-SY5Y cells (which express barely detectable levels of the LNGFR; Chao et al., 1986, Science 232:518-521; Sonnenfeld and Ishii, 1982, J. Neurosci. Res. 8:375-391; Figure 7B, upper panel). Following co-transfection with pLTR-trkB and pLTR-hyg, the NIH3T3 cells were replated at 20% confluency and then either selected in defined medium alone or in defined media supplemented with 5 nM of either bFGF, NGF, BDNF or NT-3, as well as in complete medium containing hygromycin and calf serum (Figure 8). Direct selection in defined media revealed that all plated cells survived when the medium was supplemented with bFGF, as expected. Furthermore, no colonies were found when the defined media was not supplemented or supplemented only with NGF, indicating that trkB is incapable of providing a growth signal without stimulation by its cognate ligands. In contrast, several colonies were detected when the pLTR-trkB transfected cells were grown in defined media supplemented with either BDNF or NT-3; somewhat more colonies were found in the BDNF-containing media than in the NT-3 containing media (Figure 8, left side). No colonies were seen in similar platings performed using the untransfected NIH3T3 cells, cells transfected with pLTR-hyg alone, or cells containing an expression construct for the LNGFR. Thus, transfection of NIH3T3 cells with pLTR-trkB resulted in cells which could form growing colonies in response to either BDNF or NT-3.

In order to determine whether the majority of trkB-transfected cells could respond to BDNF or NT-3, or whether the colonies described above reflected rare clones in the transfected population, the trkB-transfected cells were first selected in media containing 10% calf serum and hygromycin. These hygromycin-selected cells expressed substantial amounts of trkB transcripts (Figure 9B, lower panel) and detectable trkB protein as judged by chemical cross-linking to radiolabelled NT-3 (Figure 7A, lanes 5, 6); the number of trkB receptors in these cells is low in comparison to the number of LNGFR molecules expressed in COS cells transiently transfected with a LNGFR expression construct (Figure 7A, compare lanes 1, 2 and 5, 6). The trkB-expressing cells were then tested in "survival assays" in which the cells were plated at relatively high density (20% confluency) in defined media alone or in defined media supplemented with 5 nM of bFGF or each of the neurotrophins. Within a few days, almost complete cell death had occurred in unsupplemented defined media or in defined media supplemented with NGF (Figure 9A). Strikingly, confluent monolayers indicative of relatively uniform survival were seen in cultures of trkB-expressing cells supplemented with either bFGF, BDNF or NT-3 (Figure 9A, bottom row). Survival was supported by bFGF, but not by any of the three neurotrophins, in cultures of the parental NIH3T3 cells (Figure 9A, top row), of cells transfected with pLTR-hyg alone, or of cells containing an LNGFR expression vector.

Dose-response survival assays revealed that picomolar concentrations of bFGF, BDNF and NT-3 were effective, with bFGF and BDNF activity obvious at 0.5-5 pM, and NT-3 effects discernible at 50 pM (Figure 9B). However, NGF was not effective even at 100-1000 fold higher concentrations (Figure 9A). Thus BDNF was almost as potent as bFGF in promoting survival of trkB-transfected NIH3T3 cells, while NT-3 was at least 10-fold less effective than BDNF in this assay.

### 7.2.2. BDNF AND NT-3 ACT AS PROLIFERATIVE FACTORS FOR TrkB-EXPRESSING NIH3T3 CELLS

To determine whether BDNF and/or NT-3 activation of trkB provides mitogenic as well as survival signals to NIH 3T3 cells, we tested BDNF and NT-3 for their ability to promote DNA systhesis in trkB-expressing cells by utilizing a thymidine incorporation assay. Cells were grown to confluency in 3% serum, at which point they appeared quiescent based on the absence of mitotic cells; no cell death was apparent under these conditions. The various factors were then directly added to this media, and ³H-labeled thymidine incorporation was monitored 12 hours after factor addition. As shown in Figure 10A, only bFGF was able to induce DNA systhesis in the parental NIH 3T3 cells, while efficient DNA synthesis was also induced by picomolar concentrations of BDNF and NT-3 in the trkB-expressing NIH 3T3 cells (Figure 10B); the BDNF/NT-3 dose dependencies observed in this assay roughly paralleled those seen in the survival assay (compare Figure 9B and Figure 10B). NGF was not capable of inducing DNA synthesis in either the parental or the trkB-expressing cells (Figures 10A and 10B).

BDNF and NT-3 were also tested for their ability to promote long-term proliferation of trkB-expressing NIH 3T3 cells. In these assays factors were tested for their ability to enhance the growth rate of sparsely plated parental or trkB-expressing cells; in contrast to the cell death that follows the dense plating of these cells in mitogen-free defined medium, continued proliferation in the absence of notable cell death is seen in cells plated sparsely (at 0.5% confluency) in such medium. In addition to the expected response to bFGF, an enhanced growth rate of trkB-expressing NIH-3T3 cells was seen in response to both BDNF and NT-3 (Figure 10C); the parental NIH 3T3 cells only responded to bFGF. Again, the dose dependencies of BDNF and NT-3 for proliferation paralleled those seen for the survival and thymidine incorporation assays (compare Figures 9B, 10B and 10C).

### 7.2.3. TrkB-EXPRESSING FIBROBLASTS AND BDNF-DEPENDENT PRIMARY NEURONS DISPLAY SIMILAR DOSE-RESPONSE CURVES FOR BDNF

To determine whether the survival and proliferative effects of BDNF upon trkB-expressing fibroblasts occur at physiologically appropriate neurotrophin levels, we compared the BDNF concentrations required for these effects with the concentrations required in a classical neuronal survival assay using BDNF-dependent neurons isolated from dorsal root ganglia (DRG). Recombinant human BDNF displayed an almost indistinguishable dose-response curve (Figure 10D) for neuronal survival as was reported for BDNF purified from pig brain (Rodrigeuz-Tebar and Barde, 1988, The Journal of Neuroscience 8:3337-3342). This dose-response curve on primary neurons is very similar to those described above for survival and proliferation in fibroblasts (compare Figure 10D to Figures 9B, 10B, and 10C). Thus fibroblasts that express trkB in the absence of the LNGFR display a sensitivity to BDNF which is similar to that displayed by BDNF-dependent primary neurons.

### 7.2.4. BDNF AND NT-3 ACTIVATE TYROSINE PHOSPHORYLATION IN TrkB-EXPRESSING NIH3T3 CELLS

In order to examine the signalling pathways triggered by the binding of neurotrophins to TrkB, we have assayed for tyrosine phosphorylation in trkB-expressing NIH3T3 cells following treatment with bFGF or the neurotrophins. Parental or trkB-expressing NIH3T3 cells were treated with factors for five minutes, lysates were prepared, and then immunoprecipitated using an anti-phosphotyrosine antibody. The precipitated proteins were separated by gel electrophoresis,and detected by immunoblotting with the anti-phosphotyrosine antibody. The trkB-expressing NIH3T3 cells treated with BDNF or NT-3 displayed a major tyrosine phosphorylation product of approximately 145 kD, corresponding to the known size of TrkB (Figure 11B); this product was not seen in parental cells exposed to any of the factors, nor was it seen in trkB-expressing cells exposed to either bFGF or NGF (Figure 11A). These data suggest that the major 145 kD phosphorylation product induced by BDNF and NT-3 is trkB.

BDNF and NT-3 treatment of trkB-expressing NIH3T3 cells also induced tyrosine-phosphorylation of a 41 kD protein (Figure 11B). bFGF also induced phosphorylation of a 41 kD protein in both parental and trkB-expressing NIH3T3 cells (Figure 11B). Based on studies by Boulton et al. (1991, Cell 65:663-677), this phosphoprotein may correspond to ERK2, a serine/threonine kinase which is activated and tyrosine phosphorylated in PC12 cells in response to NGF. These data are consistent with the notion that neurotrophin and growth factor receptors phosphorylate some of the same targets in both neuronal and non-neuronal cells.

### 7.3. DISCUSSION

We have demonstrated that trkB, when expressed in LNGFR-positive PC12 cells, can mediate neurite outgrowth in response to both BDNF and NT-3 (Squinto et al., 1991, Cell 65:1-20). We now provide evidence that a member of the trk family can mediate biologically relevant responses to the neurotrophins without collaborating with the LNGFR; BDNF and NT-3 act as survival and proliferative factors for NIH3T3 cells expressing trkB but no detectable LNGFR. These survival and proliferative effects occurred with a dose-dependence very similar to that required to support survival of primary neurons in culture. Furthermore, the actions of BDNF and NT-3 on trkB-expressing fibroblasts,both biologically and biochemically, are analogous to those of a traditional growth factor, bFGF.

Our findings reveal that the neurotrophin receptors do not appear to be unusual among receptor tyrosine kinases in either their requirement for interactions with other receptor components, or in the types of signals they transduce. Therefore the apparently unique actions of the neurotrophins, in terms of promoting neuronal survival and differentiation as opposed to cell growth, can most probably be attributed to the relatively restricted distribution of the trk receptors in post-mitotic neurons rather than any unique features of the receptor system. Conversely, our data would also suggest that the apparent neurotrophic actions of traditional growth factors, including members of the FGF family, are mediated by signal transduction pathways which overlap those utilized by the neurotrophins.

The identification of the receptors utilized by the neurotrophins, as well as the establishment of non-neuronal cell lines which display survival and proliferative responses to these factors, should prove useful in understanding how the same signals can ultimately be interpreted differently in neurons and non-neuronal cells. We note that the trkB-expressing NIH3T3 cells provide the first example of an immortalized cell line which displays responses to the neurotrophins at physiologically appropriate levels; responses in PC12 cells require approximately 100-fold higher NGF levels than responses in primary neurons. Our studies indicate that survival and proliferation assays utilizing NIH3T3 cells expressing novel receptor tyrosine kinases may be used as a powerful system for the molecular cloning of the ligands for these orphan receptors. Finally, the NIH3T3 system may also be used to provide insight into how neurotrophic factors prevent neuronal cell death; it can now be determined whether the neurotrophins utilize the same or distinct pathways to prevent neuronal versus non-neuronal cell death.

An important issue raised by our data concerns the role of the LNGFR, a low-affinity binding site for all the known neurotrophins. A variety of studies indicate that introduction of the LNGFR into non-neuronal cells does not confer neurotrophin responsiveness upon the recipient cells (Hempstead et al., 1989, Science 243:373-375; Sehgal et al., 1988, Mol. Cell. Biol. 8:2242-2246). The most direct evidence supporting a functional role for the LNGFR in mediating signal transduction involved an experiment in which the LNGFR was reintroduced into a PC12 cell mutant which had apparently lost all detectable NGF binding and responsiveness (Hempstead et al., 1989, Science 243:373-375; Sehgal et al., 1988, Mol. Cell. Biol. 8:2242-2246). The resulting transfectant displayed both high and low-affinity NGF binding sites and exhibited limited responsiveness to NGF. It was assumed that this experiment demonstrated that the LNGFR was the only NGF-binding molecule expressed by PC12 cells, and that it collaborated with a second moiety that had no NGF binding ability on its own but which could convert the LNGFR to a signal-transducing high affinity receptor. The observations that the trk proteins bind the neurotrophins,and that PC12 cells normally express trkA, call for a re-interpretation of this critical experiment. While recent publications have differed as to whether trkA requires the LNGFR to generate high affinity NGF binding sites, it should be pointed out that only a single protein, now known to correspond to trkA, can be cross-linked to NGF under high-affinity binding conditions (Hosang and Shooter, 1985, J. Biol. Chem. 260:655-662). Furthermore, an antibody which efficiently prevents NGF binding to the LNGFR blocks all low-affinity but not all high-affinity NGF binding, and does not prevent NGF-induced responses in PC12 cells (Weskamp and Reichardt, 1991, Neuron 6:649-663). In any case, our functional data convincingly demonstrate that the LNGFR appeared not to be required for trkB mediated responses to physiologically appropriate doses of BDNF and NT-3. It still remains possible that the LNGFR somehow modulates trkA or trkB-mediated responses. Alternatively, the LNGFR could play a signalling role in a pathway not involving the trks, although the apparent inability of BDNF or NT-3 to elicit responses from PC12 cells seems to make this a less likely possibility (Squinto et al., 1991, Cell 65:1-20). The finding that the LNGFR is much more widely distributed than the trks (Bothwell, 1991 Curr. Top. Microbiol. Immunol. 165:63-70), particularly within apparent targets of innervation that produce neurotrophins but which are not known to respond to them, seems to support more of an "immunobilization" or "clearance" role rather than a signalling role for the LNGFR.

Our findings add the neurotrophins and the trk proteins to the growing list of factor/receptor systems in which multiple related ligands can act on the same receptor (Cross and Dexter, 1991, Cell 64:271-281). NT-3 and BDNF display dramatically reciprocal expression patterns during development (Maisonpierre et al., 1990a, Neuron 5:501-509). The NT-3 gene is expressed at much higher levels during early development than is the BDNF gene; NT-3 expression then decreases while BDNF levels increase. This reciprocal relationship between NT-3 and BDNF expression is particularly noteworthy in light of our findings that trkB can act as a BDNF receptor, and somewhat less well as an NT-3 receptor. The apparently high levels of NT-3 early in development may allow it to act more generally on receptors such as trkB; when NT-3 expression decreases to low levels unidentified, NT-3 receptors.

### 8. EXAMPLE: CLONING OF ORPHAN TYROSINE KINASE RECEPTOR MOLECULES

### 8.1. MATERIALS AND METHODS

In Figure 12E, Roman numerals indicate tyrosine kinase homology domains as identified by Hanks et al. (1988) Science 241, 42-52. Highlighted regions of sequence homology in Figure 12E (underlined and in bold; lying within the catalytic domain of these proteins) were used in designing degenerate oligonucleotide primers with which to prime PCR reactions using either adult or embryonic (E13) rat brain cDNAs. Resulting amplified DNA fragments were cloned by insertion into plasmids, sequenced and the DNA sequences were compared with those of all known tyrosine kinases.

cDNA templates were generated by reverse transcription of adult and E13 total rat brain RNAs using oligo d(T) primers. Combinatorial PCR reactions with either of the two cDNA groups were done comparing the six combinations of downstream and upstream primers (see Table I below and Figure 12A) at primer annealing temperatures of 40°C versus 45°C. Aliquots of the PCR reactions were subjected to electrophoresis on an agarose gel, blotted to nylon and probed with "internal" radiolabeled degenerate oligonucleotides complimentary to the domain VII homology box (see above). Based on the relative intensities of the hybridization signals obtained, five PCR reactions (distinguished by the indicated properties) were selected for further analysis:

**TABLE I**

| PCR # | anneal | cDNA source | Primers |
|---|---|---|---|
| 1 | 40°C | E13 | DLATRN-DVWSLG |
| 2 | 40°C | adult | DLATRN-DVWSYG |
| 3 | 40°C | E13 | DLATRN-DVWSYG |
| 4 | 40°C | adult | DLAARN-DVWSLG |
| 5 | 40°C | E13 | DLAARN-DVWSLG |

Size-selected amplified DNA fragments from these PCR reactions were cloned into plasmids as follows: Each of the five PCR pools was digested with XhoI and SstI to cleave sites in the termini of the primers (see below) and then subjected to electrophoresis through a 6% polyacrylamide gel. Ethidium bromide-stained (XhoI/SstI-cut) DNAs of 225 bp, +/- 25 bp, were purified by elution from appropriate excised polyacrylamide gel fragments. The eluted DNAs were cloned into compatible XhoI//SstI sites in the Bluescript II KS(-) plasmid, introduced into DH5α E. coli by electroporation, followed plating of transformants on selective agar. Approximately 200 ampicillin-resistant bacterial colonies from each PCR transformation were inoculated into 96-well microtiter plates and have been stored as glycerol stocks at -80°C. Individual colonies from these five pools of PCR clones have been analyzed by sequencing of plasmid DNAs that have been purified by standard plasmid miniprep procedures.

### 8.2. RESULTS AND DISCUSSION

Oligonucleotide primers corresponding to conserved regions of known tyrosine kinase molecules were used to amplify and clone DNA sequences encoding novel orphan tyrosine kinase receptor molecules. The amino acid sequences of representatives from branches of the tyrosine kinase superfamily (Hanks et al., 1988, Science 241:42-52) were aligned (Figure 12E), and regions of homology within the catalytic domain of these proteins were identified and used to design degenerate oligonucleotide primers, depicted in Table I (supra) and Figure 12A. These primers were then used to prime PCR reactions with, as template, adult or embryonic (E13) rat brain cDNAs. Resulting amplified DNA fragments were then cloned into Bluescript II KS(-) plasmid, sequenced, and the DNA sequences compared with those of known tyrosine kinases.

Figure 12B presents the amino acid sequences encoded by a number of these clones which are homologous to a number of known tyrosine kinase molecules. The five particular cloned molecules of Figure 12B bear homology to trk, CSF1R/PDGFT/kit, ret, eck (alpha), and eck (beta) and are termed respectively, Rtk-1, Rtk-6, Rtk-7, Rtk-8, and Rtk-9. Figure 12C presents DNA sequences for each of these five tyrosine kinase clones, as well as information about the cDNA source and primers used to amplify each clone. Also shown are alignments of these DNA sequences (or their cognate amino acid sequence) with near relatives from the tyrosine kinase superfamily.

Figure 12D shows the amino acid sequence of a rat brain cDNA that was obtained by probing with the Rtk-1 cDNA sequence shown in Figure 12C. The cDNA clone contains sequences identical to Rtk-1 and an additional 1440 amino acid segment of the gene, and based on alignment with trkA and trkB, may correspond to the carboxy terminus of the Rtk-1 gene product.

### 9. EXAMPLE: CLONING OF ORPHAN TRK-LIKE RECEPTOR MOLECULES

### 9.1. MATERIALS AND METHODS

PCR-mediated cloning was performed essentially as described in Section 9, supra, but using cDNA from the human neuroblastoma cell line SY5Y as template, and oligonucleotide primers shown in Table II, infra.

### 9.2. RESULTS AND DISCUSSION

The goal of this work was to identify, by PCR, DNA sequences closely related to trk and trkB genes. Thus, oligodeoxynucleotide primers were designed which correspond to protein regions strongly conserved in all protein kinases, but which at the same time would strongly bias the amplification reaction towards trk-related sequences. For example, trk-10r primer in Fig. 13A corresponds to the -PIRWMPPE- in which trk and trkB are identical,but even their closest known relatives, insulin and insulin-like growth factor receptors have two amino acid substitutions. Figure 13A shows alignment of tyrosine kinase domains of the members of insulin receptor subfamily with regions of sequence conservation identified earlier by others, and the location of all primers used in this work.

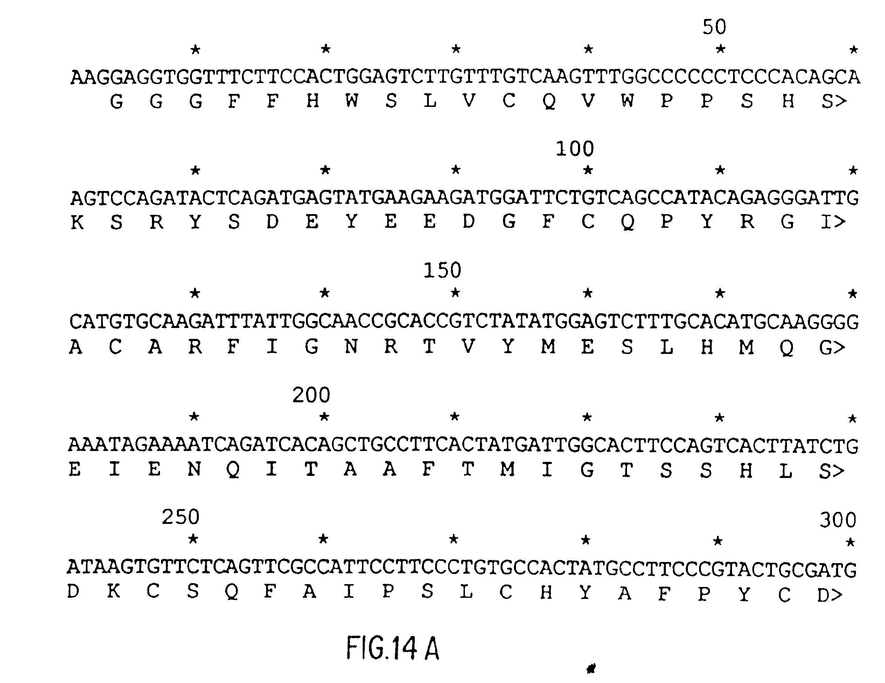

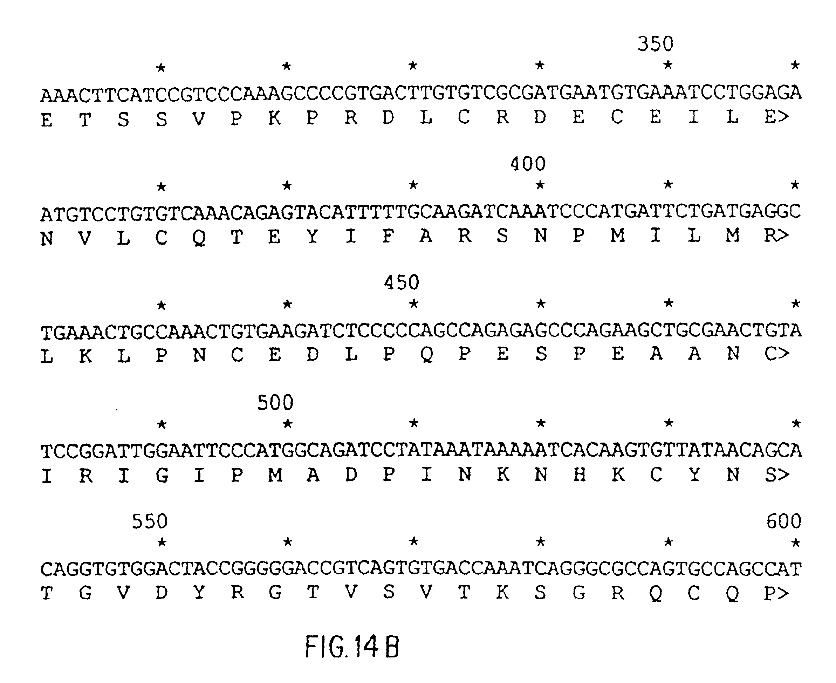

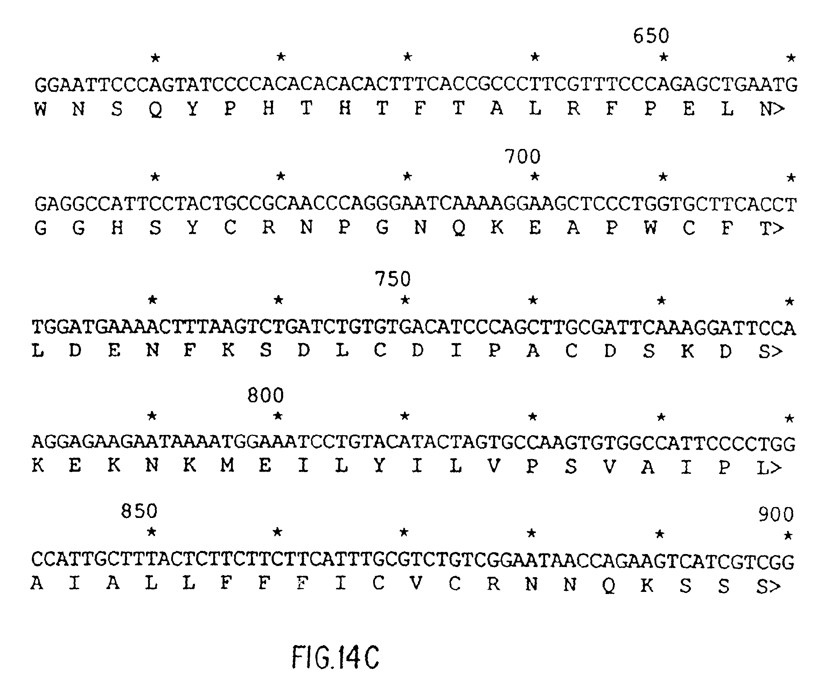

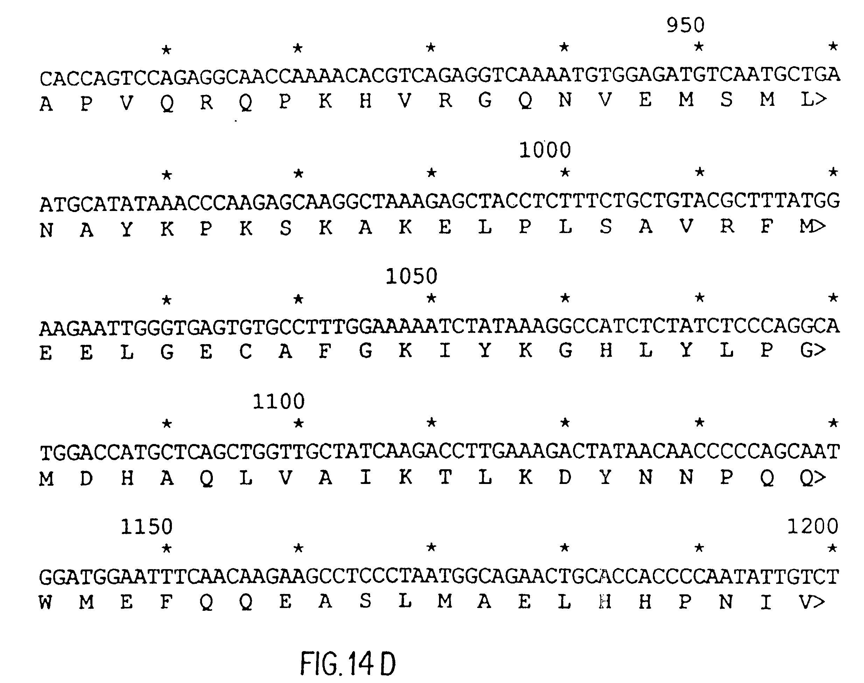

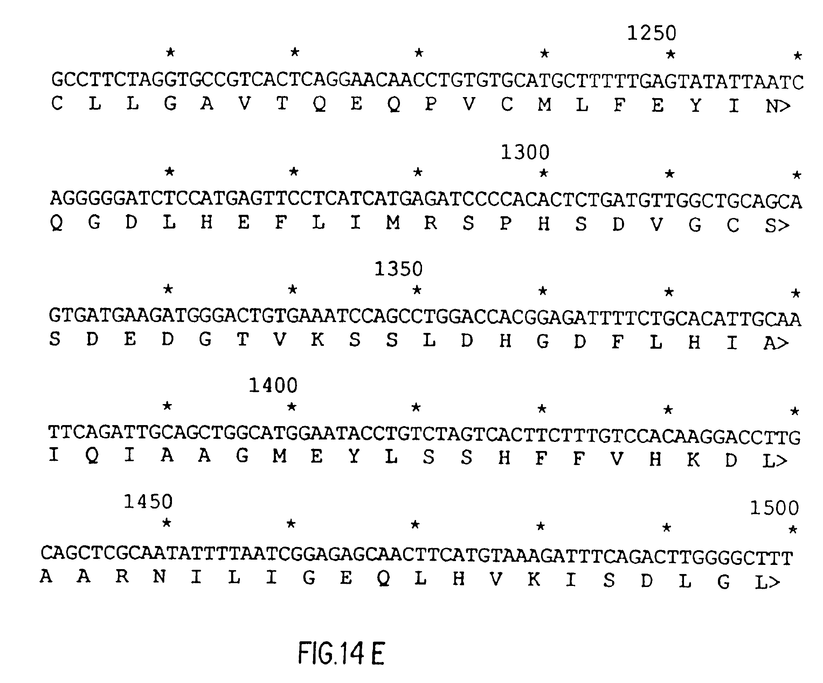

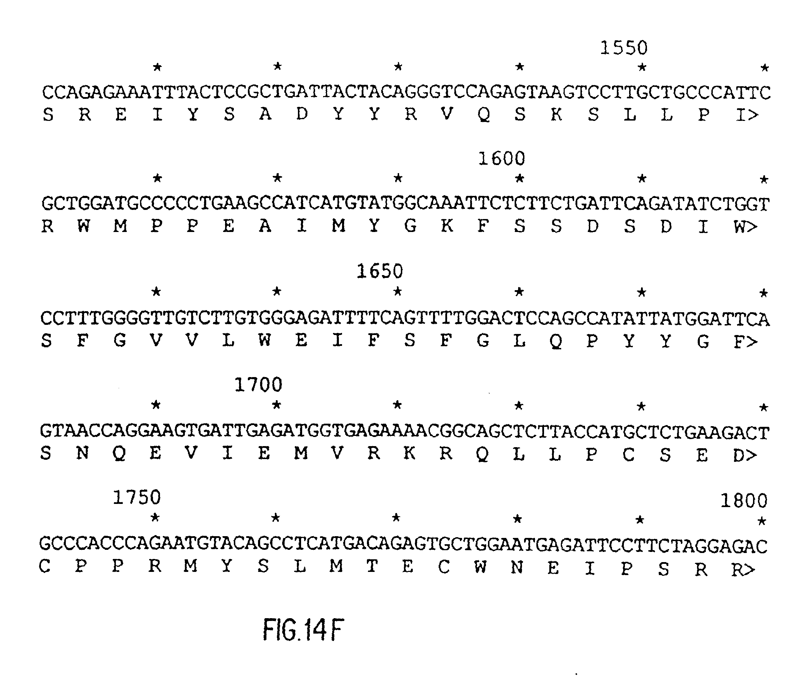

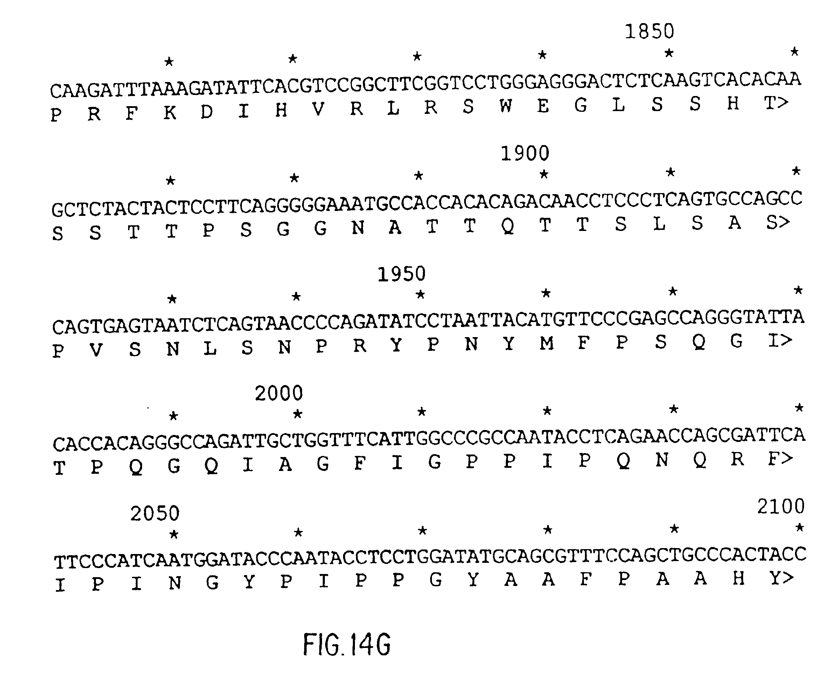

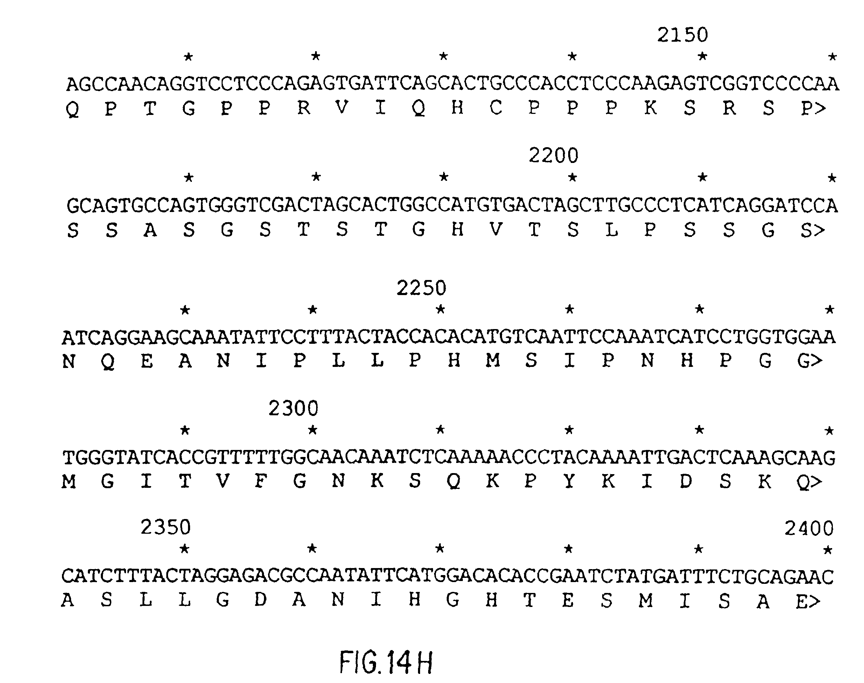

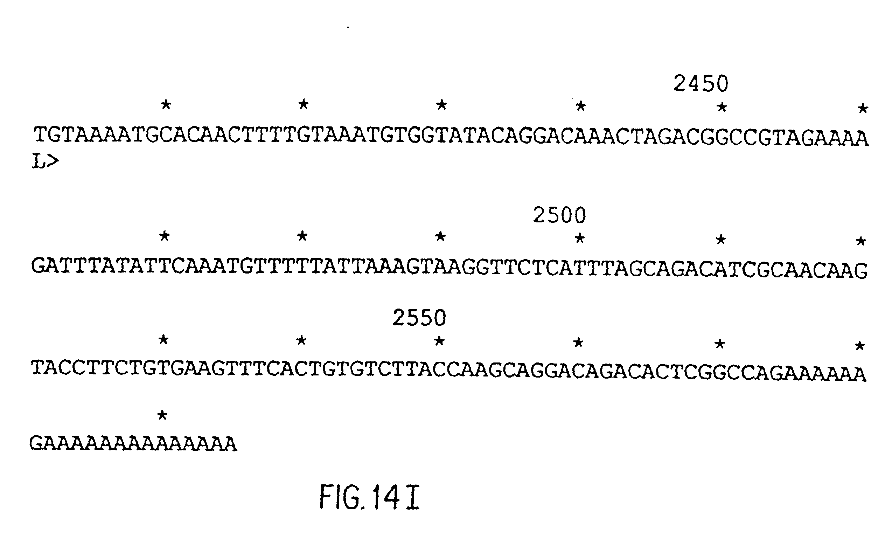

Using cDNA from human neuroblastoma cell line SY5Y as a template, polymerase chain reactions (PCRs) with various combinations of primers were performed. The DNA products were digested with restriction enzymes which cut trk and trkB sequences, and the resistant material was re-amplified and cloned. Individual clones were then sequenced. Amino acid sequences deduced from these DNA sequences revealed the presence of fragments of four novel potential receptors in the pool. These fragments, referred to as Rtk-2, Rtk-3, Rtk-4 and Rtk-5, are depicted in Figure 13B (Rtk-4 and Rtk-5), Figure 14 (Rtk-2), and Figure 15 (Rtk-3) and compared with the corresponding region of human trk in Figure 13C. Rtk-2 is shown alligned with human trk, rat trkB, insulin-related growth faction receptor, and insulin receptor in Figure 13D.

The following oligodeoxynucleotide primers were used in preparation of the PCR fragments (the restriction site "tail" used for cloning is shown in italics, and the degeneracy at particular position is indicated by the letters in parentheses):

| | TABLE II |
|---|---|
| | Rtk-2 and Rtk-3 |
| TRK-9 | ACGTCTCGAG-GC(TCAG) -GG(TCAG) -ATG-GT(TCAG) -TA(TC) - (TC)T |
| TRK-10r | CATGTCTAGA-GGC-ATC-CA(TCAG) -C(GT) (AGT) -AT (TCAG) - GG |

| | Rtk-4 |
|---|---|
| TRK-5 | ACGTCTCGAG-AA(AG)-AT(TCA)-GG(TCAG) -GA(TC) -TT(TC) -GG |
| TRK-10r | CATGTCTAGA-GGC-ATC-CA(TCAG) -C(GT) (AGT) - AT (TCAG) - GG |

| | Rtk-5 |
|---|---|
| TRK-9 | ACGTCTCGAG-GC(TCAG) -GG(TCAG) -ATG-GT(TCAG) -TA (TC) - (TC) T |
| TRK-6r | CATGTCTAGA-CC- (GA)AA- (GA)TC- (CTGA)CC-(TGA)AT- (CT)TT |

Rtk-2 and Rtk-3 sequences are of particular interest. When the deduced amino acid sequences were used to search GenBank, Release 67.0 (03/91) and EMBL (Modified) Release 26.0 (02/91) Databases with TFASTA, the highest homology scores were obtained for trkB and trk sequences. Rtk-2 and Rtk-3 contain the YxxDYY sequence characteristic for trk/insulin-R subfamily. Both sequences have an L for F substitution in the DFG motif which is very strongly conserved in all kinases.

When the Rtk-2 and Rtk-3 probes were hybridized to Northern blots, no signal was obtained with SY5Y RNA. Rtk-2 hybridized to a 6-7 kb band in RNAs from other lines, in particular from CHP100, SKES, and LAN5 cells. Rtk-3 hybridized to a 5 kb band in RNAs from SKN-SH and LAN5 cells. Screening of 1.5 x 10⁶ clones from SY5Y cDNA library in lambda ZPA II with Rtk-2 probe yielded 5 positive clones, with inserts in the range 1.4-3 kb.

Sequence data for Rtk-2, shown in Figure 14, indicates that nucleotides 801-875 appear to code for a transmembrane domain, nucleotides 1002-1850 appear to contain a tyrosine kinase domain; and 1-800 code for a continuous open reading frame comprising a ligand binding domain. A 180 amino acid stretch follows the tyrosine kinase domain, in contrast to trks, which terminate shortly following the tyrosine kinase domain.

The partial amino acid sequence of Rtk-3 shown in Figure 15, which also shows the presence of this stretch beyond the tyrosine kinase domain and shows strong homology to Rtk-2.

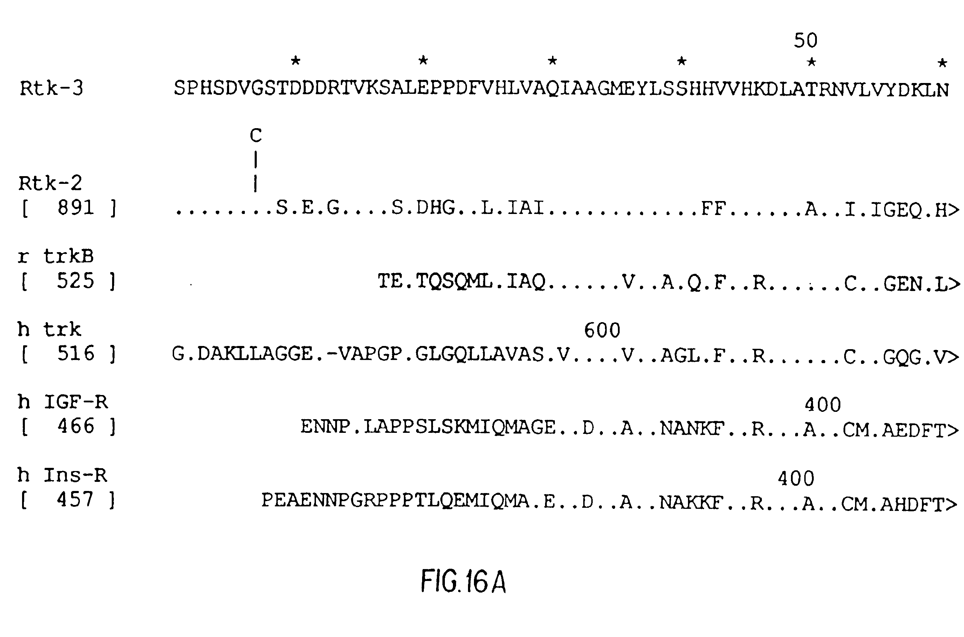

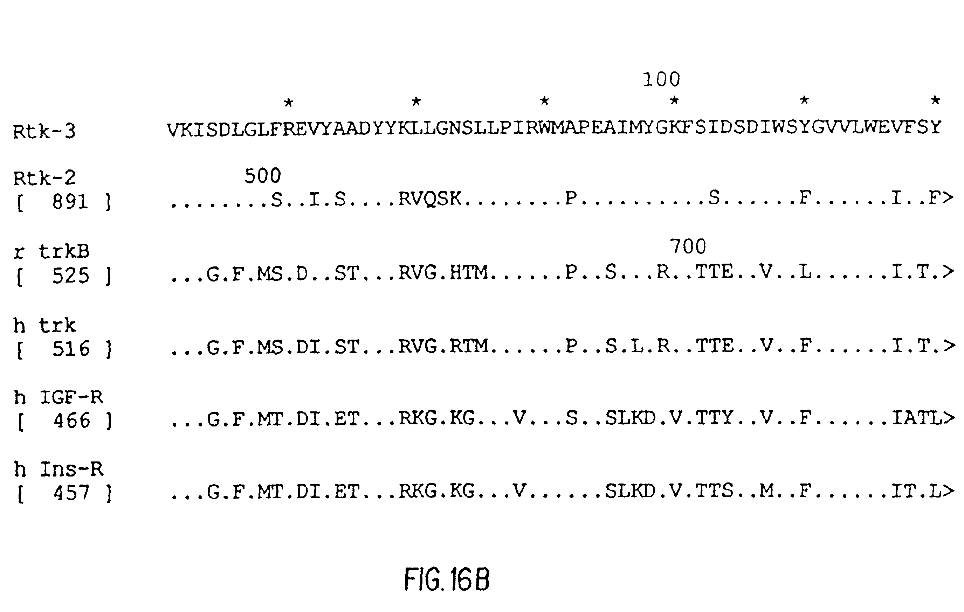

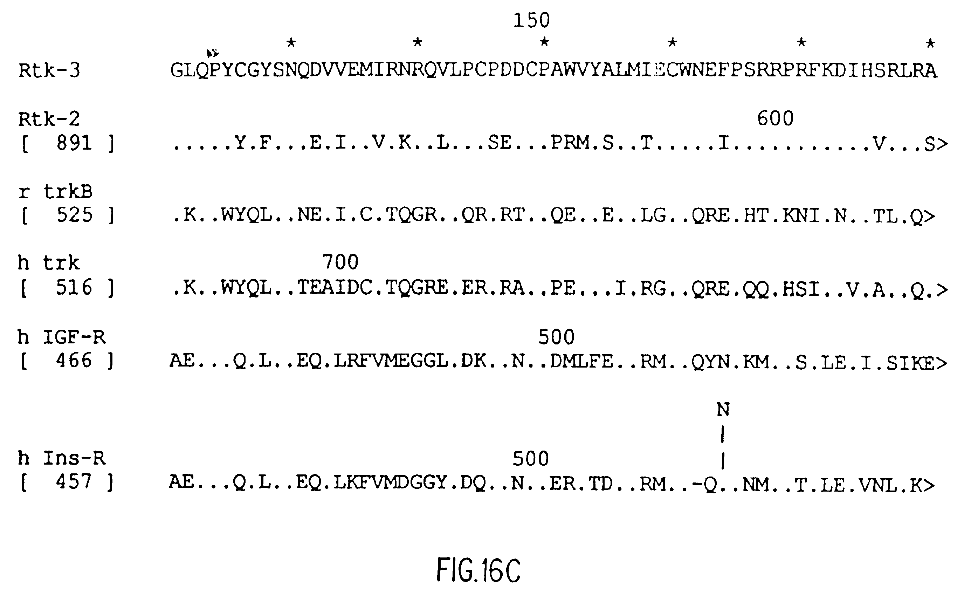

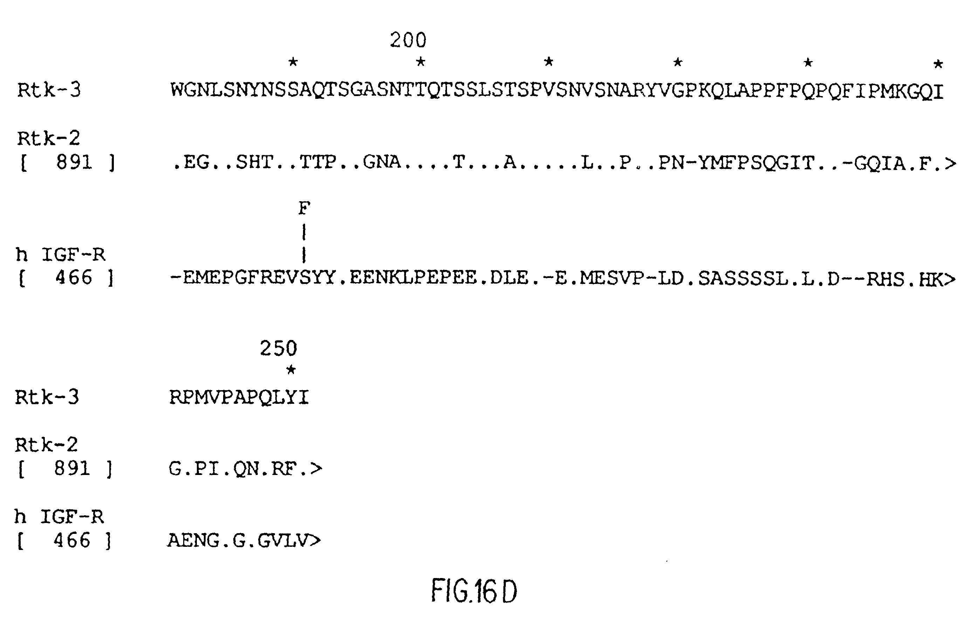

Alignment of the Rtk-3 sequence to the sequences for Rtk-2, trks, IGFR, and Insulin receptor, shown in Fig. 16, indicates that Rtk-2 and Rtk-3 share the greatest homology, suggesting they are members of a novel subfamily of tyrosine kinase receptors.

### 10. FUNCTIONAL ASSOCIATION OF TRKS WITH NEUROTROPHINS IN RAT EMBRYONIC DORSAL ROOT GANGLIAA

### 10.1. MATERIALS AND METHODS

Dorsal root ganglion (DRG) from embryonic rat (E14) were dissected and collected in serum-containing medium (F14 medium supplemented with 5% horse serum), as described in Linsay et al. (1985, Dev. Biol. 112: 319). For explant assays, ganglia were put onto 35 mm dishes precoated with polyornithine (1mg/ml) and laminin (50ug/ml). Ganglia were cultured for 24 hours in the presence or absence of neurotrophins at 37 °C in a humidified 3% CO2 atmosphere. The extent of neurite outgrowth was scored with a scale of 0-5. At the end of the culture period, ganglia were rinsed with PBS, following which RNA was prepared using guanidinium thiocyanate, as described in Chomczynski and Sacchi (1987, Analytical Biochem. 162: 156). For dissociated cell assays, ganglia were incubated with 0.1% trypsin at 37°C for 30 minutes, after which they were triturated and preplated for 2.5 hours. Neurons were then collected and seeded onto 16mm wells precoated with 100ug/ml polyornithine and 50ug/ml laminin. Neurons were cultured for 24 hours in the presence or absence of neurotrophins, and cell counts were scored. RNA from tissues were prepared using standard techniques.

### 10.2. RESULTS

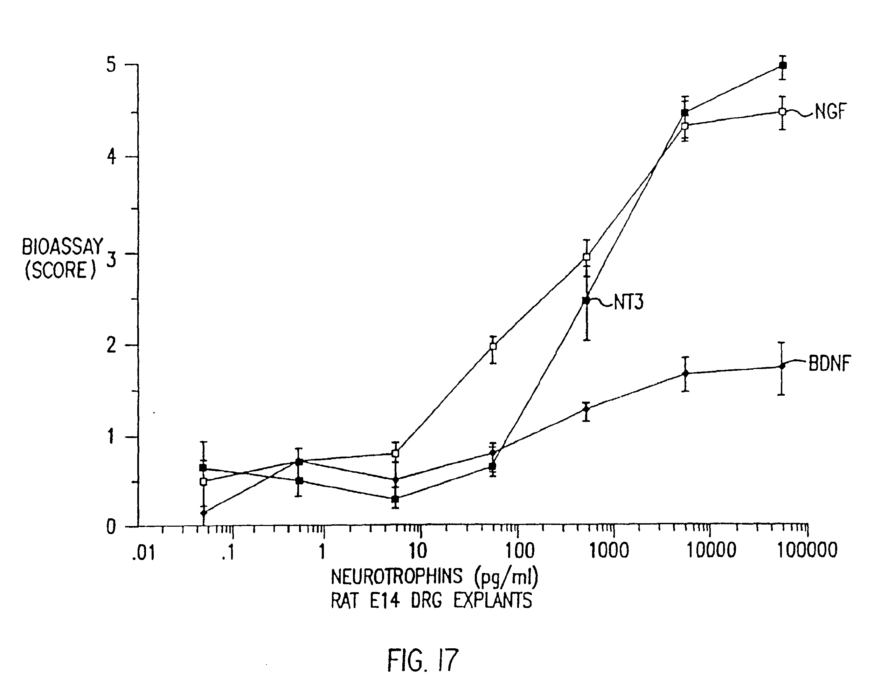

Incubation of DRG with NGF with various concentrations of NGF resulted in massive neurite outgrowth from the explant in a dose-dependent manner (Figure 17). Similar results were obtained with NT-3. The response saturates at approximately 5ng/ml of NGF or NT-3. BDNF, on the other hand, produced a weaker, but also consistent, response with DRG explants (Figure 17).

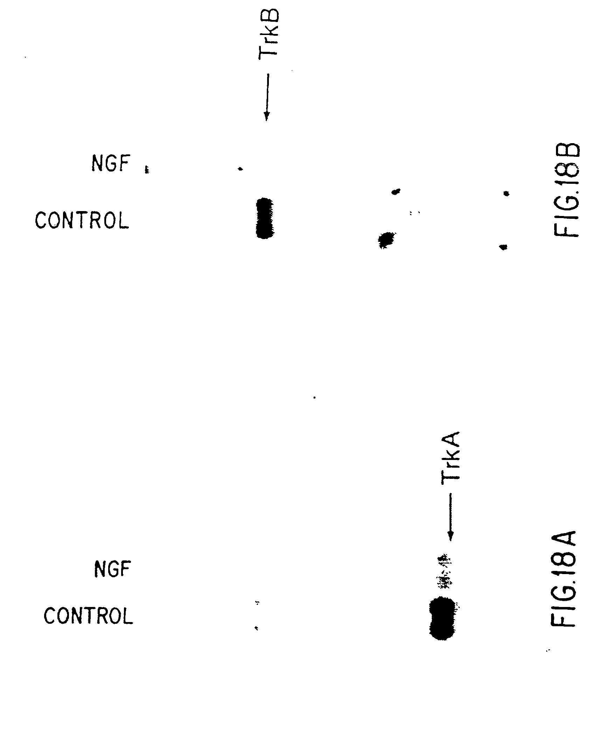

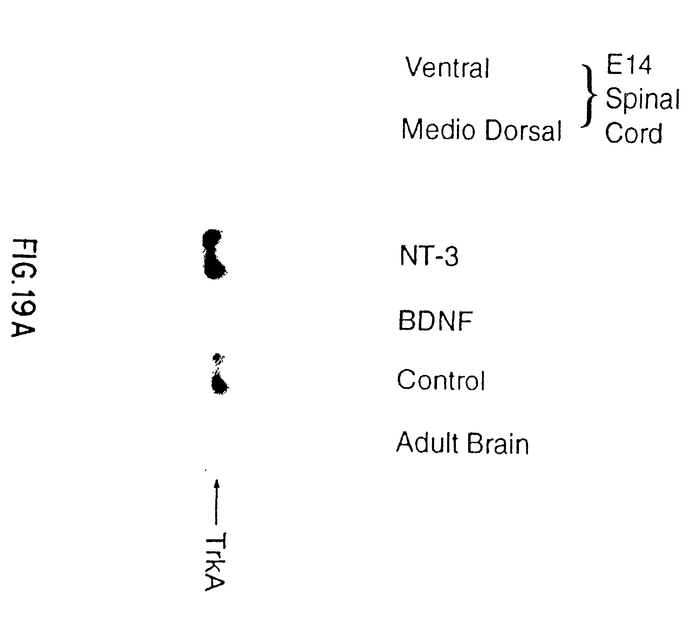

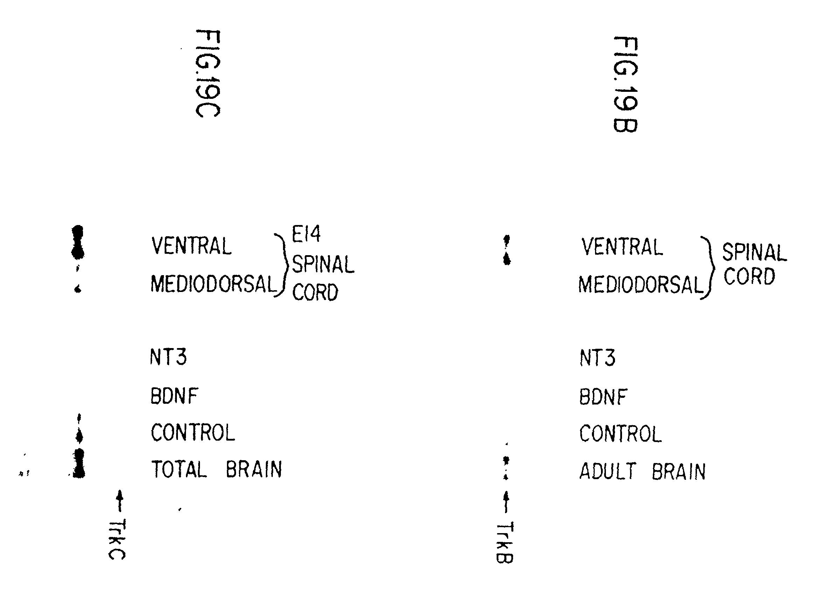

mRNA levels for trkA, B, and C were examined in the ganglia cultured in the presence or absence of various neurotrophins. In control DRG taken from E14 embryonic rat, mRNA for trkA, B and C can be detected (Figure 18 and 19). As shown in Figure 18A, ganglia incubated in the presence of NGF (50ng/ml) had a significant trkA mRNA level, but undetectable trkB message (Figure 18B). On the other hand, ganglia incubated in the presence of BDNF (50ng/ml) revealed significant trkB message (Figure 19B), but very low levels of trkA (Figure 19A) and no trkC (Figure 19C). Finally following treatment with NT-3 (50ng/ml), ganglia had significant trkC and trkA mRNA, but very low levels of trkB message. These results demonstrate that the neuronal populations surviving in the presence of these neurotrophins possess specific receptors for the respective neurotrophins. That is, following incubation with BDNF, only trkB-containing neurons appear to survive, whereas treatment with NGF resulted in survival of only trkA-containing neurons. Treatment of NT-3 appeared to result in survival of both trk-C and trk-A containing neurons. The fact that specific receptors for the respective neurotrophins are present on distinct cell types suggest that neurotrophins may be used to rescue distinct cell populations in the peripheral nervous system.

### 11. DEPOSIT OF MICROORGANISMS

The following microorganisms have been deposited with the American Type Culture Collection, 12301 Parklawn Drive, Rockville, Maryland 20852.

| | ACCESSION NUMBER |
|---|---|
| pBluescript SK-containing Rtk-2 | 75052 |
| pBluescript SK-containing Rtk-3 | 75053 |

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims.

## Claims

1. A purified protein comprising an amino acid sequence encoded by the human nucleotide sequence contained in the plasmid pBluescript SK-containing Rtk-3 as deposited with the American Type Culture Collection (ATCC) and assigned accession no. 75053.

2. An isolated nucleic acid comprising the human nucleotide sequence contained in the plasmid pBluescript SK-containing Rtk-3 as deposited with the American Type Culture Collection (ATCC) and assigned accession no. 75053.

3. An isolated nucleic acid comprising a nucleotide sequence which encodes the protein of claim 1.

4. A recombinant vector comprising the nucleic acid of claim 2 or 3.

5. A recombinant host cell containing the vector of claim 4.

6. A method for producing a Rtk-3 protein comprising growing a recombinant cell containing the vector of claim 4 such that the Rtk-3 protein encoded by said vector is expressed by the cell, and recovering the expressed Rtk-3 protein.

## Patentansprüche

1. Gereinigtes Protein, aufweisend eine Aminosäuresequenz, die kodiert wird durch die menschliche Nukleotidsequenz, die enthalten ist in dem Plasmid pBluescript SK-enthaltend Rtk-3, wie hinterlegt bei der American Type Culture Collection (ATCC) unter der Zugangsnummer 75053.

2. Isolierte Nukleinsäure, aufweisend die menschliche Nukleotidsequenz, die enthalten ist in dem Plasmid pBluescript SK-enthaltend Rtk-3, wie hinterlegt bei der American Type Culture Collection (ATCC) unter der Zugangsnummer 75053.

3. Isolierte Nukleinsäure, aufweisend eine Nukleotidsequenz, die das Protein von Anspruch 1 kodiert.

4. Rekombinanter Vektor, aufweisend die Nukleinsäure von Anspruch 2 oder 3.

5. Rekombinante Wirtszelle, enthaltend den Vektor von Anspruch 4.

6. Verfahren zur Herstellung eines Rtk-3-Proteins, aufweisend das Züchten einer den Vektor von Anspruch 4 enthaltenden, rekombinanten Zelle dergestalt, dass das von dem Vektor kodierte Rtk-3-Protein von der Zelle exprimiert wird, uns das Gewinnen des exprimierten Rtk-3-Proteins.

## Revendications

1. Une protéine purifiée comprenant une séquence d'acides aminés codée par la séquence nucléotidique humaine contenue dans le plasmide pBluescript SK contenant Rtk-3 déposé auprès du American Type Culture Collection (ATCC) et ayant le numéro d'accession n° 75053.

2. Un acide nucléique isolé comprenant la séquence nucléotidique humaine contenue dans le plasmide pBluescript SK contenant Rtk-3 tel que déposé à l'American Type Culture Collection (ATCC) et ayant le numéro d'accession n° 75053.

3. Un acide nucléique isolé comprenant une séquence nucléotidique qui code la protéine de la revendication 1.

4. Un vecteur recombinant comprenant l'acide nucléique de la revendication 2 ou 3.

5. Une cellule hôte recombinante contenant le vecteur de la revendication 4.

6. Une méthode pour produire une protéine Rtk-3 comprenant la culture d'une cellule recombinante contenant le vecteur de la revendication 4 de façon à ce que la protéine Rtk-3 codée par ledit vecteur est exprimé par la cellule, et l'extraction de la protéine Rtk-3 exprimée.
